# EUROPÄISCHE PATENTSCHRIFT

(11) **EP 0 224 794 B1**
(45) Veröffentlichungstag und Bekanntmachung des Hinweises auf die Patenterteilung: **12.09.1990**
(21) Anmeldenummer: 86116002.6
(22) Anmeldetag: 18.11.1986
(51) Int. Cl.: C07D 401/06, C07D 491/04, C07D 223/16, C07D 403/06, A61K 31/55

(54) **Neue cyclische Aminderivate, diese Verbindungen enthaltende Arzneimittel und Verfahren zu ihrer Herstellung**
Cyclic amine derivatives, medicines containing these compounds and process for their preparation
Dérivés amines cycliques, médicaments contenant ces composés et leur procédé de préparation

(30) Priorität: 27.11.1985 DE 3541811
(43) Veröffentlichungstag der Anmeldung: 10.06.1987
(73) Patentinhaber: Dr. Karl Thomae GmbH, D-88397 Biberach (DE)
(72) Erfinder: Psiorz, Manfred, Dr. Dipl.-Chem., D-7950 Biberach 1 (DE); Heider, Joachim, Dr. Dipl.-Chem., D-7951 Warthausen 1 (DE); Bomhard, Andreas, Dr. Dipl.-Chem., D-7950 Biberach 1 (DE); Reiffen, Manfred, Dr. Dipl.-Chem., D-7950 Biberach 1 (DE); Hauel, Norbert, Dr. Dipl.-Chem., D-7950 Biberach 1 (DE); Noll, Klaus, Dr. Dipl.-Chem., D-7951 Warthausen 1 (DE); Narr, Berthold, Dr. Dipl.-Chem., D-7950 Biberach 1 (DE); Lillie, Christian, Dr., A-1130 Wien (AT); Kobinger, Walter, Prof. Dr., A-1121 Wien (AT); Dämmgen, Jürgen, Dr., D-7951 Sulmingen (DE)

(56) Entgegenhaltungen:
- EP-A- 0 065 229
- EP-A- 0 161 599
- CH-A- 645 632
- DE-A- 2 639 718

## Beschreibung

In der EP A 0 065 229 und in der EP A 0 161 599 werden bereits Benzazepin- und Benzdiazepinderivate, die in 3-Stellung durch einen Phenylalkylaminoalkylrest substituiert sind, wobei die zu dem Phenylkern benachbarte Methylengruppe durch eine gegebenenfalls durch eine Benzylgruppe oder eine Alkylgruppe mit 1 bis 3 Kohlenstoffatomen substituierte Iminogruppe, ein Sauerstoff- oder Schwefelatom, eine Sulfinyl- oder Sulfonylgruppe ersetzt sein kann, und deren physiologisch verträgliche Säureadditionssalze beschrieben, welche wertvolle pharmakologische Eigenschaften aufweist, nämlich neben einer milden blutdrucksenkenden Wirkung insbesondere eine selektive herzfrequenzsenkende Wirkung.

Überraschenderweise wurde nun gefunden, daß die neuen cyclischen Aminderivate der allgemeinen Formel 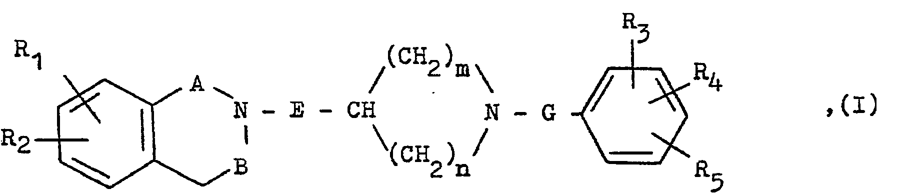 deren Enantiomeren, deren Diastereomeren und deren Säureadditionssalze, insbesondere deren physiologisch verträgliche Säureadditionssalze mit anorganischen oder organischen Säuren, noch wertvollere pharmakologische Eigenschaften aufweisen, insbesondere eine langanhaltende herzfrequenzsenkende Wirkung und eine herabsetzende Wirkung auf den 02-Bedarf des Herzens.

Gegenstand der vorliegenden Erfindung sind somit die neuen cyclischen Aminderivate der obigen allgemeinen Formel I, deren Enantiomeren, deren Diastereomeren, deren Säureadditionssalze, insbesondere für die pharmazeutische Verwendung deren physiologisch verträgliche Säureadditionssalze mit anorganischen oder organischen Säuren, Verfahren zu ihrer Herstellung und diese Verbindungen enthaltende Arzneimittel.

In der obigen allgemeinen Formel bedeuten
A eine -CH2-CH2-, -CH=CH-, 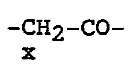 oder 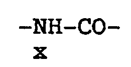 und
B eine Methylen-, Carbonyl- oder Thiocarbonylgruppe oder
A eine -CO-CO- oder 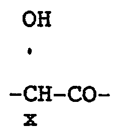 Gruppe und B eine Methylengruppe, wobei das mit x gekennzeichnete Atom jeweils mit dem Phenylkern verknüpft ist,
E eine gegebenenfalls durch eine Alkylgruppe mit 1 bis 3 Kohlenstoffatomen substituierte geradkettige Alkylengruppe mit 1 bis 3 Kohlenstoffatomen,
G eine gegebenenfalls durch eine Alkylgruppe mit 1 bis 3 Kohlenstoffatomen substituierte geradkettige Alkylengruppe mit 1 bis 5 Kohlenstoffatomen, wobei die zu dem Phenylkern benachbarte Methylengruppe einer gegebenenfalls durch eine Alkylgruppe substituierten geradkettigen Alkylengruppe mit 3 bis 5 Kohlenstoffatomen durch ein Sauerstoff- oder Schwefelatom, eine Imino-, Methylimino-, Sulfinyl-oder Sulfonylgruppe ersetzt sein kann,
R₁ ein Wasserstoff- oder Halogenatom, eine Trifluormethyl-, Nitro-, Amino-, Alkylamino-, Dialkylamino-, Alkyl-, Hydroxy-, Alkoxy- oder Phenylalkoxygruppe, wobei jeder Alkylteil jeweils bis 3 Kohlenstoffatome enthalten kann,
R₂ ein Wasserstoffatom oder Halogenatom, eine Hydroxy-, Alkoxy-, Phenylalkoxy- oder Alkylgruppe, wobei jeder Alkylteil jeweils I bis 3 Kohlenstoffatome enthalten kann, oder
R₁ und R₂ zusammen eine Alkylendioxygruppe mit I oder 2 Kohlenstoffatomen,
R₃ ein Wasserstoff- oder Halogenatom, eine Alkyl- oder Alkoxygruppe mit jeweils I bis 3 Kohlenstoffatomen im Alkylteil, eine Hydroxy-, Nitro-, Cyano- oder Trifluormethylgruppe,
R₄ ein Wasserstoffatom, eine Alkoxy-, Alkansulfonyloxy-, Amino-, Alkylamino- oder Dialkylaminogruppe mit jeweils I bis 3 Kohlenstoffatomen in jedem Alkylteil, oder eine Alkanoylaminogruppe mit 2 oder 3 Kohlenstoffatomen im Alkanoylteil oder
R₃ und R₄ zusammen eine Alkylendioxygruppe mit I oder 2 Kohlenstoffatomen,
Rₛ ein Wasserstoff- oder Halogenatom, eine Hydroxy-, Alkyl- oder Alkoxygruppe mit jeweils I bis 3 Kohlenstoffatomen im Alkylteil,
m die Zahl I, 2, 3, 4 oder 5 und
n die Zahl 0, I oder 2, wobei jedoch n + m die Zahl 3, 4 oder 5 darstellen muß.

Für die bei der Definition der Reste eingangs erwähnten Bedeutungen kommt beispielsweise für R₁ die des Wasserstoff-, Fluor-, Chlor- oder Bromatoms, der Methyl-, Ethyl-, n-Propyl-, Isopropyl- , Trifluormethyl-, Hydroxy-, Methoxy-, Ethoxy-, n-Propoxy-, Isopropoxy-, Nitro-, Amino-, Methylamino- , Ethylamino-, n-Propylamino-, Isopropylamino-, Dimethylamino-, Diethylamino-, Di-n-propylamino-, Diisopropylamino-, Methyl-ethylamino-, Methyl-n-propylämino-, Methyl-isopropylamino-, Ethyl-n-propylamino-, Benzyloxy-, I-Phenylethoxy-, I-Phenylpropoxy-, 2-Phenylethoxy- oder 3-Phenylpropoxygruppe, für R₂ die des Wasserstoff-, Chlor- oder Bromatoms, der Methyl-, Ethyl-, n-Propyl-, Isopropyl-, Hydroxy-, Methoxy-, Ethoxy-, n-Propoxy-, Isopropoxy-, Benzyloxy-, I-Phenylethoxy-, 2-Phenylethoxy-, 2-Phenylpropoxy- oder 3-Phenylpropoxygruppe oder zusammen mit R₁ die der Methylendioxy- oder Ethylendioxygruppe,
für R₃ die des Wasserstoff-, Fluor-, Chlor- oder Bromatoms, der Methyl-, Ethyl-, n-Propyl-, Isopropyl- , Hydroxy-, Methoxy-, Ethoxy-, n-Propoxy-, Isopropoxy-, Nitro-, Cyano- oder Trifluormethylgruppe,
für R₄ die des Wasserstoffatoms, der Methoxy-, Ethoxy-, n-Propoxy-, Isopropoxy-, Methansulfonyloxy-, Ethansulfonyloxy-, n-Propansulfonyloxy-, Amino-, Methylamino-, Ethylamino-, n-Propylamino-, Isopropylamino-, Dimethylamino-, Diethylamino-, Di-n-propylamino-, Diisopropylamino-, Methylethylamino-, Methyl-n-propylamino-, Methyl-isopropylamino-, Ethyl-n-propylamino-, Acetylamino- oder Propionylaminogruppe oder zusammen mit R₃ die der Methylendioxy- oder Ethylendioxygruppe,
für R₅ die des Wasserstoff-, Chlor- oder Bromatoms, der Methyl-, Ethyl-, n-Propyl-, Isopropyl-, Hydroxy-, Methoxy-, Ethoxy-, n-Propoxy- oder Isopropoxygruppe,
für E die der Methylen-, Äthylen-, n-Propylen-, Ethyliden-, n-Propyliden-, n-Butyliden-, 2-Methyl-n-propyliden-, I-Methyl-ethylen-, I-Ethyl-ethylen-, 2-Methyl-ethylen-, 2-Ethyl-ethylen-, I-n-Propyl-ethylen-, I-Methyl-n-propylen, 2-Methyl-n-propylen-, 3-Methyl-n-propylen-, I-Ethyl-n-propylen-, 2-n-Propyl-n-propylen- oder 3-Ethyl-n-propylengruppe und
für G die der Methylen-, Ethyliden-, n-Propyliden-, n-Butyliden-, 2-Methyl-propyliden-, Ethylen-, I-Methyl-ethylen-, I-Ethyl-ethylen-, I-Propyl-ethylen-, 2-Methyl-ethylen-, 2-Ethyl-ethylen-, n-Propylen-, n-Butylen-, n-Pentylen-, I-Methyl-n-propylen-, I-Methyl-n-butylen-, I-Methyl-n-pentylen, I-Ethyl-n-propylen-, 2-Ethyl-n-propylen-, I-Ethyl-n-butylen-, Ethylenoxy-, n-Propylenoxy-, n-Butylenoxy-, Ethylenthio-, n-Propylenthio-, n-Butylenthio-, Ethylensulfinyl-, Ethylensulfonyl-, n-Propylensulfinyl-, n-Propylensulfonyl-, n-Butylensulfinyl-, Ethylenamino-, n-Propylenamino-, n-Butylenamino-, N-Methylethylenamino-, N-Methyl-n-propylenamino- oder N-Methyl-n-butylenaminogruppe in Betracht.

Erfindungsgemäß fallen somit folgende Verbindungen unter die vorstehend genannte allgemeine Formel I:
3-[(N-(2-(3,4-Dimethoxy-phenyl)-ethyl)-piperidin-3-yl)-methyl]-7,8-dimethoxy-1,3,4,5-tetrahydro-2H-3- benzazepin-2-on
3-[(N-(2-(3,4-Dimethoxy-phenyl)-ethyl)-piperidin-3-yl)-methyl]-7,8-dimethoxy-1,3-dihydro-2H-3-benzazepin-2-on
3-[(N-(2-(3,4-Dimethoxy-phenyl)-ethyl)-piperidin-3-yl)-methyl]-7,8-dimethoxy-2,3,4,5-tetrahydro-IH-3- benzazepin
3-[(N-(2-(3,4-Dimethoxy-phenyl)-ethyl)-piperidin-3-yl)-methyl]-7,8-dimethoxy-1,3,4,5-tetrahydro-2H-3- benzazepin-2-thion
3-[(N-(2-(3,4-Dimethoxy-phenyl)-ethyl)-piperidin-3-yl)-methyl]-7,8-dimethoxy-1,3,4,5-tetrahydro-2H-3- benzazepin-1,2-dion
3-[(N-(2-(3,4-Dimethoxy-phenyl)-ethyl)-piperidin-3-yl)-methyl]-7,8-dimethoxy-1-hydroxy-1,3,4,5-te- trahydro-2H-3-benzazepin-2-on
3-[(N-(2-(4-Amino-phenyl)-ethyl)-piperidin-3-yl)-methyl]-7,8-dimethoxy-1,3,4,5-tetrahydro-2H-3-benzazepin-2-on
3-[(N-(2-(4-Acetamino-phenyl)-ethyl)-piperidin-3-yl)-methyl]-7,8-dimethoxy-1,3,4,5-tetrahydro-2H-3- benzazepin-2-on
3-[(N-(3-(4-Amino-3,5-dibrom-phenoxy)-propyl)-piperidin-3-yl)-methyl]-7,8-dimethoxy-1,3,4,5-tetrahy- dro-2H-3-benzazepin-2-on
3-[(N-(2-(3,4-Dimethoxy-phenyl)-ethyl)-piperidin-3-yl)-methyl]-7,8-methylendioxy-1,3,4,5-tetrahydro-2H-3-benzazepin-2-on
3-[(N-(3,4-Dimethoxy-benzyl)-piperidin-3-yl)-methyl]-7,8-methylendioxy-1,3,4,5-tetrahydro-2H-3-benzazepin-2-on
3-[(N-(2-Phenylethyl)-piperidin-3-yl)-methyl]-7,8-dimethoxy-1,3,4,5-tetrahydro-2H-3-benzazepin-2-on 3-[(N-(2-(3-Nitro-4-acetamino-phenyl)-ethyl)-piperidin-3-yl)-methyl]-7,8-dimethoxy-1,3,4,5-tetrahydro-2H-3-benzazepin-2-on
3-[(N-(2-(3,4,5-Trimethoxy-phenyl)-ethyl)-piperidin-3-yl)-methyl]-7,8-dimethoxy-1,3,4,5-tetrahydro-2H-3-benzazepin-2-on
3-[(N-(3-(4-Methoxy-phenyl)-propyl)-piperidin-3-yl)-methyl]-7,8-dimethoxy-1,3,4,5-tetrahydro-2H-3- benzazepin-2-on
3-[(N-(2-(4-Methoxy-phenyl)-ethyl)-piperidin-3-yl)-methyl]-7,8-dimethoxy-1,3,4,5-tetrahydro-2H-3- benzazepin-2-on
3-[(N-(2-(4-Nitro-phenyl)-ethyl)-piperidin-3-yl)-methyl]-7,8-dimethoxy-1,3,4,5-tetrahydro-2H-3-benzazepin-2-on
3-[(N-(2-(3-Methyl-phenyl)-ethyl)-piperidin-3-yl)-methyl]-7,8-dimethoxy-1,3,4,5-tetrahydro-2H-3-benzazepin-2-on
3-[(N-(2-(3-Methoxy-phenyl)-ethyl)-piperidin-3-yl)-methyl]-7,8-dimethoxy-1,3,4,5-tetrahydro-2H-3- benzazepin-2-on
3-[(N-(2-(4-Methyl-phenyl)-ethyl)-piperidin-3-yl)-methyl]-7,8-dimethoxy-1,3,4,5-tetrahydro-2H-3-benzazepin-2-on
3-[(N-(3-(4-Brom-phenyl)-propyl)-piperidin-3-yl)-methyl]-7,8-dimethoxy-1,3,4,5-tetrahydro-2H-3-benzazepin-2-on
3-[(N-(2-(3,4-Dimethoxy-phenyl)-ethyl)-piperidin-3-yl)-methyl]-7,8-dimethoxy-1,3-dihydro-2H-3-benzazepin-2-on
3-[(N-(2-(3,4-Dimethoxy-phenyl)-ethyl)-piperidin-2-yl)-ethyl-2]-7,8-dimethoxy-1,3,4,5-tetrahydro-2H-3-benzazepin-2-on
3-[(N-(2-(3,4-Dimethoxy-phenyl)-ethyl)-hexahydro-azepin-2-yl)-ethyl-2]-7,8-dimethoxy-1,3,4,5-te- trahydro-2H-3-benzazepin-2-on
3-[(N-(3-(4-Amino-3,5-dibrom-phenoxy)-propyl)-hexahydro-azepin-3-yl)-methyl]-7,8-dimethoxy-1,3,4,5-tetrahydro-2H-3-benzazepin-2-on
3-[(N-(2-(3,4-Dimethoxy-phenyl)-ethyl)-hexahydro-azepin-2-yl)-ethyl-2]-7,8-dimethoxy-1,3-dihydro-2H-3-benzazepin2-on
3-[(N-(2-(3,4-Methylendioxy-phenyl)-ethyl)-piperidin-3-yl)-methyl]-7,8-methylendioxy-1,3,4,5-tetrahy- dro-2H-3-benzazepin-2-on
3-[(N-(3,4-Dichlor-benzyl)-piperidin-3-yl)-methyl]-7,8-methylendioxy-1,3,4,5-tetrahydro-2H-3-benzazepin-2-on
3-[(N-(2-(3,4-Dimethoxy-phenyl)-ethyl)-pyrrolidin-3-yl)-methyl]-7,8-dimethoxy-1,3-dihydro-2H-3-benzazepin-2-on
3-[(N-(2-(3,4-Dimethoxy-phenyl)-ethyl)-pyrrolidin-3-yl)-methyl]-7,8-dimethoxy-1,3,4,5-tetrahydro-2H-3-benzazepin-2-on
3-[(N-(3-(3-Methoxy-phenoxy)-propyl)-piperidin-3-yl)-methyl]-7,8-methylendioxy-1,3,4,5-tetrahydro-2H-3-benzazepin-2-on
3-[(N-(3-(3-Methyl-phenoxy)-propyl)-piperidin-3-yl)-methyl]-7,8-methylendioxy-1,3,4,5-tetrahydro-2H-3-benzazepin-2-on
3-[(N-(2-(4-Amino-3,5-dichlor-phenyl)-ethyl)-piperidin-3-yl)-methyl]-7,8-dimethoxy-1,3,4,5-tetrahydro-2H-3-benzazepin-2-on
3-[(N-(3-(3,4-Methylendioxy-phenoxy)-propyl)-piperidin-3-yl)-methyl]-7,8-methylendioxy-1,3,4,5-te- trahydro-2H-3-benzazepin-2-on
3-[(N-(4-(4-Methoxy-phenyl)-butyl)-piperidin-3-yl)-methyl]-7,8-methylendioxy-1,3,4,5-tetrahydro-2H-3-benzazepin-2-on
3-[(N-(2-(4-Methoxy-phenyl)-ethyl)-piperidin-3-yl)-methyl]-7,8-methylendioxy-1,3,4,5-tetrahydro-2H-3-benzazepin-2-on
3-[(N-(2-(Phenoxy)-ethyl)-piperidin-3-yl)-methyl]-7,8-methylendioxy-1,3,4,5-tetrahydro-2H-3-benzazepin-2-on
3-[(N-(2-(4-Methoxy-phenyl)-ethyl)-piperidin-2-yl)-ethyl-2]-7,8-methylendioxy-1,3,4,5-tetrahydro-2H-3-benzazepin-2-on
3-[(N-(4-Methoxy-phenyl)-methyl)-piperidin-2-yl)-ethyl-2]-7,8-methylendioxy-1,3,4,5-tetrahydro-2H-3- benzazepin-2-on
3-[(N-(3,4-Dimethoxy-phenyl)-methyl)-piperidin-2-yl)-ethyl-2]-7,8-methylendioxy-1,3,4,5-tetrahydro-2H-3-benzazepin-2-on
3-[(N-(2-(4-Nitro-phenyl)-ethyl)-piperidin-2-yl)-ethyl-2]-7,8-methylendioxy-1,3,4,5-tetrahydro-2H-3- benzazepin-2-on
3-[(N-(2-(3-Trifluormethylphenyl)-ethyl)-piperidin-3-yl)-methyl]-7,8-dimethoxy-1,3,4,5-tetrahydro-2H-3-benzazepin-2-on
3-[(N-(3-(3,5-Dimethoxy-phenoxy)-propyl)-piperidin-3-yl)-methyl]-7,8-methylendioxy-1,3,4,5-tetrahy- dro-2H-3-benzazepin-2-on
3-[(N-(2-(3-Methoxy-4-methansulfonyloxy-phenyl)-ethyl)-piperidin-3-yl)-methyl]-7,8-dimethoxy-1,3,4,5-tetrahydro-2H-3-benzazepin-2-on
3-[(N-(2-(4-Benzyloxy-3-methoxy-phenyl)-ethyl)-piperidin-3-yl)-methyl]-7,8-dimethoxy-1,3,4,5-tetrahy- dro-2H-3-benzazepin-2-on
3-[N-(2-(2-Fluorphenyl)-ethyl)-piperidin-3-yl)-methyl]-7,8-dimethoxy-1,3,4,5-tetrahydro-2H-3-benzazepin-2-on
3-[N-(2-(4-Fluorphenyl)-ethyl)-piperidin-3-yl)-methyl]-7,8-dimethoxy-1,3,4,5-tetrahydro-2H-3-benzazepin-2-on
3-[(N-(2-(3,4-Dimethoxy-phenyl)-ethyl)-hexahydro-azepin-2-yl)-ethyl-2]-1,3,4,5-tetrahydro-2H-3-benzazepin-2-on
3-[(N-(2-(4-Amino-phenyl)-ethyl)-piperidin-2-yl)-ethyl-2]-7,8-dimethoxy-1,3,4,5-tetrahydro-2H-3-benzazepin-2-on
3-[(N-(3-(4-Amino-3,5-dibrom-phenoxy)-propyl)-piperidin-2-yl)-ethyl-2]-7,8-dimethoxy-1,3,4,5-tetrahy- dro-2H-3-benzazepin-2-on
3-[(N-(3,4-Dimethoxy-benzyl)-piperidin-2-yl)-ethyl-2]-7,8-dimethoxy-1,3,4,5-tetrahydro-2H-3-benzazepin-2-on
3-[(N-(2-Phenylethyl)-piperidin-2-yl)-ethyl-2]-7,8-dimethoxy-1,3,4,5-tetrahydro-2H-3-benzazepin-2-on
3-[(N-(2-(3,4,5-Trimethoxy-phenyl)-ethyl)-piperidin-2-yl)-ethyl-2]-7,8-dimethoxy-1,3,4,5-tetrahydro-2H-3-benzazepin-2-on
3-[(N-(3-(4-Methoxy-phenyl)-propyl)-piperidin-2-yl)-ethyl-2]-7,8-dimethoxy-1,3,4,5-tetrahydro-2H-3- benzazepin-2-on
3-[(N-(2-(4-Methoxy-phenyl)-ethyl)-piperidin-2-yl)-ethyl-2]-7,8-dimethoxy-1,3,4,5-tetrahydro-2H-3- benzazepin-2-on
3-[(N-(2-(4-Nitro-phenyl)-ethyl)-piperidin-2-yl)-ethyl-2]-7,8-dimethoxy-1,3,4,5-tetrahydro-2H-3-benzazepin-2-on
3-[(N-(2-(3-Methyl-phenyl)-ethyl)-piperidin-2-yl)-ethyl-2]-7,8-dimethoxy-1,3,4,5-tetrahydro-2H-3-benzazepin-2-on
3-[(N-(2-(3-Methoxy-phenyl)-ethyl)-piperidin-2-yl)-ethyl-2]-7,8-dimethoxy-1,3,4,5-tetrahydro-2H-3- benzazepin-2-on
3-[(N-(2-(4-Methyl-phenyl)-ethyl)-piperidin-2-yl)-ethyl-2]-7,8-dimethoxy-1,3,4,5-tetrahydro-2H-3- benzazepin-2-on
3-[(N-(3-(4-Brom-phenyl)-propyl)-piperidin-2-yl)-ethyl-2]-7,8-dimethoxy-1,3,4,5-tetrahydro-2H-3-benzazepin-2-on
3-[(N-(2-(3,4-Dimethoxy-phenyl)-ethyl)-piperidin-2-yl)-ethyl-2]-7,8-dimethoxy-1,3,4,5-tetrahydro-2H-3-benzazepin-2-on
3-[(N-(2-(3,4-Dimethoxy-phenyl)-ethyl)-piperidin-3-yl)-methyl]-7-methoxy-1,3,4,5-tetrahydro-2H-3- benzazepin-2-on
3-[(N-(2-(3,4-Dimethoxy-phenyl)-ethyl)-piperidin-3-yl)-methyl]-7-trifluormethyl-1,3,4,5-tetrahydro-2H-3-benzazepin-2-on
3-[(N-(2-(3,4-Dimethoxy-phenyl)-ethyl)-piperidin-3-yl)-methyl]-7-methylamino-1,3,4,5-tetrahydro-2H-3-benzazepin-2-on
3-[(N-(2-(3,4-Dimethoxy-phenyl)-ethyl)-piperidin-3-yl)-methyl]-7-dimethylamino-1,3,4,5-tetrahydro-2H-3-benzazepin-2-on
3-[(N-(2-(3,4-Dimethoxy-phenyl)-ethyl)-piperidin-3-yl)-methyl]-7,8-dichlor-1,3,4,5-tetrahydro-2H-3- benzazepin-2-on
3-[(N-(2-(3,4-Dimethoxy-phenyl)-ethyl)-piperidin-3-yl)-methyl]-7-methylamino-8-methoxy-1,3,4,5-te- trahydro-2H-3-benzazepin-2-on
3-[(N-(2-(3,4-Dimethoxy-phenyl)-ethyl)-piperidin-3-yl)-methyl]-7-brom-8-methoxy-1,3,4,5-tetrahydro-2H-3-benzazepin-2-on
3-[(N-(2-(3,4-Dimethoxy-phenyl)-ethyl)-piperidin-3-yl)-methyl]-7-chlor-8-methoxy-1,3,4,5-tetrahydro-2H-3-benzazepin-2-on
3-[(N-(2-(3,4-Dimethoxy-phenyl)-ethyl)-piperidin-3-yl)-methyl]-7-hydroxy-8-methoxy-1,3,4,5-tetrahy- dro-2H-3-benzazepin-2-on
3-[(N-(2-Phenylethyl)-piperidin-3-yl)-methyl]-7-methoxy-1,3,4,5-tetrahydro-2H-3-benzazepin-2-on
3-[(N-(2-Phenylethyl)-piperidin-3-yl)-methyl]-7-trifluormethyl-1,3,4,5-tetrahydro-2H-3-benzazepin-2- on
3-[(N-(2-Phenylethyl)-piperidin-3-yl)-methyl]-7-methylamino-1,3,4,5-tetrahydro-2H-3-benzazepin-2-on
3-[(N-(2-Phenylethyl)-piperidin-3-yl)-methyl]-7-dimethylamino-1,3,4,5-tetrahydro-2H-3-benzazepin-2- on
3-[(N-(2-Phenylethyl)-piperidin-3-yl)-methyl]-7,8-dichlor-1,3,4,5-tetrahydro-2H-3-benzazepin-2-on
3-[(N-(2-Phenylethyl)-piperidin-3-yl)-methyl]-7-methylamino-8-methoxy-1,3,4,5-tetrahydro-2H-3-benzazepin-2-on
3-[(N-(2-Phenylethyl)-piperidin-3-yl)-methyl]-7-brom-8-methoxy-1,3,4,5-tetrahydro-2H-3-benzazepin-2-on
3-[(N-(2-Phenylethyl)-piperidin-3-yl)-methyl]-7-chlor-8-methoxy-1,3,4,5-tetrahydro-2H-3-benzazepin-
3-[(N-(2-Phenylethyl)-piperidin-3-yl)-methyl]-7-hydroxy-8-methoxy-1,3,4,5-tetrahydro-2H-3-benzazepin-2-on
3-[(N-(3-(3,4-Dimethoxy-phenyl)-propyl)-piperidin-3-yl)-methyl]-7,8-dimethoxy-1,3,4,5-tetrahydro-2H-1,3-benzodiazepin-2-on
3-[(N-(2-(3,4-Dimethoxy-phenyl)-ethyl)-piperidin-3-yl)-methyl]-7,8-dimethoxy-1,3,4,5-tetrahydro-2H-1,3-benzodiazepin-2-on
3-[(N-(2-(3,4-Methylendioxy-phenyl)-ethyl)-piperidin-3-yl)-methyl]-7,8-dimethoxy-1,3,4,5-tetrahydro-2H-1,3-benzodiazepin-2-on
3-[(N-(2-(3-Methoxy-phenyl)-ethyl)-piperidin-3-yl)-methyl]-7,8-dimethoxy-1,3,4,5-tetrahydro-2H-1,3-benzodiazepin-2-on
3-[(N-(2-(4-Methoxy-phenyl)-ethyl)-piperidin-3-yl)-methyl]-7,8-dimethoxy-1,3,4,5-tetrahydro-2H-1,3-benzodiazepin-2-on
3-[(N-(2-(2-Methoxy-phenyl)-ethyl)-piperidin-3-yl)-methyl]-7,8-dimethoxy-1,3,4,5-tetrahydro-2H-1,3-benzodiazepin-2-on
3-[(N-(2-(N-(3,4-Dimethoxy-phenyl)-methylamino)-ethyl))-piperidin-3-yl)-methyl]-7,8-dimethoxy-1,3,4,5-tetrahydro-2H-1,3-benzodiazepin-2-on
3-[(N-(3-(3,4-Dimethoxy-phenylthio)-propyl)-piperidin-3-yl)-methyl]-7,8-dimethoxy-1,3,4,5-tetrahydro-2H-3-benzazepin-2-on
3-[(N-(2-Phenylthioethyl)-piperidin-3-yl)-methyl]-7,8-dimethoxy-1,3,4,5-tetrahydro-2H-3-benzazepin-2-on
3-[(N-(2-Phenylaminoethyl)-piperidin-3-yl)-methyl]-7,8-dimethoxy-1,3,4,5-tetrahydro-2H-3-benzazepin-2-on
3-[(N-(2-Phenoxyethyl)-piperidin-3-yl)-methyl]-7,8-dimethoxy-1,3,4,5-tetrahydro-2H-3-benzazepin-2- on
3-[(N-(2-(3,5-Dichlor-4-methoxy-phenoxy)-ethyl)-piperidin-3-yl)-methyl]-7,8-dimethoxy-1,3,4,5-te- trahydro-2H-3-benzazepin-2-on
3-[(N-(2-(3,4-Dimethoxy-phenylamino)-ethyl)-piperidin-3-yl)-methyl]-7,8-dimethoxy-1,3,4,5-tetrahydro-2H-3-benzazepin-2-on
3-[(N-(3-(3,4-Dimethoxy-phenylsulfinyl)-propyl)-piperidin-3-yl)-methyl]-7,8-dimethoxy-1,3,4,5-tetrahy- dro-2H-3-benzazepin-2-on
3-[(N-(3-(3,4-Dimethoxy-phenylsulfonyl)-propyl)-piperidin-3-yl)-methyl]-7,8-dimethoxy-1,3,4,5-tetrahy- dro-2H-3-benzazepin-2-on
3-[(N-(3-(4-Dimethylamino-phenoxy)-propyl)-piperidin-3-yl)-methyl]-7,8-dimethoxy-1,3,4,5-tetrahydro-2H-3-benzazepin-2-on
3-[(N-(2-(4-Amino-3,5-dibrom-phenylsulfonyl)-ethyl)-piperidin-3-yl)-methyl]-7,8-dimethoxy-1,3,4,5-te- trahydro-2H-3-benzazepin-2-on
3-[(N-(2-(4-Amino-3,5-dibrom-phenylsulfinyl)-ethyl)-piperidin-3-yl)-methyl]-7,8-dimethoxy-1,3,4,5-te- trahydro-2H-3-benzazepin-2-on
3-[(N-(2-(4-Amino-3,5-dibrom-phenylthio)-ethyl)-piperidin-3-yl)-methyl]-7,8-dimethoxy-1,3,4,5-tetrahy- dro-2H-3-benzazepin-2-on
3-[(N-(2-(3,4-Dichlor-phenoxy)-ethyl)-piperidin-3-yl)-methyl]-7,8-dimethoxy-1,3,4,5-tetrahydro-2H-3- benzazepin-2-on
3-[(N-(2-(3,4-Dimethoxy-phenoxy)-ethyl)-piperidin-3-yl)-methyl]-7,8-dimethoxy-1,3,4,5-tetrahydro-2H-3-benzazepin-2-on
3-[(N-(3-(N-Phenyl-N-methyl-amino)-propyl)-piperidin-3-yl)-methyl]-7,8-dimethoxy-1,3,4,5-tetrahydro-2H-3-benzazepin-2-on
3-[(N-(2-(4-Methoxy-phenoxy)-ethyl)-piperidin-3-yl)-methyl]-7,8-dimethoxy-1,3,4,5-tetrahydro-2H-3- benzazepin-2-on
3-[(N-(2-(3,4-Methylendioxy-phenoxy)-ethyl)-piperidin-3-yl)-methyl]-7,8-dimethoxy-1,3,4,5-tetrahydro-2H-3-benzazepin-2-on
3-[(N-(3-(4-Amino-3,5-dichlor-phenylamino)-propyl)-piperidin-3-yl)-methyl]-7,8-dimethoxy-1,3,4,5-te- trahydro-2H-3-benzazepin-2-on
3-[(N-(2-(4-Hydroxy-3-methoxy-phenyl)-ethyl)-piperidin-3-yl)-methyl]-7,8-dimethoxy-1,3,4,5-tetrahy- dro-2H-3-benzazepin-2-on
3-[(N-(2-(3,4-Dimethoxy-phenyl)-ethyl)-pyrrolidin-3-yl)-methyl]-7,8-dimethoxy-1,3,4,5-tetrahydro-2H-3-benzazepin-2-on
3-[(N-(2-(3,4-Dimethoxy-phenyl)-ethyl)-pyrrolidin-2-yl)-ethyl-2]-7,8-dimethoxy-1,3,4,5-tetrahydro-2H-3-benzazepin-2-on
3-[(N-(3-(4-Amino-3,5-dibrom-phenoxy)-propyl)-pyrrolidin-3-yl)-methyl]-7,8-dimethoxy-1,3,4,5-te- trahydro-2H-3-benzazepin-2-on
3-[(N-(3-(4-Methoxy-phenyl)-propyl)-pyrrolidin-3-yl)-methyl]-7,8-dimethoxy-1,3,4,5-tetrahydro-2H-3- benzazepin-2-on
3-[(N-(2-(4-Methoxy-phenyl)-ethyl)-pyrrolidin-3-yl)-methyl]-7,8-dimethoxy-1,3,4,5-tetrahydro-2H-3- benzazepin-2-on
3-[(N-(2-(3-Methyl-phenyl)-ethyl)-pyrrolidin-3-yl)-methyl]-7,8-dimethoxy-1,3,4,5-tetrahydro-2H-3- benzazepin-2-on
3-[(N-(2-(3-Methoxy-phenyl)-ethyl)-pyrrolidin-3-yl)-methyl]-7,8-dimethoxy-1,3,4,5-tetrahydro-2H-3- benzazepin-2-on
3-[(N-(2-(4-Nitro-phenyl)-ethyl)-pyrrolidin-3-yl)-methyl]-7,8-dimethoxy-1,3,4,5-tetrahydro-2H-3-benzazepin-2-on
3-[(N-(3-(4-Brom-phenyl)-propyl)-pyrrolidin-3-yl)-methyl]-7,8-dimethoxy-1,3,4,5-tetrahydro-2H-3-benzazepin-2-on
3-[(N-(3-(4-Brom-phenyl)-propyl)-hexahydro-azepin-3-yl)-methyl]-7,8-dimethoxy-1,3,4,5-tetrahydro-2H-3-benzazepin-2-on
3-[(N-(2-(4-Nitro-phenyl)-ethyl)-hexahydro-azepin-3-yl)-methyl]-7,8-dimethoxy-1,3,4,5-tetrahydro-2H-3-benzazepin-2-on
3-[(N-(2-(4-Methoxy-phenyl)-ethyl)-hexahydro-azepin-3-yl)-methyl]-7,8-dimethoxy-1,3,4,5-tetrahydro-2H-3-benzazepin-2-on
3-[(N-(2-(4-Methyl-phenyl)-ethyl)-hexahydro-azepin-3-yl)-methyl]-7,8-dimethoxy-1,3,4,5-tetrahydro-2H-3-benzazepin-2-on
3-[(N-(2-Phenylethyl)-hexahydro-azepin-3-yl)-methyl]-7,8-dimethoxy-1,3,4,5-tetrahydro-2H-3-benzazepin-2-on
3-[(N-(2-Phenylethyl)-hexahydro-azepin-2-yl)-ethyl-2]-7,8-dimethoxy-1,3,4,5-tetrahydro-2H-3-benzazepin-2-on
3-[(N-(2-(3-Methyl-phenyl)-ethyl)-hexahydro-azepin-2-yl)-ethyl-2]-7,8-methylendioxy-1,3,4,5-tetrahy- dro-2H-3-benzazepin-2-on
3-[(N-(2-(4-Fluor-phenyl)-ethyl)-hexahydro-azepin-2-yl)-ethyl-2]-7,8-methylendioxy-1,3,4,5-tetrahy- dro-2H-3-benzazepin-2-on
   deren Enantiomere, deren Diastereomere und deren Säureadditionssalze.

Bevorzugte Verbindungen der obigen allgemeinen Formel sind jedoch diejenigen, in denen
A, B, m und n wie eingangs definiert sind, wobei jedoch m + n die Zahl 3, 4 oder 5 darstellen muß,
E eine Methylen- oder Ethylengruppe,
G eine n-Alkylengruppe mit 1 bis 4 Kohlenstoffatomen, wobei die zu dem Phenylkern benachbarte Methylengruppe einer n-Propylen- oder n-Butylengruppe durch ein Sauerstoff- oder Schwefelatom, eine Imino-, Methylimino-, Sulfinyl- oder Sulfonylgruppe ersetzt sein kann,
R₁ ein Wasserstoff-, Fluor-, Chlor- oder Bromatom, eine Hydroxy-, Methoxy-, Trifluormethyl-, Methylamino- oder Dimethylaminogruppe,
R₂ ein Wasserstoff-, Chlor- oder Bromatom oder eine Methoxygruppe oder
R₁ und R₂ zusammen eine Methylendioxygruppe,
R₃ ein Wasserstoff-, Fluor-, Chlor- oder Bromatom, eine Methyl-, Hydroxy-, Methoxy- oder Nitrogruppe,
R₄ ein Wasserstoffatom, eine Methoxy-, Methansulfonyloxy-, Amino- oder Acetylaminogruppe oder
R₃ und R₄ zusammen eine Methylendioxygruppe und
Rs ein Wasserstoff-, Chlor- oder Bromatom oder eine Methoxygruppe.

Besonders bevorzugte Verbindungen der allgemeinen Formel sind jedoch diejenigen, in denen m und n wie eingangs definiert sind, wobei jedoch m + n die Zahl 3, 4 oder 5 darstellen muß,
A eine -CH2-CH2- oder -CH=CH-Gruppe und B eine Methylen- oder Carbonylgruppe oder
A eine -CO-CO-Gruppe und B eine Methylengruppe,
E eine Methylen- oder Ethylengruppe,
G eine n-Alkylengruppe mit 2 bis 4 Kohlenstoffatomen, wobei die zu dem Phenylkern benachbarte Methylengruppe einer n-Propylen- oder n-Butylengruppe durch ein Sauerstoffatom ersetzt sein kann,
R₁ ein Wasserstoffatom oder eine Methoxygruppe,
R₂ ein Wasserstoffatom oder eine Methoxygruppe oder
Ri und R₂ zusammen eine Methylendioxygruppe,
R₃ ein Wasserstoffatom, eine Methyl-, Hydroxy- oder Methoxygruppe,
R₄ ein Wasserstoffatom oder eine Methoxygruppe oder
R₃ und R₄ zusammen eine Methylendioxygruppe und
Rs ein Wasserstoffatom bedeuten, deren Enantiomere, deren Diastereomere und deren Säureadditionssalze.

Erfindungsgemäß erhält man die neuen Verbindungen der allgemeinen Formel nach folgenden Verfahren:
a.) Umsetzung einer Verbindung der allgemeinen Formel 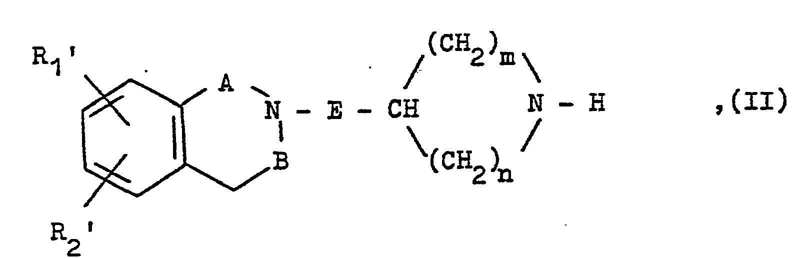 in der
A, B, E, m und n wie eingangs definiert sind,
Rᵢ' einen durch einen Schutzrest geschützte Hydroxy-, Amino- oder Alkylaminogruppe darstellt oder die für R₁ eingangs erwähnten Bedeutungen besitzt und
R₂' einen durch einen Schutzrest geschützte Hydroxygruppe darstellt oder die für R₂ eingangs erwähnten Bedeutungen besitzt, mit einer Verbindung der allgemeinen Formel 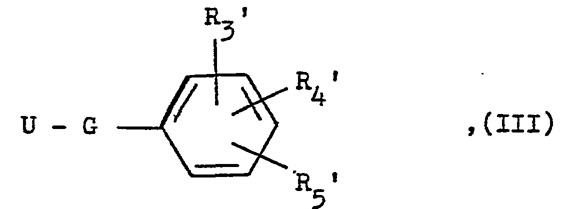 in der
Rs', R₄' und Rs' die für Rs, R₄ und R₅ eingangs erwänten Bedeutungen besitzen, wobei jedoch die in den Resten R₃ bis R₅ enthaltenen Hydroxy-, Amino- oder Alkylaminogruppen durch einen Schutzrest geschützt sein können, und
U eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, darstellt und gegebenenfalls anschließende Abspaltung eines verwendeten Schutzrestes.

Als Schutzrest für eine Hydroxygruppe kommt beispielsweise die Trimethylsilyl-, Acetyl-, Benzoyl-, Benzyl- oder Tetrahydropyranylgruppe und
als Schutzrest für eine Amino- oder Alkylaminogruppe die Acetyl-, Benzoyl-, Ethoxycarbonyl- oder Benzylgruppe in Betracht.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuß der eingesetzten Verbindungen der allgemeinen Formeln II und/oder 111 und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150_{°}C, vorzugsweise bei Temperaturen zwischen 50 und 120_{°}C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel III durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100_{°}C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von I bis 7 bar, vorzugsweise jedoch von 3 bis 5 bar.

b.) Zur Herstellung von Verbindungen der allgemeinen Formel I, in der G mit Ausnahme der eine Sulfenyl-, Sulfinyl- oder Sulfonylgruppe enthaltenden Reste die für G eingangs erwähnten Bedeutungen besitzt, A die -CH₂-CH₂-Gruppe, B die Methylen- oder Carbonylgruppe und m + n die Zahl 4 darstellen:

Hydrierung einer Verbindung der allgemeinen Formel 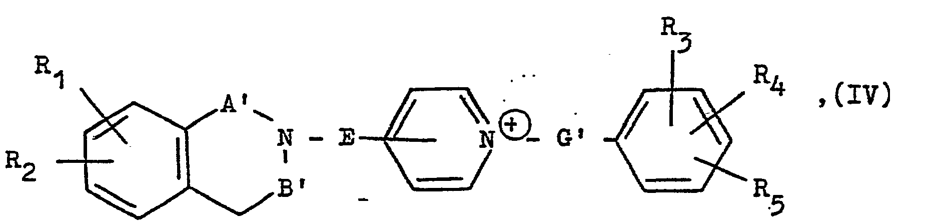 in der
Rᵢ bis R₅ und E wie eingangs definiert sind,
G' mit Ausnahme der ein Schwefelatom, eine Sulfinyl- oder Sulfonylgruppe enthaltenden Reste die für G eingangs erwähnten Bedeutungen besitzt,
A' die -CH=CH- oder -CH₂CH₂-Gruppe und
B' die Methylen- oder Carbonylgruppe darstellen.

Die Hydrierung wird vorzugsweise in einem Lösungsmittel oder Lösungsmittelgemisch wie Methanol, Ethanol, Essigsäureethylester oder Eisessig vorzugsweize mit katalytisch angeregtem Wasserstoff, z.B. mit Wasserstoff in Gegenwart von Platin oder Palladium/ Kohle, gegebenenfalls in Gegenwart einer Base wie eines Alkoholats, z.B. Natriummethylat, bei einem Wasserstoffdruck von 1 bis 7 bar, vorzugsweise jedoch von 3 bis 5 bar, und bei Temperaturen zwischen 0 und 75_{°}C, vorzugsweise jedoch bei Temperaturen zwischen 20 und 50_{°}C, durchgeführt.

Bei der Umsetzung kann eine gegebenenfalls vorhandene Benzyloxygruppe in die entsprechende Hydroxygruppe übergeführt werden.

c.) Zur Herstellung von Verbindungen der allgemeinen Formel I, in der B die Carbonyl- oder Methylengruppe darstellt:

Umsetzung einer Verbindung der allgemeinen Formel 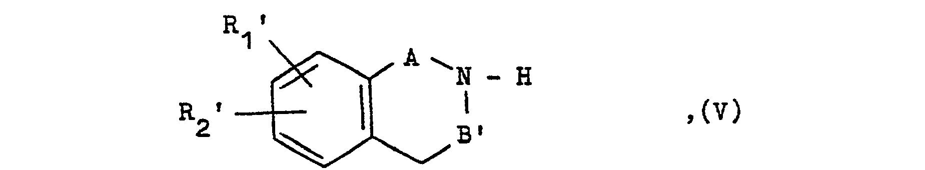 in der A wie eingangs definiert ist,
R₁' eine durch einen Schutzrest geschützte Hydroxy-, Amino- oder Alkylaminogruppe darstellt oder die für R₁ eingangs erwähnten Bedeutungen besitzt,
R_{2'} eine durch einen Schutzrest geschützte Hydroxygruppe darstellt oder die für R₂ eingangs erwähnten Bedeutungen besitzt, und

B' eine Carbonyl- oder Methylengruppe darstellt, mit einer Verbindung der allgemeinen Formel 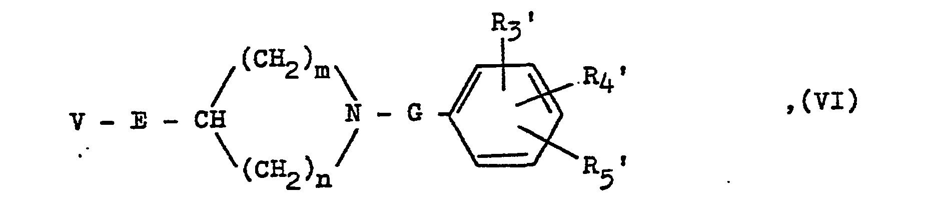 in der
E, G, m und n wie eingangs definiert sind,
Rs', R₄' und Rs' die für Rs, R₄ und R₅ eingangs erwähnten Bedeutungen besitzen, wobei jedoch die in den Resten R₃ bis R₅ enthaltenen Hydroxy-, Amino- oder Alkylaminogruppen durch einen Schutzrest geschützt sein können, und
V eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, darstellt und gegebenenfalls anschließende Abspaltung eines verwendeten Schutzrestes.

Als Schutzrest für eine Hydroxygruppe kommt beispielsweise die Trimethylsilyl-, Acetyl-, Benzoyl-, Benzyl- oder Tetrahydropyranylgruppe und
als Schutzrest für eine Amino- oder Alkylaminogruppe die Acetyl-, Benzoyl-, Ethoxycarbonyl- oder Benzylgruppe in Betracht.

Die Umsetzung wird zweckmäßigerweise in einem Lösungsmittel oder Lösungsmittelgemisch wie Methylformamid, Dimethylformamid, Dimethylsulfoxid, Benzol, Chlorbenzol, Tetrahydrofuran, BenzoI/Tetrahydrofuran oder Dioxan in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid oder eines Alkalihydrids wie Natriumhydrid zweckmäßigerweise bei Temperaturen zwischen 0 und 150_{°}C, vorzugsweise bei Temperaturen zwischen 0 und 50°C, durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100_{°}C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50_{°}C, vorzugsweise jedoch bei Raumtemperatur, und einem Wasserstoffdruck von I bis 7 bar, vorzugsweise jedoch von 3 bis 5 bar.

Bei der Umsetzung kann eine gegebenenfalls vorhandene Benzyloxygruppe in die entsprechende Hydroxygruppe übergeführt werden.

d) Zur Herstellung von Verbindungen der allgemeinen Formel I, in der A eine -CH₂CH₂- oder - CH=CH-Gruppe und B eine Thiocarbonylgruppe darstellen:

Umsetzung einer Verbindung der allgemeinen Formel 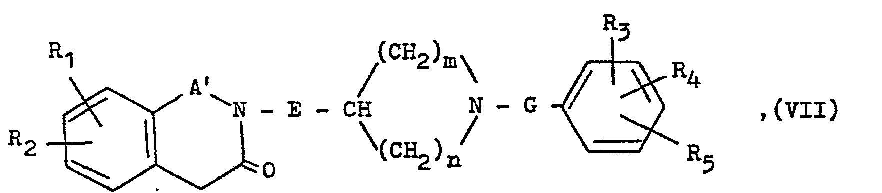 in der
R₁ bis Rₛ, E, G, m und n wie eingangs definiert sind und A' eine -CH₂-CH₂- oder -CH=CH-Gruppe darstellt, mit einem schwefeleinführenden Mittel.

Die Umsetzung wird mit einem schwefeleinführenden Mittel wie Phosphorpentasulfid oder 2,4-Bis(4-methoxyphenyl)-1,3-dithia-2,4-diphosphetan-2,4-disulfid zweckmäßigerweise in einem Lösungsmittel wie Toluol oder Xylol bei Temperaturen zwischen 50 und 150_{°}C, z.B. bei der Siedetemperatur des Reaktionsgemisches, durchgeführt.

e) Zur Herstellung von Verbindungen der allgemeinen Formel I, in der A eine 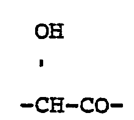 Gruppe und B eine Methylengruppe darstellen:

Reduktion einer Verbindung der allgemeinen Formel 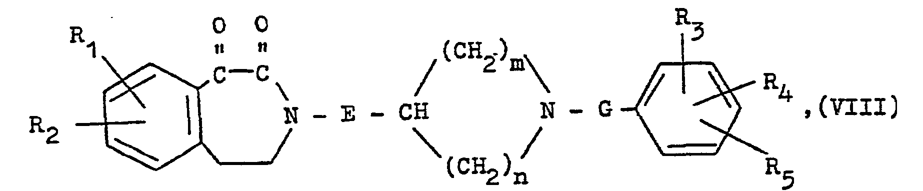 in der Ri bis Rs, E, G, m und n wie eingangs definiert sind.

Die Umsetzung wird in Gegenwart eines geeigneten Reduktionsmittels wie einem Metallhydrid, z.B. Natriumborhydrid, in einem geeigneten Lösungsmittel wie Wasser/Methanol oder Methanol/Ether, bei Temperaturen zwischen 0 und 80_{°}C, vorzugsweise jedoch bei Temperaturen zwischen 15 und 40°C, durchgeführt.

f) Zur Herstellung von Verbindungen der allgemeinen Formel I, in der A eine -CH₂-CH₂- oder - CH=CH- und B eine Methylengruppe darstellen:

Reduktion einer Verbindung der allgemeinen Formel 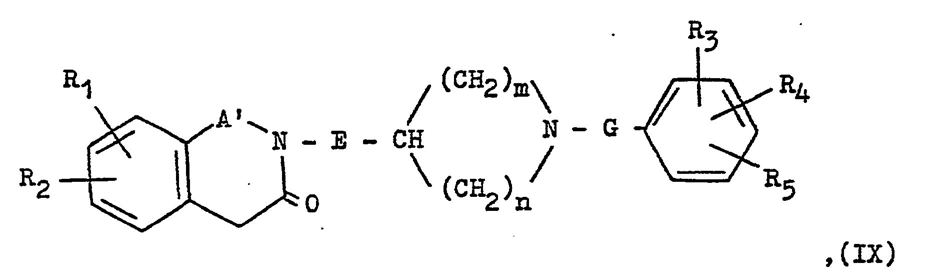 in der
Ri bis R₅, E, G, m und n wie eingangs definiert sind und A' eine -CH₂-CH₂- oder -CH=CH-Gruppe darstellt.

Die Reduktion wird vorzugsweise mit einem Metallhydrid wie Lithiumaluminiumhydrid oder Diboran oder mit einem Komplex aus Boran und einem Thioäther, z.B. mit Boran-Dimethylsulfid-Komplex, in einem geeigneten Lösungsmittel wie Diäthyläther oder Tetrahydrofuran bei Temperaturen zwischen 0 und 50°C, vorzugsweise jedoch bei Temperaturen zwischen 10 und 25_{°}C, durchgeführt.

g) Zur Herstellung von Verbindungen der allgemeinen Formel in der A die -COCO-Gruppe darstellt: Oxidation einer Verbindung der allgemeinen Formel 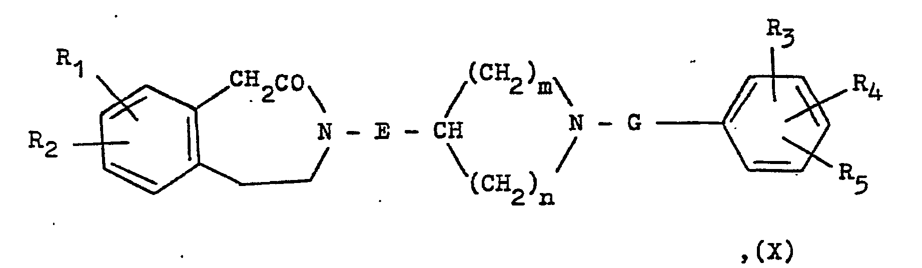 in der R₁ bis Rₛ, E, G, m und n wie eingangs definiert sind.

Die Oxidation wird vorzugsweise mit einem Oxidationsmittel wie Kaliumpermanganat, Selendioxid oder Natriumdichromat in einem geeigneten Lösungsmittel oder Lösungsmittelgemiscü wie Wasser, Wasser/Dioxan, Eisessig, Wasser/Essigsäure oder Acetanhydrid bei Temperaturen zwischen 0 und 100_{°}C, vorzugsweise bei Temperaturen zwischen 20 und 80_{°}C, durchgeführt.

h) Zur Herstellung von Verbindungen der allgemeinen Formel I, in der G mit Ausnahme der ein Schwefelatom, eine Sulfinyl- oder Sulfonylgruppe enthaltenden Reste die für G eingangs erwähnten Bedeutungen besitzt, A die -CH2-CH2Gruppe und B die Methylen- oder Carbonylgruppe darstellen:

Hydrierung einer Verbindung der allgemeinen Formel 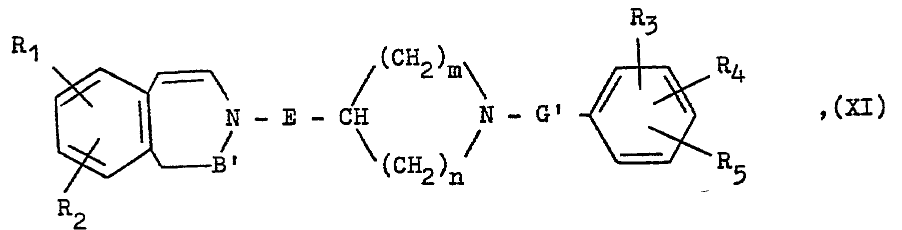 in der R₁ bis Rs, E, m und n wie eingangs definiert sind, G, mit Ausnahme der ein Schwefelatom, eine Sulfinyl- oder Sulfonylgruppe enthaltenden Reste die für G eingangs erwähnten Bedeutungen besitzt und B, die Methylen- oder Carbonylgruppe dsrstellt.

Die Hydrierung wird in einem Lösungsmittel oder Lösungsmittelgemisch wie Methanol, Ethanol, Essigsäureethylester oder Eisessig vorzugsweise mit katalytisch angeregtem Wasserstoff, z.B. mit Wasserstoff in Gegenwart von Platin oder Palladium/ Kohle, bei einem Wasserstoffdruck von 1 bis 7 bar, vorzugsweise jedoch von 3 bis 5 bar, und bei Temperaturen zwischen 0 und 75_{°}C, vorzugsweise jedoch bei Temperaturen zwischen 20 und 50_{°}C, durchgeführt.

Enthält eine Verbindung der allgemeinen Formel XI eine Benzyloxygruppe, so wird diese bei der Reduktion in die entsprechende Hydroxygruppe überführt.

Erhält man erfindungsgemäß eine Verbindung der allgemeinen Formel I, in der R₁ und/oder R₃ eine Nitrogruppe darstellt, so kann diese mittels Reduktion in eine entsprechende Aminoverbindung der allgemeinen Formel übergeführt werden oder

eine Verbindung der allgemeinen Formel I, in der R₄ eine Hydroxy- oder Aminogruppe darstellt, so kann diese mittels Acylierung in eine entsprechende Alkansulfonyloxy- oder Alkanoylaminoverbindung der allgemeinen Formel übergeführt werden.

Die nachträgliche Reduktion der Nitroverbindung wird vorzugsweise in einem Lösungsmittel wie Wasser, Wasser/äthanol, Methanol, Eisessig, Essigsäureäthylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(11)sulfat, Zinn(II)-chlorid oder Natriumdithionit oder mit Hydrazin in Gegenwart von Raney-Nickel bei Temperaturen zwischen 0 und 50_{°}C, vorzugsweise jedoch bei Raumtemperatur, durchgeführt.

Die nachträgliche Acylierung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid vorzugsweise mit einem reaktiven Derivat der Säure, beispielsweise mit Methansulfonsäurechlorid, Ethansulfonsäurechlorid, n-Propansulfonsäurechlorid, Acetylchlorid, Acetanhydrid oder Propionsäureanhydrid, und gegebenenfalls in Gegenwart einer anorganischen Base wie Natriumkarbonat oder einer tertiären organischen Base wie Triäthylamin oder Pyridin, welche gleichzeitig als Lösungsmittel dienen können, bei Temperaturen zwischen -25_{°}C und 100_{°}C, vorzugsweise jedoch bei Temperaturen zwischen -10_{°}C und der Siedetemperatur des verwendeten Lösungsmittels, durchgeführt.

Die erhaltenen Verbindungen der allgemeinen Formel 1 lassen sich, da diese mindestens ein chirales Zentrum besitzen, mittels üblichen Methoden in ihre Diastereomeren, beispielsweise durch Säulenchromatographie, und in ihre Enantiomeren auftrennen, beispielsweise durch Säulenchromatographie an einer chiralen Phase oder durch Kristallisation mit optisch aktiven Säuren, z.B. mit D- oder L-Monomethylweinsäure, D- oder L-Diacetylweinsäure, D- oder L-Weinsäure, D- oder L-Milchsäure oder D- oder L-Camphersäure.

Die erhaltenen Verbindungen der allgemeinen Formel I lassen sich ferner in ihre Säureadditionssalze, insbesondere für ihre pharmazeutische Anwendung in ihre physiologisch verträglichen Säureadditionssalze mit anorganischen oder organischen Säuren überführen. Als Säuren kommen hierbei beispielsweise Salzsäure, Bromwasserstoffsäure, Schwefelsäure, Phosphorsäure, Essigsäure, Milchsäure, Zitronensäure, Weinsäure, Bernsteinsäure, Maleinsäure oder Fumarsäure in Betracht.

Die als Ausgangsstoffe verwendeten Verbindungen der allgemeinen Formeln II bis XI sind teilweise literaturbekannt bzw. man erhält sie nach an sich bekannten Verfahren.

So erhält man beispielsweise eine Ausgangsverbindung der allgemeinen Formel II durch Umsetzung eines entsprechenden Benzazepins mit einer entsprechenden Halogenverbindung und gegebenenfalls durch anschließende Umsetzung mit einem entsprechenden Amin. Das hierfür erforderliche in 3-Stellung unsubstituierte entsprechende Benzazepin der allgemeinen Formel V erhält man durch Cyclisierung einer entsprechenden Verbindung, z.B. durch Cyclisierung einer Verbindung der allgemeinen Formel 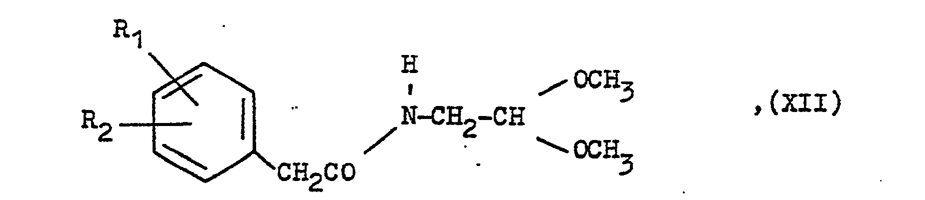 oder auch der allgemeinen Formel 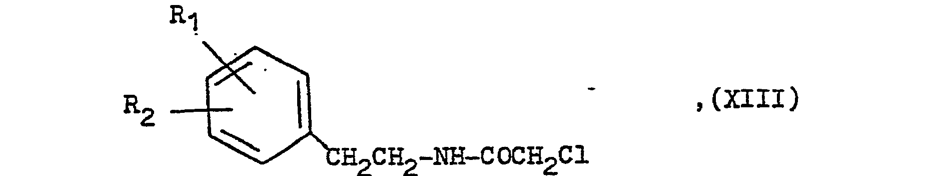 gegebenenfalls anschließender katalytischer Hydrierung und- oder Reduktion der Carbonylgruppe beispielsweise mit Natriumborhydrid/Eisessig (siehe EP-AI 0.007.070, EP-AI 0.065.229 und EP-AI 0.109.639) und/oder Oxidation, z.B. mit Selendioxid.

Eine als Ausgangsstoff verwendete Verbindung der allgemeinen Formeln IV und VII bis XI erhält man vorzugsweise durch Umsetzung einer entsprechenden Halogenverbindung mit einem entsprechenden Amin und gegebenenfalls anschließende Quarternierung und/oder Abspaltung von Schutzresten, die zum Schutz von Hydroxy- und/oder Aminogruppen verwendet werden.

Wie bereits eingangs erwähnt, weisen die neuen Verbindungen der allgemeinen Formel I und deren physiologisch verträgliche Säureadditionssalze mit anorganischen oder organischen Säuren wertvolle pharmakologische Eigenschaften auf, insbesondere bei geringen zentralen Nebenwirkungen eine besonders lang anhaltende herzfrequenzsenkende Wirkung sowie eine Herabsetzung des 02-Bedarfs des Herzens.

Beispielsweise wurden die Verbindungen
A = 3-[(N-(2-(3,4-Dimethoxy-phenyl)-ethyl)-piperidin-3-yl)-methyl]-7,8-dimethoxy-1,3,4,5-tetrahy- dro-2H-3-benzazepin-2-on,
B = 3-[(N-(2-(3-Methyl-phenyl)-ethyl)-piperidin-3-yl)-methyl]-7,8-dimethoxy-1,3,4,5-tetrahydro-2H-3-benzazepin-2-on,
C = 3-[(N-(3-(4-Methoxy-phenyl)-propyl)-piperidin-3-yl)-methyl]-7,8-dimethoxy-1,3,4,5-tetrahydro-2H-3-benzazepin-2-on und
D = 3-[(N-(2-(3,4-Dimethoxy-phenyl)-ethyl)-piperidin-2-yl)-ethyl-2]-7,8-dimethoxy-1,3,4,5-tetrahy- dro-2H-3-benzazepin-2-on

auf ihre biologischen Eigenschaften wie folgt untersucht:

### Wirkung auf die Herzfreauenz an Ratten:

Die Wirkung der zu untersuchenden Substanzen auf die Herzfrequenz wurde pro Dosis an 2 Ratten mit einem durchschnittlichen Gewicht von 250-300 g untersucht. Hierzu wurden die Ratten mit Pentobarbital (50 mg/kg i.p. und 20 mg/kg s.c.) narkotisiert. Die zu untersuchenden Substanzen wurden in wäßriger Lösung in die Vena jugularis injiziert (0,1 ml/100 g).

Der Blutdruck wurde über in eine A. carotis eingebundene Kanüle gemessen und die Herzfrequenz wurde aus einem mit Nadelelektroden abgeleiteten EKG (II. oder III. Ableitung) registriert. Die Herzfrequenz der Tiere in der Kontrollperiode lagen zwischen 350 und 400 Schlägen/Minuten (S/min).

Die nachfolgende Tabelle enthält die gefundenen Werte:

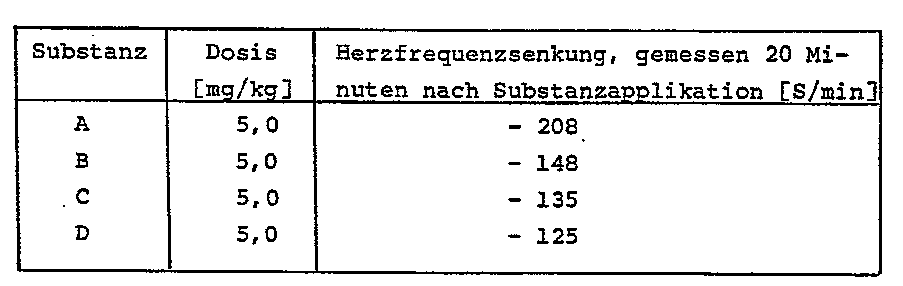

Die erfindungsgemäß hergestellten Verbindungen weisen in therapeutischen Dosen keinerlei toxische Nebenwirkungen auf. So konnten beipielsweise bei einer intravenösen Applikation der Substanz A und D auch in einer hohen Dosis von 20 mg/kg an Mäusen, außer einer geringen Sedation keine toxischen Nebenwirkungen beobachtet werden.

Aufgrund ihrer pharmakologischen Eigenschaften eignen sich die erfindungsgemäß hergestellen Verbindungen zur Behandlung von Sinustachykardien verschiedener Genese und zur Prophylaxe und Therapie ischämischer Herzerkrankungen.

Die zur Erzielung einer entsprechenden Wirkung erforderlichen Dosierung beträgt zweckmäßigerweise ein- bis zweimal täglich 0,01 bis 0,2 mg/kg Körpergewicht, vorzugsweise 0,03 bis 0,15 mg/kg Körpergewicht. Hierzu lassen sich die erfindungsgemäß hergestellten Verbindungen der allgemeinen For mel 1 sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Carboxymethylcellulose oder fetthaltige Substanzen wie Hartfett oder deren geeignete Gemische, in übliche galenische Zubereitungen wie Tabletten, Dragees, Kapseln, Pulver, Suspensionen, Tropfen, Ampullen, Säfte oder Zäpfchen einarbeiten.

Die nachfolgenden Beispiele sollen die Erfindung näher erläutern:

### Beispiel A

### N-Benzvl-3-(hvdroxvmethvll-piperidin

Ein Gemisch von 40,3 g (0,35 Mol) 3-(Hydroxymethyl)-piperidin, 97,4 ml (0,70 Mol) Triethylamin und 40,3 ml (0,35 Mol) Benzylchlorid wird innerhalb von 30 Minuten auf 95_{°}C erhitzt und 2 Stunden bei dieser Temperatur belassen. Das abgekühlte Reaktionsgemisch wird in einer Mischung aus 2 molarer Natronlauge und Essigester gelöst. Die organische Phase wird mit Wasser gewaschen, abgetrennt, über Magnesiumsulfat getrocknet und im Vakuum eingedampft.
Ausbeute: 57,2 g (79,6 % der Theorie),
Rf-Wert: 0,45 (Aluminiumoxid neutral, Laufmittel: 3 % Ethanol in Methylenchlorid).

### Beispiel B

### N-Benzvi-3-(brommethvh-DiDeridin

Zu 400 ml 48%iger Bromwasserstoffsäure gibt man unter starkem Rühren 55,1 g (0,268 Mol) N-Benzyl-3-(hydroxymethyl)-piperidin und erhitzt I Stunde unter Rückfluß. Anschließend wird bis zur Sättigung Bromwasserstoff eingeleitet (ca. I Stunde), eine weitere Stunde unter Rückfluß erhitzt und über Nacht stehen gelassen. Anschließend wird unter Eiskühlung mit festem Kaliumcarbonat neutralisiert und dann mit Methylenchlorid extrahiert. Die organische Phase wird über Magnesiumsulfat getrocknet und im Vakuum eingedampft.
Ausbeute: 52,0 g (72,2 % der Theorie),
Rf-Wert: 0,85 (Aluminiumoxid neutral, Laufmittel: Methylenchlorid).

### Beispiel C

### 3-[(N-Benzyl-piperidin-3-yl)-methyl]-7,8-dimethoxy-1,3-dihydro-2H-3-benzazepin-2-on

17,54 g (0,08 Mol) 7,8-Dimethoxy-1,3-dihydro-2H-3-benzazepin-2-on werden in 150 ml Dimethylsulfoxid suspendiert und unter Rühren mit 8,98 g (0,08 Mol) Kalium-tert.butylat versetzt. Nach 45 Minuten wird zu der erhaltenen Lösung unter Rühren 21,45 g (0,08 Mol) N-Benzyl-3-(brommethyl)-piperidin, gelöst in 50 ml Dimethylsulfoxid, zugetropft. Nach 2 Stunden gießt man auf Eiswasser. Die wässrige Phase wird 3 x mit je 150 ml Essigester extrahiert. Die vereinigten organischen Phasen werden mit Wasser gewaschen, über Magnesiumsulfat getrocknet, im Vakuum eingedampft und über 800 g Aluminiumoxid (neutral, Aktivität II-III) mit Methylenchlorid und anschließend mit steigenden Anteilen von Ethanol (bis 3 %) gereinigt.
Ausbeute: 14,3 g (44 % der Theorie),
Rf-Wert: 0,35 (Aluminiumoxid neutral, Laufmittel: I % Ethanol in Methylenchlorid).

### Beispiel D

### 3-[(Piperidin-3-yl)-methyl]-7,8-dimethoxy-1,3,4,5-tetrahydro-2H-3-benzazepin-2-on

14,3 g (0,0352 Mol) 3-[(N-Benzyl-piperidin-3-yl)-methyl]-7,8-dimethoxy-1,3-dihydro-2H-3-benzazepin-2-on werden in 120 ml Eisessig in Anwesenheit von 1,5 g 10%iger Palladium/Kohle 4 Stunden bei 50_{°}C und 5 bar Wasserstoff hydriert. Anschliessend wird der Katalysator abgesaugt, der Eisessig im Vakuum abdestilliert und der Rückstand nach Zugabe von Wasser mit Kaliumcarbonat neutralisiert. Der schmierige Niederschlag wird mit Methylenchlorid extrahiert, die organische Phase über Magnesiumsulfat getrocknet und im Vakuum eingedampft.
Ausbeute: 9,3 g (83 % der Theorie),
Schmelzpunkt: 152-156_{°}C.

### Beispiel E

### 3-[(Pyridin-3-yl)-methyl]-7,8-dimethoxy-1,3-dihydro-2H-3-benzazepin-2-on

2,2 g (0,01 Mol) 7,8-Dimethoxy-1,3-dihydro-2H-3-benzazepin-2-on werden in 10 ml Dimethylsulfoxid suspendiert und unter Rühren mit 1,12 g (0,01 Mol) Kalium-tert.butylat versetzt. Nach 60 Minuten wird zu der erhaltenen Lösung unter Rühren 1,3 g (0,01 Mol) 3-Picolylchlorid, gelöst in 10 ml Dimethylsulfoxid, zugetropft. Nach 1 Stunde gießt man auf Eiswasser. Die Wasserphase wird 2 x mit Essigester extrahiert. Die vereinigten organischen Phasen werden mit Wasser gewaschen, über Magnesiumsulfat getrocknet, im Vakuum eingedampft und über 200 g Aluminiumoxid (neutral, Aktivität II-III) mit Methylenchlorid und anschließend mit steigenden Anteilen von Ethanol (bis 0,8 %) gereinigt.
Ausbeute: 1,4 g (45,2 % der Theorie),
Schmelzpunkt: 144-146_{°}C.

### Beispiel F

### 3-[(N-(2-(3,4-Dimethoxy-phenyl)-ethyl)-pyridinium-3-yl)-methyl]-7,8-dimethoxy-1,3-dihydro-2H-3-benzazepin-2-on-bromid

Ein Gemisch von 1,1 g (0,0035 Mol) 3-[(Pyridin-3-yl)-methyl]- 7,8-dimethoxy₋i,3-dihydro-2H-3-benzazepin-2-on und 2-(3,4-Dimethoxyphenyl)-ethylbromid wird 6 Stunden auf 110°C erhitzt. Das abgekühlte Reaktionsgemisch wird in wenig Methanol/Methylenchlorid gelöst und unter starkem Rühren in 200 ml Diethylether getropft. Der entstandene Niederschlag wird abgesaugt und getrocknet.
Ausbeute: 1,6 g (80 % der Theorie),
Schmelzpunkt: 147-150°C.

### Beispiel G

### N-[2-(3,4-Dimethoxy-phenyl)-ethyl]-3-(hydroxymethyl)-piperidin

Ein Gemisch von 2,30 g (0,02 Mol) 3-(Hydroxymethyl)-piperidin, 5,5 ml (0,04 Mol) Triethylamin und 4,90 g (0,02 Mol) 2-(3,4-Dimethoxy-phenyl)-ethylbromid wird 2 Stunden unter Rückfluß erhitzt. Das abgekühlte Reaktionsgemisch wird in einem Gemisch aus 2 molarer Natronlauge und Methylenchlorid gelöst. Die organische Phase wird mit Wasser gewaschen, abgetrennt, über Magnesiumsulfat getrocknet, im Vakuum eingedampft und über 300 g Aluminiumoxid (neutral, Aktivität II-III) mit Methylenchlorid und anschließend mit steigenden Anteilen Ethanol (bis 2 %) gereinigt.
Ausbeute: 4,4 g (78,7 % der Theorie),
Schmelzpunkt: 87,5-89_{°}C.

### Beispiel H

### N-[2-(3,4-Dimethoxy-phenyl)-ethyl]-3-(brommethyl)-piperidin

4,4 g (0,0157 Mol) N-[2-(3,4-Dimethoxy-phenyl)-ethyl]-3-(hydroxymethyl)-piperidin werden in 70 ml Tetrachlorkohlenstoff gelöst und auf 0_{°}C abgekühlt. Anschließend wird 1,63 ml (0,0173 Mol) Phosphortribromid zugegeben, wobei sofort ein voluminöser Niederschlag ausfällt. Man rührt 15 Stunden bei Raumtemperatur, versetzt das Gemisch mit Wasser und neutralisiert mit 2 molarer Natronlauge. Die organische Phase wird abgetrennt, mit Wasser gewaschen, über Magnesiumsulfat getrocknet, im Vakuum eingedampft und über 310 g Aluminiumoxid (neutral, Aktivität II-III) mit Methylenchlorid und anschließend mit steigenden Anteilen von Ethanol (bis 5 %) gereinigt.
Ausbeute: 2,0 g (37,2 % der Theorie),
Rf-Wert: 0,5 (Aluminiumoxid neutral, Laufmittel: 2 % Ethanol in Methylenchlorid).

### Beispiel

### N-Benzvl-caprolactam

33,9 g (0,3 Mol) Caprolactam werden in 200 ml absolutem Dimethylsulfoxid und 100 ml absolutem Tetramethylharnstoff gelöst und mit 14,4 g (0,33 Mol) 55%iger Natriumhydrid-ÖI-Dispersion portionsweise versetzt. Der entstehende gallertartige Niederschlag wird 2 Stunden bei Raumtemperatur gerührt. Anschließend werden 38 g = 34,4 ml (0,3 Mol) Benzylchlorid zugetropft, 2 Stunden wird bei Raumtemperatur gerührt und anschließend auf Eiswasser gegossen. Die wässrige Phase wird 2 mal mit Essigester ausgeschüttelt. Die organischen Phasen werden vereinigt, 4 mal mit Wasser gewaschen, über Magnesiumsulfat getrocknet und im Vakuum eingeengt. Der verbleibende Rückstand wird im Vakuum destilliert. Ausbeute: 49,9 g (81,8 % der Theorie),
Kpo,27 mm _{H}g: 110-114°C

### Beispiel K

### I-Benzvl-caprolactam-3-carbonsäure

Zu 33,9 g = 47,1 ml (0,33 Mol) Diisopropylamin in 450 ml absolutem Äther werden unter Rühren und Stickstoff bei -60_{°}C 180 ml 1,6 molare Butyllithium-Lösung in n-Hexan zugegeben. Anschließend tropft man unter weiterer Kühlung 48,8 g (0,24 Mol) N-Benzyl-carpolactam, gelöst in 150 ml absolutem Ether, zu. Nach 10-minütigem Rühren entfernt man das Kältebad und leitet 15 Minuten lang Kohlendioxid ein. Das Reaktionsgemisch wird auf Eis gegossen, die ätherische Phase abgetrennt und 2 mal mit 2 molarer Natronlauge ausgeschüttelt. Die wässrig-alkoholischen Phasen werden vereinigt, mit Ether ausgeschüttelt, mit konzentrierter Salzsäure angesäuert und 2 mal mit Methylenchlorid ausgeschüttelt. Die vereinigten Methylenchlorid-Phasen werden über Magnesiumsulfat getrocknet und das Lösungsmittel im Vakuum abdestilliert.
Ausbeute: 15,7 g (26,5 % der Theorie),
IR-Spektrum (Methylenchlorid): 1735 und 1600 cm-¹ (CO)

### Beispiel L

### 1-Benzyl-3-hydroxymethyl-hexahydro-azepin

Zu 6,84 g (0,18 Mol) Lithiumaluminiumhydrid in 300 ml absolutem Tetrahydrofuran werden 14,8 g (0,06 Mol) I-Benzyl-caprolactam-3-carbonsäure, gelöst in 300 ml absolutem Tetrahydrofuran, getropft. Danach wird 6 Stunden unter Rückfluß erhitzt, anschließend unter Eiswasserkühlung mit 6,8 ml Wasser, 6,8 ml 2-molarer Natronlauge und 21 ml Wasser versetzt. Der Niederschlag wird abgesaugt, mit Tetrahydrofuran nachge waschen und das Filtrat im Vakuum eingeengt. Der Rückstand wird säulenchromatographisch über Aluminiumoxid N (Aktivität II, Elutionsmittel: Methylenchlorid) gereinigt.
Ausbeute: 8,4 g (63,8 % der Theorie),
IR-Spektrum (Methylenchlorid): 3620 cm-¹ (OH)

### Beispiel M

### 1-Benzyl-3-brommethyl-hexahydro-azepin

8,3 g (0,038 Mol) I-Benzyl-3-hydroxymethyl-hexahydro-azepin werden in 200 ml Tetrachlorkohlenstoff gelöst und mit 16 ml Phosphortribromid versetzt. 6 Stunden wird bei Raumtemperatur gerührt, anschließend unter Eiswasserkühlung mit Wasser zersetzt und mit 2-molarer Natronlauge schwach alkalisch gestellt. Die wässrige Lösung wird abgetrennt und 2 mal mit Methylenchlorid ausgeschüttelt. Die organischen Phasen werden vereinigt, über Magnesiumsulfat getrocknet und im Vakuum eingeengt. Ausbeute: 8,9 g (82 % der Theorie).

### Beispiel N

### 3-[(N-Benzyl-hexahydro-azepin-3-yl)-methyl]-7,8-dimethoxy-1,3-dihydro-2H-3-benzazepin-2-on

Zu einer Lösung von 4,4 g (0,02 Mol) 7,8-Dimethoxy-1,3-dihydro-2H-3-benzazepin-2-on in 100 ml absolutem Dimethylsulfoxid gibt man 2,3 g (0,02 Mol) Kalium-tert.butylat. Nach 30-minütigem Rühren bei Raumtemperatur versetzt man mit 5,6 g (0,020 Mol) I-Benzyl-3-brommethyl-hexahydro-azepin und rührt 2 Stunden bei Raumtemperatur. Das Reaktionsgemisch wird in Essigester gelöst und mehrmals mit Wasser ausgeschüttelt. Die organische Phase wird über Magnesiumsulfat getrocknet und im Vakuum eingeengt. Der Rückstand wird säulenchromatographisch über Aluminiumoxid N (Aktivität II, Elutionsmittel: Methylenchlorid, Methylenchlorid + 0,3 % Äthanol) gereinigt.
Ausbeute: 4,2 g (50 % der Theorie),
IR-Spektrum (Methylenchlorid): i655 cm⁻¹ (CO)

### Beispiel 0

### 3-[(Hexahydro-azepin-3-yl)-methyl]-7,8-dimethoxy-1,3,4,5-tetrahydro-2H-3-benzazepin-2-on

4,2 g (0,01 Mol) 3-[(N-Benzyl-hexahydro-azepin-3-yl)-methyl]-7,8-dimethoxy-1,3-dihydro-2H-3-benzazepin-2-on werden in 100 ml Eisessig in Anwesenheit von 0,5 g 10%igem Palladium-Kohle 14 Stunden bei 50 psi und 50_{°}C hydriert. Der Katalysator wird abgesaugt und der Eisessig im Vakuum abdestilliert. Der Rückstand wird in Wasser aufgenommen, mit 2-molarer Natronlauge alkalisch gestellt und mehrmals mit Methylenchlorid ausgeschüttelt. Der organische Extrakt wird über Magnesiumsulfat getrocknet und im Vakuum eingeengt. Die säulenchromatographische Reinigung erfolgt über Aluminiumoxid N (Aktivität II, Elutionsmittel: Methylenchlorid + I % Äthanol).
Ausbeute: 2,6 g (78,2 % der Theorie),
IR-Spektrum (Methylenchlorid): 1650 cm-1 (CO)

### Beispiel P

### 7-Carbethoxvmethvl-caprolactam

Zu 50 ml konzentrierter Schwefelsäure werden bei 0_{°}C 9,2 g = 9 ml (0,05 Mol) Cyclohexanon-2-essigsäureethylester getropft. Anschließend werden 3,25 g (0,05 Mol) Natriumazid portionsweise zugegeben. Nach 10-stündigem Rühren bei 0_{°}C wird das Reaktionsgemisch auf Eiswasser gegossen und mit konzentriertem Ammoniak unter weiterer Kühlung neutralisiert. Nach Sättigung der Lösung mit Natriumchlorid wird mehrmals mit n-Butanol, dem 10 % Methylenchlorid zugesetzt werden, extrahiert. Der Extrakt wird im Vakuum eingeengt und der Rückstand über Aluminiumoxid N (Aktivität 11, Elutionsmittel: Methylenchlorid + 0,5 % Äthanol) säulenchromatographisch getrennt.
Ausbeute: 5,7 g (56,6 % der Theorie),
Schmelzpunkt: 108-109_{°}C.

### Beispiel Q

### 2-(2-Hvdroxvethvl)-hexahvdro-azepin-hvdrochlorid

Zu 2,6 g (0,06 Mol) Lithiumaluminiumhydrid in 100 ml absolutem Dioxan werden unter Rühren und Rückfluß 5,6 g (0,028 Mol) 7-Carbethoxymethyl-caprolactam, gelöst in 50 ml absolutem Dioxan, getropft. 18 Stunden wird unter Rückfluß gekocht und anschließend unter Eiswasserkühlung mit 2,3 ml Wasser, 2,3 ml 15%iger Natronlauge und 6,9 ml Wasser versetzt. Der Niederschlag wird abgesaugt und mit Ether gewaschen. Das Filtrat wird im Vakuum eingeengt, der Rückstand in Ether gelöst und mit etherischer Salzsäure gefällt.
Ausbeute: 3 g (59,6 % der Theorie),
Schmelzpunkt: 75_{°}C.

### Beispiel R

### N-[2-(3,4-Dimethoxyphenyl)-ethyl]-2-(2-hydroxyethyl)-hexahydro-azepin

2,9 g (0,016 Mol) 2-(2-Hydroxyethyl)-hexahydro-azepin-hydrochlorid werden mit konzentrierter Natronlauge freigesetzt, in Methylenchlorid aufgenommen und nach Trocknung über Magnesiumsulfat eingeengt. Der Rückstand wird mit 3,9 g (0,016 Mol) 2-(3,4-Dimethoxy-phenyl)-ethylbromid in 10 ml Triethylamin 2 Stunden unter Rückfluß gekocht. Nach Abkühlung wird das Reaktionsgemisch mit 2-molarer Natronlauge/Methylenchlorid versetzt. Die abgetrennte alkalische Phase wird 2 mal mit Methylenchlorid ausgeschüttelt und die vereinigten organischen Phasen über Magnesiumsulfat getrocknet. Das Lösungsmittel wird im Vakuum abdestilliert und der Rückstand säulenchromatographisch über Aluminiumoxid N (Aktivität II, Elutionsmittel: Methylenchlorid) gereinigt.
Ausbeute: 3,7 g (75,2 % der Theorie)

### Beispiel S

### 2-(2-Bromethyl)-1-[2-(3,4-dimethoxy-phenyl)-ethyl]-hexahydro-azepin

Zu 2,9 g (9,4 mMol) N-[2-(3,4-Dimethoxy-phenyl)-ethyl]-2-(2-hydroxyethyl)-hexahydro-azepin in 100 ml Tetrachlorkohlenstoff werden unter Eiswasserkühlung 4 ml Phosphortribromid getropft und 15 Stunden bei Raumtemperatur gerührt. Anschließend wird unter Eiswasserkühlung mit Wasser zersetzt und mit 2-molarer Natronlauge schwach alkalisch gestellt. Die wässrig-alkalische Lösung wird abgetrennt und 2 mal mit Methylenchlorid ausgeschüttelt. Die vereinigten organischen Lösungen werden über Magnesiumsulfat getrocknet und im Vakuum eingeengt.
Ausbeute: 3,6 g (100 % der Theorie).

### Beispiel T

### 3-[(Pyridin-3-yl)-methyl]-7,8-dimethoxy-1,3-dihydro-2H-3-benzazepin-2-on

### a.) (Pyridin-3-yl)-methylamino-N-acetaldehyd-dimethylacetal

5,36 g (0,050 Mol) Pyridin-3-aldehyd und 5,26 g (0,050 Mol) Aminoacetaldehyd-dimethylacetal werden in 80 ml Ethanol in Anwesenheit von 0,8 g 10%igem Palladium/Aktivkohle 2 Stunden bei 20_{°}C und 5 bar hydriert. Anschließend wird der Katalysator abgesaugt und das Ethanol im Vakuum abdestilliert.
Ausbeute: 9,4 g (96 % der Theorie),
Rf-Wert: 0,25 (Aluminiumoxid, Laufmittel: 2% Ethanol in Methylenchlorid).

### b.) 3,4-Dimethoxy-phenylessigsäure-N-(acetaldehyd-dimethylacetal)-N-[(pyridin-3-yl)-methyl])-amid

7,85 g (0,040 Mol) (Pyridin-3-yl)-methylamino-N-acetaldehyd-dimethylacetal und 4,4 g (0,044 Mol) Triethylamin werden in 50 ml Methylenchlorid gelöst. Unter Eiskühlung werden zu dieser Mischung 8,58 g (0,040 Mol) 3,4-Dimethoxy-phenylessigsäurechlorid zugetropft und I Stunde bei 20_{°}C gerührt. Anschließend extrahiert man 3 mal mit Wasser und trocknet die organische Phase über Magnesiumsulfat, welche anschließend eingeengt wird.
Ausbeute: 12,6 g (84 % der Theorie)
Rf-Wert: 0,5 (auf Kieselgel, Laufmittel: 5 % Ethanol in Methylenchlorid).

### c.) 3-[(Pyridin-3-yl)-methyl]-7,8-dimethoxy-1,3-dihydro-2H-3-benzazepin-2-on

3,74 g (0,010 Mol) 3,4-Dimethoxy-phenylessigsäure-N-(acetaldehyd-dimethylacetal)-N-[(pyridin-3-yl)-methyl]-amid werden in 10 ml konzentrierter Salzsäure und 10 ml Eisessig gelöst und 60 Stunden bei 20°C gerührt. Anschließend wird auf Eiswasser gegossen, mit 25%iger Natronlauge neutralisiert und 2 mal mit Methylenchlorid extrahiert. Die organische Phase wird über Magnesiumsulfat getrocknet, abfiltriert und einrotiert.
Ausbeute: 1,85 g (60 % der Theorie),
Schmelzpunkt: 144-146_{°}C (aus Aceton).

### Beispiel U

### N-[2-(3,4-Dimethoxy-phenyl)-ethyl]-3-tosyloxymethyl-pyrrolidin

### a) N-Benzyl-2-pyrrolidon

Zu 25,5 g (0,3 Mol) 2-Pyrrolidon in 300 ml absolutem Dimethylsulfoxid werden-portionsweise 14,4 g (0,33 Mol) 50%ige Natriumhydrid-Dispersion in 01 eingetragen. Anschließend wird 5 Stunden bei 40 bis 50_{°}C gerührt und bei 25-30_{°}C 56,4 g = 39,2 ml (0,33 Mol) Benzylbromid zugetropft. Nach 10-stündigem Rühren bei Raumtemperatur wird das Reaktionsgemisch in 500 ml Essigester gelöst und mehrmals mit Wasser ausgeschüttelt. Die organische Phase wird abgetrennt über Magnesiumsulfat getrocknet und das Lösungsmittel im Vakuum entfernt. Der erhaltene Rückstand wird über 900 g Aluminiumoxid (neutral, Aktivität II) mit Methylenchlorid und 0,1 % Ethanol gereinigt.
Ausbeute: 35,6 g (67,7 % der Theorie),
Rf-Wert: 0,77 (Aluminiumoxid, neutral, Laufmittel: 5 % Ethanol in Methylenchlorid).

### b) N-Benzvl-2-pvrrolidon-3-carbonsäure

Zu 28,3 g = 39,3 ml (0,28 Mol) Diisopropylamin in 400 ml absolutem Ether werden unter Rühren und unter Stickstoff bei -60_{°}C 150 ml 1,6 molare Butyllithium-Lösung in n-Hexan gegeben. Hierzu tropft man 35,1 g (0,2 Mol) N-Benzyl-2-pyrrolidon, gelöst in 150 ml absolutem Ether, bei -60_{°}C. Man entfernt das Kältebad und leitet 15 Minuten trockenes Kohlendioxid ein. Nach 10-minütigem Rühren wird auf Eis gegossen, die organische Phase abgetrennt und 2 mal mit 2 molarer Natronlauge ausgeschüttelt. Die vereinigten wässrigen Phasen werden einmal mit Ether ausgeschüttelt und anschließend unter Kühlung mit konzentrierter Salzsäure angesäuert. Die wässrige Phase wird 2 mal mit Methylenchlorid ausgeschüttelt und nach dem Trocknen der organischen Phase über Magnesiumsulfat im Vakuum eingeengt.
Ausbeute: 35 g (79,8 % der Theorie),
Rf-Wert: 0,42 (Kieselgel, Laufmittel: 5 % Ethanol in Methylenchlorid).

### c) N-Benzyl-3-hydroxymethyl-pyrrolidin

Zu 12,2 g (0,32 Mol) Lithiumaluminiumhydrid in 350 ml absolutem Tetrahydrofuran tropft man unter Rühren 35 g (0,16 Mol) N-Benzyl-2-pyrrolidon-3-carbonsäure, gelöst in 250 ml absolutem Tetrahydrofuran. Nach 6-stündigem Erhitzen unter Rückfluß versetzt man unter Eiswasserkühlung mit 18,2 ml Wasser, 12,2 ml 15%Iger Natriumlauge und 36,6 ml Wasser. Der entstandene Niederschlag wird abgesaugt und mit Tetrahydrofuran gewaschen. Die vereinigten Filtrate werden im Vakuum eingeengt und der erhaltene Rückstand über 900 g Aluminiumoxid (neutral, Aktivität II) mit Methylenchlorid und anschließend mit steigenden Anteilen von Ethanol (bis 2 %).
Ausbeute: 16 g (52,3 % der Theorie),
Rf-Wert: 0,42 (Aluminiumoxid, neutral, Laufmittel: 5 % Ethanol in Methylenchlorid).

### d) 3-Hydroxymethyl-pyrrolidin

14 g (0,073 Mol) N-Benzyl-3-hydroxymethyl-pyrrolidin werden 7 Stunden lang bei 50°C und 5 bar in 300 ml Methanol und in Gegenwart von 1,5 g 20%igem Palladiumhydroxid/Aktivkohle hydriert. Anschließend wird der Katalysator abgesaugt und das Filtrat im Vakuum eingeengt.
Ausbeute: 7,3 g (99 % der Theorie),
Massenspektrum: Molpeak 101

### e) N-[2-(3,4-Dimethoxy-phenyl)-ethyl]-3-hydroxymethyl-pyrrolidin

3 g (0,03 Mol) 3-Hydroxymethyl-pyrrolidin und 7,5 g 2-(3,4-Dimethoxy-phenyl)-ethylbromid werden in 20 ml Triethylamin 7 Stunden bei 100_{°}C erhitzt. Anschließend wird das überschüssige Triethylamin im Vakuum abdestilliert und der erhaltene Rückstand in Methylenchlorid und 6 molarer Natronlauge gelöst. Die organische Phase wird abgetrennt, über Magnesiumsulfat getrocknet und im Vakuum eingeengt. Anschließend wird der erhaltene Rückstand über 400 g Aluminiumoxid (neutral, Aktivität II) mit Methylenchlorid und mit steigenden Anteilen von Ethanol (bis 1 %) gereinigt.
Ausbeute: 5,4 g (67,8 % der Theorie),
Rf-Wert: 0,41 (Aluminiumoxid, neutral, Laufmittel: 5 % Ethanol in Methylenchlorid).

### f) N-[2-(3,4-Dimethoxy-phenyl)-ethyl]-3-tosyloxymethyl-pyrrolidin

1,3 g (0,005 Mol) N-[2-(3,4-Dimethoxy-phenyl)-ethyl]-3-hydroxymethyl-pyrrolidin werden in 10 ml Pyridin gelöst, mit 1,05 g (0,0055 Mol) p-Toluolsulfonsäurechlorid versetzt und 6 Stunden bei Raumtemperatur gerührt. Anschließend wird das überschüssige Pyridin im Vakuum abdestilliert, der erhaltene Rückstand in Methylenchlorid gelöst und die organische Phase mit Eiswasser ausgeschüttelt. Nach dem Trocknen der organischen Phase über Magnesiumsulat wird im Vakuum eingeengt.
Ausbeute: 1,4 g (66,7 % der Theorie),
Rf-Wert: 0,40 (Aluminiumoxid, neutral, Laufmittel: 2 % Ethanol in Methylenchlorid).

### Beispiel

### 3-[(N-(2-(3,4-Dimethoxy-phenyl)-ethyl)-piperidin-3-yl)-methyl-7,8-dimethoxy-1,3,4,5-tetrahydro-2H-3- benzazepin-2-on-hvdrochlorid

Ein Gemisch von 6,37 g (0,020 Mol) 3-[(Piperidin-3-yl)-methyl]-7,8-dimethoxy-I,3,4,5-tetrahydro-2H-3-benzazepin-2-on, 5,6 ml (0,040 Mol) Triethylamin und 4,90 g (0,020 Mol) 2-(3,4-Dimethoxy-phenyl)-ethylbromid wird 2 Stunden unter Rückfluß erhitzt. Die anfängliche Suspension geht in eine klare Lösung über und beginnt nach etwa 30 Minuten gallertartig auszufallen. Das abgekühlte Reaktionsgemisch wird in einem Gemisch aus 2 molarer Natronlauge und Methylenchlorid gelöst. Die organische Phase wird abgetrennt, mit Wasser gewaschen, über Magnesiumsulfat getrocknet, im Vakuum eingedampft und über 800 g Aluminiumoxid (neutral, Aktivität II-III) mit Methylenchlorid und anschließend mit steigenden Anteilen von Ethanol (bis 2 %) gereinigt. Aus einer Lösung in Aceton wird mit methanolischer Salzsäure das Hydrochlorid gefällt.
Ausbeute: 6,2 g (59,7 % der Theorie),
Schmelzpunkt: 218-219_{°}C
Ber.:C64,79H7,57N5,40
Gef.:64,887,555,21

### Beispiel 2

### 3-[(N-(2-(3,4-Dimethoxy-phenyl)-ethyl)-piperidin-3-yl)-methyl]-7,8-dimethoxy-1,3,4,5-tetrahydro-2H-3- benzazepin-2-on-hvdrobromid

3,7 g (0,0067 Mol) 3-[(N-(2-(3,4-Dimethoxy-phenyl)-ethyl)- pyridinium-3-yl)-methyl]-7,8-dimethoxy-1,3-dihydro-2H-3-benzazepin-2-on-bromid werden in 70 ml Methanol in Anwesenheit von 0,7 g Platindioxid 3 Stunden bei Raumtemperatur und 5 bar hydriert. Der Katalysator wird abgesaugt, das Methanol im Vakuum abdestilliert und der Rückstand in wenig Methanol gelöst und mit Aceton versetzt. Der Niederschlag wird abgesaugt und getrocknet.
Ausbeute: 2,7 g (71,4 % der Theorie),
Schmelzpunkt: 225-227_{°}C
Ber.:C59,68H6,98N4,97
Gef.:59,457,105,00

### Beis^{p}ie) 3

### 3-[(N-(2-(3,4-Dimethoxy-phenyl-ethyl)-piperidin-3-yl-methyl]-7,8-dimethoxy-1,3-dihydro-2H-3-benzazepin-2-on-hvdrochlorid

1,01 g (0,005 Mol) 7,8-Dimethoxy-1,3-dihydro-2H-3-benzazepin-2-on werden in 10 ml Dimethylsulfoxid suspendiert und unter Rühren mit 0,56 g (0,005 Mol) Kalium-tert.butylat versetzt. Nach 45 Minuten wird zu der erhaltenen Lösung unter Rühren 1,7 g (0,005 Mol) N-[2-(3,4-Dimethoxy-phenyl)-ethyl]-3-(brommethyl)-piperidin, gelöst in 5 ml Dimethylsulfoxid, zugetropft. Nach 40 Minuten gießt man auf Eiswasser. Die wässrige Phase wird 3 mal mit Essigester extrahiert. Die vereinigten organischen Phasen werden mit Wasser gewaschen, über Magnesiumsulfat getrocknet, im Vakuum eingedampft und über 200 g Aluminiumoxid (neutral, Aktivität II-III) mit Methylenchlorid und anschließend mit steigenden Anteilen von Ethanol (bis 0,5 %) gereinigt. Aus einer Lösung in Aceton wird mit methanolischer Salzsäure das Hydrochlorid gefällt.
Ausbeute: 0,62 g (23,9 % der Theorie),
Schmelzpunkt: 117-121_{°}C
Ber.:C65,04H7,21N5,42
Gef.:64,867,185,35

### Beispiel 4

### 3-[(N-(2-(3,4-Dimethoxy-phenyl)-ethyl)-piperidin-3-yl)-methyl]-7,8-dimethoxy-2,3,4,5-tetrahydro-1H-3- benzazepin-dihvdrochlorid

Eine Lösung von 0,96 g (0,002 Mol) 3-[(N-(2-(3,4-Dimethoxy-phenyl)-ethyl)-piperidin-3-yl)-methyl]-7,8-dimethoxy-1,3,4,5-tetrahydro-2H-3-benzazepin-2-on in 20 ml Tetrahydrofuran wird unter Stickstoff zu einer Lösung von 0,24 ml (0,002 Mol) Bortrifluoridetherat und 0,3 ml (0,003 Mol) Borandimethylsulfidkomplex (10 molare Lösung in Toluol) zugetropft und anschließend 3 Stunden unter Rückfluß gekocht. Das abgekühlte Reaktionsgemisch wird tropfenweise mit Methanol versetzt. Dann werden 2 ml methanolische Salzsäure zugegeben und 2 Stunden unter Rückfluß erhitzt. Das Methanol und Tetrahydrofuran wird abdestilliert und der Rückstand nach Zugabe von Wasser mit 2 molarer Natronlauge neutralisiert. Der schmierige Niederschlag wird mit Methylenchlorid extrahiert. Die organische Phase wird über Magnesiumsulfat getrocknet, im Vakuum eingedampft und über 50 g Aluminiumoxid (neutral, Aktivität II-III) mit Methylenchlorid und anschließend mit steigenden Anteilen von Ethanol (bis 0,5 %) gereinigt. Aus einer Lösung in Aceton wird mit methanolischer Salzsäure das Dihydrochlorid gefällt.
Ausbeute: 0,28 g (27,7 % der Theorie),
Schmelzpunkt: 238-240°C
Ber.:C62,09H7,81N5,17
Gef.:61,887,845,42

### Beispiel 5

### 3-[(N-(2-(3,4-Dimethoxy-phenyl)-ethyl)-piperidin-3-yl)-methyl]-7,8-dimethoxy-1,3,4,5-tetrahydro-2H-3- benzazepin-2-thion-hvdrochlorid

1,4 g (0,0029 Mol) 3-[(N-(2-(3,4-Dimethoxy-phenyl)-ethyl)-piperidin-3-yl)-methyl]-7,8-dimethoxy-1,3,4,5-tetrahydro-2H-3-benzazepin-2-on und 0,41 g (0,0018 Mol) Phosphorpentasulfid werden in 20 ml Pyridin 3 Stunden auf 100_{°}C erhitzt. Nach Einengung im Vakuum wird der erhaltene Rückstand über 120 g Aluminiumoxid (neutral, Aktivität II-III) mit Essigester-Cyclohexan (80/20) gereinigt. Aus einer Lösung in Aceton wird mit methanolischer Salzsäure das Hydrochlorid gefällt.
Ausbeute: 0,47 g (30,3 % der Theorie),
Schmelzpunkt: 206-207_{°}C
Ber.:C62,84H7,35N5,24S5,99
Gef.:62,547,435,356,15

### Beispiel 6

### 3-[(N-(2-(3,4-Dimethoxy-phenyl)-ethyl)-piperidin-3-yl-methyl]-7,8-dimethoxy-1,3,4,5-tetrahydro-2H-3- benzazepin-1.2-dion

3,8 g (0,0079 Mol) 3-[(N-(2-(3,4-Dimethoxy-phenyl)-ethyl)-piperidin-3-yl)-methyl]-7,8-dimethoxy-1,3,4,5-tetrahydro-2H-3-benzazepin-2-on werden bei 70_{°}C zu einer Suspension von 1,4 g (0,0128 Mol) Selendioxid und 0,8 g Kieselgur in Dioxan/Wasser gegeben und 16 Stunden am Rückfluß gekocht. Nach dem Abkühlen wird mit wenig Ethanol verdünnt und abgesaugt. Das Filtrat wird im Vakuum eingedampft und über 310 g Aluminiumoxid (neutral, Aktivität II-III) mit Methylenchlorid und steigenden Anteilen von Ethanol (bis I %) gereinigt.
Ausbeute: 2,15 g (54,8 % der Theorie),
Schmelzpunkt: 130-132_{°}C
Ber.:C67,72H7,31N5,64
Gef.:67,537,145,65

### Beispiel 7

### 3-[(N-(2-(3,4-Dimethoxy-phenyl-ethyl)-piperidin-3-yl)-methyl]-7,8-dimethoxy-1-hydroxy-1,3,4,5-te- trahvdro-2H-3-benzazepin-2-on-hvdrochiorid

0,70 g (0,0014 Mol) 3-[(N-(2-(3,4-Dimethoxy-phenyl)-ethyl)-piperidin-3-yl)-methyl]-7,8-dimethoxy-1,3,4,5-tetrahydro-2H-3-benzazepin-1,2-dion werden in einem Gemisch aus Methanol-Wasser (95:5) gelöst, mit 0,060 g (0,0016 Mol) Natriumborhydrid versetzt und bei Raumtemperatur 20 Minuten gerührt. Danach wird mit 2 molarer Salzsäure angesäuert, mit Ammoniak neutralisiert und mit Methylenchlorid extrahiert. Die organische Phase wird über Magnesiumsulfat getrocknet, im Vakuum eingedampft und der erhaltene Rückstand über 100 g Aluminiumoxid (neutral, Aktivität II-III) mit Methylenchlorid und anschließend mit steigenden Anteilen von Ethanol (bis 15 %) gereinigt. Aus einer Lösung in Aceton wird mit metha-
nolischer Salzsäure das Hydrochlorid gefällt.
Ausbeute: 0,47 g (62,3 % der Theorie),
Schmelzpunkt: 118-124°C
Ber.:C62,85H7,35N5,24
Gef.:62,607,395,30

### Beispiel 8

### 3-[(N-(2(-4-Amino-phenyl)-ethyl)-piperidin-3-yl)-methyl]-7,8-dimethoxy-1,3,4,5-tetrahydro-2H-3-benzazepin-2-on-dihvdrochlorid

1,7 g (0,0036 Mol) 3-[(N-(2-(4-Nitro-phenyl)-ethyl)-piperidin-3-yl)-methyl]-7,8-dimethoxy-1,3,4,5-te- trahydro-2H-3-benzazepin-2-on werden in 40 ml Methanol in Anwesenheit von 0,3 g 10%iger Palladium/Kohle 2 Stunden bei Raumtemperaur und 5 bar Wasserstoff hydriert. Anschließend wird der Katalysator abgesaugt und das Methanol im Vakuum abdestilliert. Aus einer Lösung des erhaltenen Rückstandes in Aceton wird mit methanolischer Salzsäure das Hydrochlorid gefällt.
Ausbeute: 1,1 g (59,8 % der Theorie),
Schmelzpunkt: 236-240_{°}C
Ber.:C61,17H7,31N8,23
Gef.:60,857,638,12

### Beispiel 9

### 3-[(N-(2-(4-Acetamino-phenyl)-ethyl)-piperidin-3-yl)-methyl]-7,8-dimethoxy-1,3,4,5-tetrahydro-2H-3- benzazepin-2-on-hydrochlorid

0,88 g (0,002 Mol) 3-[(N-(2-(4-Amino-phenyl)-ethyl)-piperidin-3-yl)-methyl]-7,8-dimethoxy-1,3,4,5-te- trahydro-2H-3-benzazepin-2-on und 0,3 ml (0,0022 Mol) Triethylamin werden in ml Methylenchlorid gelöst und unter Rühren 0,16 ml (0,0022 Mol) Acetylchlorid zugetropft. Nach 30 Minuten versetzt man mit Wasser. Die wässrige Phase wird 3 x mit Methylenchlorid extrahiert. Die organische Phase wird über Magnesiumsulfat getrocknet und im Vakuum eingedampft. Aus einer Lösung des erhaltenen Rückstandes in Aceton wird mit methanolischer Salzsäure das Hydrochlorid gefällt.
Ausbeute: 0,61 g (59,1 % der Theorie),
Schmelzpunkt: 187-192°C
Ber.:C65,16H7,42N8,14
Gef.:64,957,457,94

### Beispiel 10

### 3-[(N-(3-(4-Amino-3,5-dibrom-phenoxy)-propyl)-Piperidin-3-yl-methyl]-7,8-dimethoxy-1,3,4,5-tetrahy- dro-2H-3-benzazepin-2-on-hydrochlorid

Hergestellt aus 3-[(Piperidin-3-yl)-methyl]-7,8-dimethoxy-1,3,4,5-tetrahydro-2H-3-benzazepin-2-on und 3-(4-Amino-3,5-dibrom-phenoxy)-propylchlorid analog Beispiel I.
Ausbeute: 20,4 % der Theorie,
Schmelzpunkt: > 95°C (Zers.)
Ber.:C49,00H5,48N6,35Br24,15
Gef.:49,125,805,8324,00

### Beispiel 11

### 3-[(N-(2-(3,4-Dimethoxy-phenyl)-ethyl)-piperidin-3-yl)-methyl]-7,8-methylendioxy-1,3,4,5-tetrahydro-2H-3-benzazepin-2-on-hydrochlorid

Hergestellt aus 3-[(Piperidin-3-yl)-methyl]-7,8-methylendioxy-1,3,4,5-tetrahydro-2H-3-benzazepin-2-on und 2-(3,4-Dimethoxy-phenyl)-ethylbromid analog Beispiel I.
Ausbeute: 31,7 % der Theorie,
Schmelzpunkt: 142-143°C
Ber.:C64,47H7,01N5,57
Gef.:64,367,175,42

### Beispiel 12

### 3-[(N-(3,4-Dimethoxy-benzyl)-piperidin-3-yl)-methyl]-7,8-methylendioxy-1,3,4,5-tetrahydro-2H-3-benzazepin-2-on-hydrochlorid

Hergestellt aus 3-[(Piperidin-3-yl)-methyl]-7,8-methylendioxy-1,3,4,5-tetrahydro-2H-3-benzazepin-2-on und 3,4-Dimethoxy-benzylchlorid analog Beispiel I.
Ausbeute: 30,7 % der Theorie,
Schmelzpunkt: 135°C (Zers.)
Ber.:C63,68H6,80N5,73
Gef.:63,457,025,41

### Beispiel 13

### 3-[(N-(2-Phenylethyl)-piperidin-3-yl)-methyl]-7,8-dimethoxy-1,3,4,5-tetrahydro-2H-3-benzazepin-2- on-hydrochlorid

Hergestellt aus 3-[(Piperidin-3-yl)-methyl]-7,8-dimethoxy-1,3,4,5-tetrahydro-2H-3-benzazepin-2-on und 2-Phenylethylbromid analog Beispiel I.
Ausbeute: 43,5 % der Theorie,
Schmelzpunkt: 241-243_{°}C
Ber.:C68,03H7,69N6,10
Gef.:67,897,896,36

### Beispiel 14

### 3-[(N-(2-(3-Nitro-4-acetamino-phenyl)-ethyl)-piperidin-3-yl)-methyl]-7,8-dimethoxy-1,3,4,5-tetrahydro-2H-3-benzazepin-2-on-hydrochlorid

Hergestellt aus 3-[(Piperidin-3-yl)-methyl]-7,8-dimethoxy-1,3,4,5-tetrahydro-2H-3-benzazepin-2-on und 2-(3-Nitro-4-acetamino-phenyl)-ethylbromid analog Beispiel I.
Ausbeute: 22,3 % der Theorie,
Schmelzpunkt: > 173_{°}C (Zers.)
Ber.:C59,94H6,65N9,99
Gef.:59,926,779,98

### Beispiel 15

### 3-[(N-(2-(3,4,5-Trimethoxy-phenyl)-ethyl)-piperidin-3-yl)-methyl]-7,8-dimethoxy-1,3,4,5-tetrahydro-2H-3-benzazepin-2-on-hvdrochlorid

Hergestellt aus 3-[(Piperidin-3-yl)-methyl]-7,8-dimethoxy-1,3,4,5-tetrahydro-2H-3-benzazepin-2-on und 2-(3,4,5-Trimethoxy-phenyl)-ethylbromid analog Beispiel I.
Ausbeute: 22,6 % der Theorie,
Schmelzpunkt: 135-137_{°}C
Ber.:C63,53H7,53N5,10
Gef.:63,507,825,09

### Beispiel 16

### 3-[(N-(3-(4-Methoxy-phenyl)-propyl)-piperidin-3-yl)-methyl]-7,8-dimethoxy-1,3,4,5-tetrahydro-2H-3- benzazepin-2-on-hydrochlorid

Hergestellt aus 3-[(Piperidin-3-yl)-methyl]-7,8-dimethoxy-1,3,4,5-tetrahydro-2H-3-benzazepin-2-on und 3-(4-Methoxy-phenyl)-propylbromid analog Beispiel I.
Ausbeute: 29,4 % der Theorie,
Schmelzpunkt: 215-218_{°}C
Ber.:C66,85H7,81N5,57
Gef.:66,677,655,53

### Beispiel 17

### 3-[(N-(2-(3,4-Dimethoxy-phenyl)-ethyl)-piperidin-3-yl)-methyl]-7,8-dimethoy-2,3-dihydro-1H-benzazepin

Eine Suspension von 0,06 g (0,0016 Mol) Lithiumaluminiumhydrid in 20 ml absolutem Tetrahydrofuran wird mit 0,31 g (0,00065 Mol) 3-[(N-(2-(3,4-Dimethoxy-phenyl)-ethyl)-piperidin-3-yl)-methyl]-7,8-dimethoxy-1,3-dihydro-2H-3-benzazepin-2-on versetzt und anschließend I Stunde bei Raumtemperatur gerührt. Unter Eiswasserkühlung wird mit 10%iger Ammoniumchlorid-Lösung versetzt und der gebildete Niederschlag abgesaugt. Das Filtrat wird im Vakuum eingeengt, der Rückstand über 30 g Aluminiumoxid (neutral, Aktivität II-III) mit Methylenchlorid gereinigt.
Ausbeute: 0,05 g (16.5 % der Theorie),
Rf-Wert: 0,5 (Aluminiumoxid, Laufmittel: 2 % Ethanol in Methylenchlorid)
Ber.:C72,07H8,21N6,00
Gef.:71,908,395,89

### Beispiel 18

### 3-[(N-(2-(4-Methoxy-phenyl)-ethyl)-piperidin-3-yl)-methyl]-7,8-dimethoxy-1,3,4,5-tetrahydro-2H-3- benzazepin-2-on-hvdrochlorid

Hergestellt aus 3-[(Piperidin-3-yl)-methyl]-7,8-dimethoxy-1,3,4,5-tetrahydro-2H-3-benzazepin-2-on und 2-(4-Methoxy-phenyl)-ethylbromid analog Beispiel I.
Ausbeute:19,8 % der Theorie,
Schmelzpunkt: 227-230°C
Ber.:C66,31H7,63N5,73
Gef.:66,467,575,73

### Beispiel 19

### 3-[(N-(2-(4-Nitro-phenyl-ethyl)-piperidin-3-yl)-methyl]-7,8-dimethoxy-1,3,4,5-tetrahydro-2H-3-benzazepin-2-on-hydrochlorid

Hergestellt aus 3-[(Piperidin-3-yl)-methyl]-7,8-dimethoxy-1,3,4,5-tetrahydro-2H-3-benzazepin-2-on und 2-(4-Nitro-phenyl)-ethylbromid analog Beispiel I.
Ausbeute: 66,8 % der Theorie,
Schmelzpunkt: 239-245°C
Ber.:C61,91H6,80N8,34
Gef.:62,256,668,23

### Beispiel 20

### 3-[(N-(2-(3-Methyl-phenyl)-ethyl)-piperidin-3-yl)-methyl]-7,8-dimethoxy-1,3,4,5-tetrahydro-2H-3-benzazepin-2-on-hvdrochlorid

Hergestellt aus 3-[(Piperidin-3-yl)-methyl]-7,8-dimethoxy-1,3,4,5-tetrahydro-2H-3-benzazepin-2-on und 2-(3-Methyl-phenyl)-ethylbromid analog Beispiel I.
Ausbeute: 38,1 % der Theorie,
Schmelzpunkt: 234-237_{°}C
Ber.:C68,55H7,88N5,92
Gef.:68,687,876,14

### Beispiel 21

### 3-[(N-(2-(3-Methoxy-phenyl)-ethyl)-piperidin-3-yl)-methyl]-7,8-dimethoxy-1,3,4,5-tetrahydro-2H-3- benzazepin-2-on-hydrochlorid

Hergestellt aus 3-[(Piperidin-3-yl)-methyl]-7,8-dimethoxy-1,3,4,5-tetrahydro-2H-3-benzazepin-2-on und 2-(3-Methoxy-phenyl)-ethylbromid analog Beispiel I.
Ausbeute: 23,7 % der Theorie, Schmelzpunkt: 199-202°C
Ber.:C66,31H7,63N5,73
Gef.:66,617,595,91

### Beispiel 22

### 3-[(N-(2-(4-Methyl-phenyl)-ethyl)-piperidin-3-yl)-methyl]-7,8-dimethoxy-1,3,4,5-tetrahydro-2H-3-benzazepin-2-on-hvdrochlorid

Hergestellt aus 3-[(Piperidin-3-yl)-methyl]-7,8-dimethoxy-1,3,4,5-tetrahydro-2H-3-benzazepin-2-on und 2-(4-Methyl-phenyl)-ethylbromid analog Beispiel I.
Ausbeute: 34,7 % der Theorie,
Schmelzpunkt: 233-236°C
Ber.:C68,55H7,88N5,92
Gef.:68,307,895,84

### Beispiel 23

### 3-[(N-(3-(4-Brom-phenyl)-propyl)-piperidin-3-yl)-methyl]-7,8-dimethoxy-1,3,4,5-tetrahydro-2H-3-benzazepin-2-on-hydrochlorid

Hergestellt aus 3-[(Piperidin-3-yl)-methyl]-7,8-dimethoxy-1,3,4,5-tetrahydro-2H-3-benzazepin-2-on und 3-(4-Brom-phenyl)-propylbromid analog Beispiel I.
Ausbeute: 34,8 % der Theorie,
Schmelzpunkt: 100-104_{°}C
Ber.:C58,75H6,57N5,08Br14,48
Gef.:58,406,664,7914,21

### Beispiel 24

### 3-[(N-(2-(3,4-Dimethoxy-phenyl)-ethyl)-piperidin-3-yl)-methyl]-7,8-methylendioUpxy-1,3-dihidro-2H-3- benzazepin-2-on-hvdrochlorid

Hergestellt aus 7,8-Methylendioxy-1,3-dihydro-2H-3-benzazepin-2-on und N-[2-(3,4-Dimethoxyphenyl)-ethyl]-3-(brom-methyl)-piperidin analog Beispiel 3.
Ausbeute: 8,6 % der Theorie,
Schmelzpunkt: 199-201°C
Ber.:C64,73H6,64N5,59
Gef.:64,776,555,57

### Beispiel 25

### 3-[(N-(2-(3,4-Dimethoxyphenyl)-ethyl)-piperidin-2-yl)-ethyl-2]-7,8-dimethoxy-1,3,4,5-tetrahydro-2H-3-benzazepin-2-on-hydrochlorid

Hergestellt aus 3-[(Piperidin-2-yl)-ethyl-2]-7,8-dimethoxy-1,3,4,5-tetrahydro-2H-3-benzazepin-2-on und 2-(3,4-Dimethoxy-phenyl)-ethylbromid analog Beispiel I.
Ausbeute: 23,8 % der Theorie,
Schmelzpunkt: 113-115°C
Ber.:C65,33H7,75N5,26
Gef.:65,117,675,04

### Beispiel 26

### 3-[(N-(2-(3,4-Dimethoxy-phenyl)-ethyl)-hexahydro-azepin-3-yl)-methyl]-7 8-dimethoxy-1,3,4,5-tetrahy- dro-2H-3-benzazepin-2-on-hydrochlorid

660 mg (2 mMol) 3-[(Hexahydro-azepin-3-yl)-methyl]-7,8-dimethoxy-1,3,4,5-tetrahydro-2H-3-benzazepin-2-on und 540 mg (2,2 mMol) 2-(3,4-Dimethoxyphenyl)-ethylbromid werden in 3 ml Triethylamin I Stunde unter Rückfluß gekocht. Das Reaktionsgemisch wird abgekühlt, in Methylenchlorid und 2-molarer Natronlauge aufgenommen. Die alkalische Phase wird abgetrennt und 2 mal mit Methylenchlorid aus- geschüttelt. Die vereinigten organischen Phasen werden über Magnesiumsulfat getrocknet und im Yakuum eingeengt. Die säulenchromatographische Reinigung erfolgt über 100 g Aluminiumoxid (Aktivität II, Elutionsmittel: Methylenchlorid + 0,3 % Äthanol). Die erhaltenen Fraktionen werden im Vakuum eingeengt, der Rückstand in Aceton gelöst und mit etherischer Salzsäure wird das Hydrochlorid gefällt. Ausbeute: 600 mg (56,3 % der Theorie),
Schmelzpunkt: 164-165_{°}C
Ber.:C65,33H7,75N5,25
Gef.:65,127,595,22

### Beispiel 27

### 3-[(N-(3-(4-Amino-3,5-dibrom-phenoxy)-propyl)-hexahydro-azepin-3-yl)-methyl]-7,8-dimethoxy-1,3,4,5-tetrahydro-2H-3-benzazepin-2-on-dihydrochlorid

Hergestellt aus 1,2 g (3,6 mMol) 3-[(Hexahydro-azepin-3-yl)- methyl]-7,8-dimethoxy-1,3,4,5-tetrahy- dro-2H-3-benzazepin-2-on und 1,36 g (3,96 mMol) 3-(4-Amino-3,5-dibrom-phenoxy)-propylchlorid in 5 ml Triethylamin analog Beispiel 26.
Ausbeute: 350 mg (13,7 % der Theorie),
Schmelzpunkt: 134-136°C
Ber.:C47,22H5,52Br22,42N5,36
Gef.:47,405,8622,225,49

### Beispiel 28

### 3-[(N-(2-(3,4-Dimethoxy-phenyl)-ethyl)-hexahydro-azepin-2-yl)-ethyl-21-7.8-dimethoxy-1.3-dihydro-2H-3-benzaze^{p}in-2-on

Hergestellt aus 3,6 g (9,4 mMol) 2-(2-Bromethyl)-I-[2-(3,4-dimethoxy-phenyl)-ethyl]-hexahydro-azepin und 2,06 g (9,4 mMol) 7,8-Dimethoxy-1,3-dihydro-2H-3-benzazepin-2-on analog Beispiel 3. Ausbeute: 1,3 g (27,2 % der Theorie),
Öl, IR-Spektrum (Methylenchlorid): 1655 cm⁻¹ (CO)
Ber.:C70,83H7,93N5,51
Gef.:70,567,805,27

### Beispiel 29

### 3-[(N-(2-(3,4-Dimethoxy-phenyl)-ethyl)-hexahydro-azepin-2-yl)-ethyl-2]-7,8-dimethoxy-1,3,4,5-te- trahydro-2H-3-benzazepin-2-on

1,2 g (2,36 mMol) 3-[N-(2-(3,4-Dimethoxy-phenyl)-ethyl)-hexahydro-azepin-2-yl)-ethyl-2]-7,8-dimethoxy-1,3-dihydro-2H-3-benzazepin-2-on werden in 80 ml Eisessig und 4 Stunden bei 45_{°}C und 5 bar in Anwesenheit von I g 10%igem Palladium/Aktivkohle (10 %) hydriert. Der Katalysator wird abgesaugt, das Filtrat im Vakuum eingeengt, der Rückstand in 100 ml Methylenchlorid gelöst und einmal mit 50 ml 2 n Natronlauge ausgeschüttelt. Die organische Phase wird über Magnesiumsulfat getrocknet und im Vakuum eingeengt. Die Reinigung erfolgt säulenchromatographisch über 100 g Aluminiumoxid (neutral, Elutionsmittel: Methylenchlorid + I % Äthanol).
Ausbeute: 200 mg (17 % der Theorie),
Ber.:C70,56H8,29N5,49
Gef.:70,608,345,37
IR-Spektrum (Methylenchlorid): 1650 cm-¹ (CO)

### Beispiel 30

### 3-[(N-(2-(3,4-Methylendioxy-phenyl)-ethyl)-piperidin-3-ym)-methyl]-7,8-methylendioxy-1,3,4,5-tetrahy- dro-2H-3-benzazepin-2-on-hvdrochlorid

Hergestellt aus 3-[(Piperidin-3-yl)-methyl]-7,8-methylendioxy-1,3,4,5-tetrahydro-2H-3-benzazepin-2-on und 2-(3,4-Methylendioxy-phenyl)-ethylbromid analog Beispiel I.
Ausbeute: 85,7 % der Theorie,
Schmelzpunkt: 234-235_{°}C
Ber.:C66,73H6,03N5,99
Gef.:66,586,315,94

### Beispiel 31

### 3-[(N-(3,4-Dichlor-benzyl)-piperidin-3-yl)-methyl]-7,8-methylendioxy-1,3,4,5-tetrahydro-2H-3-benzazepin-2-on-hvdrochlorid

Hergestellt aus 3-[(Piperidinyl-3-yl)-methyl]-7,8-methylendioxy-1,3,4,5-tetrahydro-2H-3-benzazepin-2-on und 3,4-Dichlor-benzylchlorid analog Beispiel I.
Ausbeute: 80 % der Theorie,
Schmelzpunkt: 240-242_{°}C
Ber.:C57,90H5,47N5,63
Gef.:57,775,355,46

### Beispiel 32

### 3-[(N-(2-(3,4-Dimethoxy-phenyl)-ethyl)-pyrrolidin-3-yl)-methyl]-7 8-dimethoxy-1,3-dihydro-2H-3-benzazepin-2-on

0,79 g (3,6 mMol) 7,8-Dimethoxy-1,3-dihydro-2H-3-benzazepin-2-on werden in 30 ml absolutem Dimethylsulfoxid suspendiert und mit 160 ml (3,6 mMol) 55%iger Natriumhydrid-Dispersion in Öl versetzt. Nach 2-stündigem Rühren bei Raumtemperatur und 1/2-stündigem Rühren bei 40_{°}C versetzt man mit 1,3 g (3 mMol) N-[2-(3,4-Dimethoxy-phenyl)-ethyl]-3-tosyloxy-methyl-pyrrolidin und erwärmt 3 Stunden lang auf 50 bis 55_{°}C. Anschließend wird die abgekühlte Reaktionsmischung in Essigester gelöst, mehrmals mit Wasser und anschließend 2 mal mit 25%iger Essigsäure ausgeschüttelt. Der erhaltene saure Extrakt wird mit 6 molarer Natronlauge alkalisch gestellt und 2 mal mit Methylenchlorid ausgeschüttelt. Nach dem Trocknen der organischen Phase wird das Lösungsmittel im Vakuum entfernt und der erhaltene Rückstand über 100 g Aluminiumoxid (neutral, Aktivität II) mit Methylenchlorid und anschließend mit steigenden Anteilen von Ethanol (bis 2 %) gereinigt.
Ausbeute: 270 mg (19,3 % der Theorie),
IR-Spektrum (Methylenchlorid): 1655 cm-i (CO)
Ber.:C69,50H7,35N6,00
Gef.:69,377,386,12

### Beispiel 33

### 3-[(N-(2-(3,4-Dimethoxy-phenyl)-ethyl)-pyrrolidin-3-yl)-methyl]-7,8-dimethoxy-1,3,4,5-tetrahydro-2H-3-benzazepin-2-on

Hergestellt aus 3-[(N-(2-(3,4-Dimethoxy-phenyl)-ethyl)-pyrrolidin-3-yl)-methyl]-7,8-dimethoxy-1,3-dihydro-2H-3-benzazepin-2-on analog Beispiel 29.
Ausbeute: 21,7 % der Theorie,
IR-Spektrum (Methylenchlorid): 1650 cm⁻¹ (CO)
Ber.:C69,29H7,75N5,98
Gef.:69,207,845,92

### Beispiel 34

### 3-[(N-(3-(3-Methoxy-phenoxy)-propyl)-piperidin-3-yl)-methyl]-7,8-methylendioxy-1,3,4,5-tetrahydro-2H-3-benzazepin-2-on

Hergestellt aus 3-[(Piperidin-3-yl)-methyl]-7,8-methylendioxy-1,3,4,5-tetrahydro-2H-3-benzazepin-2-on und 3-(3-Methoxy-phenoxy)-propylchlorid analog Beispiel I.
Ausbeute: 42 % der Theorie,
Schmelzpunkt: 135-138°C
Ber.:C64,46H7,01N5,57
Gef.:64,467,025,57

### Beispiel 35

### 3-[(N-(3-(3-Methyl-phenoxy)-propyl)-piperidin-3-yl)-methyl]-7,8-methylendioxy-1,3,4,5-tetrahydro-2H-3-benzazepin-2-on-hvdrochlorid

Hergestellt aus 3-[(Piperidin-3-yl)-methyl]-7,8-methylendioxy-1,3,4,5-tetrahydro-2H-3-benzazepin-2-on und 3-(3-Methyl-phenoxy)-propylchlorid analog Beispiel I.
Ausbeute: 34 % der Theorie,
Schmelzpunkt: 122-124°C
Ber.:C65,36H7,32N5,65
Gef.:65,017,615,64

### Beispiel 36

### 3-[(N-(2-(4-Amino-3,5-dichlor-phenyl)-ethyl)-piperidin-3-yl)-methyl]-7,8-dimethoxy-1,3,4,5-tetrahydro-2H-3-benzazepin-2-on-hvdrochlorid

Hergestellt aus 3-[(Piperidin-3-yl)-methyl]-7,8-dimethoxy-1,3,4,5-tetrahydro-2H-3-benzazepin-2-on und 2-(4-Amino-3,5-dichlor-phenyl)-ethylbromid analog Beispiel I.
Ausbeute: 60 % der Theorie,
Schmelzpunkt: 137-140°C
Ber.:C57,03H5,58N6,98
Gef.:57,275,826,59

### Beispiel 37

### 3-[(N-(3-(3,4-Methylendioxy-phenoxy)-propyl)-piperidin-3-yl)-methyl]-7,8-methylendioxy-1,3,4,5-te- trahvdro-2H-3-benzazepin-2-on-hvdrochlorid

Hergestellt aus 3-[(Piperidin-3-yl)-methyl]-7,8-methylendioxy-1,3,4,5-tetrahydro-2H-3-benzazepin-2-on und 3-(3,4-Methylendioxy-phenoxy)-propylchlorid analog Beispiel I.
Ausbeute: 39,9 % der Theorie,
Schmelzpunkt: 127-129_{°}C
Ber.:C62,92H6,43N5,42
Gef.:62,986,415,05

### Beispiel 38

### 3-[(N-(4-(4-Methoxy-phenyl)-butyl)-piperidin-3-yl)-methyl)-7,8-methylendioxy-1,3,4,5-tetrahydro-2H-3-benzazepin-2-on-hvdrochlorid

Hergestellt aus 3-[(Piperidin-3-yl)-methyl]-7,8-methylendioxy-1,3,4,5-tetrahydro-2H-3-benzazepin-2-on und 4-(4-Methoxy-phenyl)-butylbromid analog Beispiel I.
Ausbeute: 42 % der Theorie,
Schmelzpunkt: 158-163°C
Ber.:C67,12H7,44N5,59
Gef.:66,987,275,51

### Beispiel 39

### 3-[(N-(2-(4-Methoxy-phenyl)-ethyl)-piperidin-3-yl)-methyl]-7,8-methylendioxy-1,3,4,5-tetrahydro-2H-3-benzazeoin-2-on-hvdrochlorid

Hergestellt aus 3-[(Piperidin-3-yl)-methyl]-7,8-methylendioxy-1,3,4,5-tetrahydro-2H-3-benzazepin-2-on und 2-(4-Methoxy-phenyl)-ethylchlorid in Dimethylformamid/Kaliumcarbonat bei 120_{°}C analog Beispiel I.
Ausbeute: 55,6 % der Theorie,
Schmelzpunkt: 226-228_{°}C
Ber.:C66,02H7,03N5,92
Gef.:66,187,035,87

### Beispiel 40

### 3-[(N-(2-(Phenoxy)-ethyl)-piperidin-3-yl)-methyl]-7,8-methylendioxy-1,3,4,5-tetrahydro-2H-3-benzaze- _{D}in-2-on-hvdrochlorid

Hergestellt aus 3-[(Piperidin-3-yl)-methyl]-7,8-methylendioxy-1,3,4,5-tetrahydro-2H-3-benzazepin-2-on und 2-Phenoxy-ethylbromid analog Beispiel I.
Ausbeute: 55,7 % der Theorie,
Schmelzpunkt: 124-127°C
Ber.:C64,16H6,98N6,10
Gef.:64,427,026,14

### Beispiel 41

### 3-[(N-(2-(4-Methoxy-phenyl)-ethyl)-piperidin-2-yl)-ethyl-21-7,8-methylendioxy-1,3,4,5-tetrahydro-2H-3-benzazepin-2-on-hydrochlorid

Hergestellt aus 3-[(Piperidin-2-yl)-ethyl-2]-7,8-methylendioxy-1,3,4,5-tetrahydro-2H-3-benzazepin-2-on und 2-(4-Methoxy-phenyl)-ethylbromid analog Beispiel I.
Ausbeute: 34,6 % der Theorie,
Schmelzpunkt: 110-115°C
Ber.:C66,58H7,24N5,75
Gef.:66,507,185,70

### Beispiel 42

### 3-[(N-(4-Methoxy-phenyl)-methyl)-piperidin-2-yl)-ethyl-2]-7,8-methylendioxy-1,3,4,5-tetrahydro-2H-3- benzazepin-2-on-hydrochlorid

Hergestellt aus 3-[(Piperidin-2-yl)-ethyl-2]-7,8-methylendioxy-1,3,4,5-tetrahydro-2H-3-benzazepin-2-on und 4-Methoxy-benzylbromid analog Beispiel I.
Ausbeute: 52 % der Theorie,
Schmelzpunkt: 148-152°C
Ber.:C66,02H7,03N5,92
Gef.:65,907,105,98

### Beispiel 43

### 3-[(N-(3,4-Dimethoxy-phenyl)-methyl)-piperidin-2-yl)-ethyl-2]-7,8-methylendioxy-1,3,4,5-tetrahydro-2H-3-benzazeDin-2-on-hvdrobromid

Hergestellt aus 3-[(Piperidin-2-yl)-ethyl-2]-7,8-methylendioxy-1,3,4,5-tetrahydro-2H-3-benzazepin-2-on und 3,4-Dimethoxy-benzylbromid analog Beispiel I.
Ausbeute: 27 % der Theorie,
Schmelzpunkt: 138-140_{°}C
Ber.:C59,23H6,42N5,11
Gef.:59,406,495,23

### Beispiel 44

### 3-[(N-(2-(4-Nitro-phenyl)-ethyl)-piperidin-2-yl)-ethyl-2]-7,8-methylendioxy-1,3,4,5-tetrahydro-2H-3- benzazepin-2-on-hydrochlorid

Hergestellt aus 3-[(Piperidin-2-yl)-ethyl-2]-7,8-methylendioxy-1,3,4,5-tetrahydro-2H-3-benzazepin-2-on und 2-(4-Nitro-phenyl)-ethylbromid analog Beispiel I.
Ausbeute: 22 % der Theorie,
Schmelzpunkt: 130-132°C
Ber.:C62,20H6,43N8,37
Gef.:62,166,578,32

### Beispiel 45

### 3-[(N-(2-(3-Trifluormethylphenyl)-ethyl)-piperidin-3-yl)-methyl]-7,8-dimethoxy-1,3,4,5-tetrahydro-2H-3-benzazepin-2-on-hydrochlorid

Hergestellt aus 3-[(Piperidin-3-yl)-methyl]-7,8-dimethoxy-1,3,4,5-tetrahydro-2H-3-benzazepin-2-on und 2-(3-Trifluormethyl-phenyl)-ethylbromid analog Beispiel I.
Ausbeute: 32 % der Theorie,
Schmelzpunkt: ab 150_{°}C (Zers.)
Ber.:C61,53H6,50N5,32
Gef.:61,706,425,27
Rf-Wert: 0,36 (Kieselgel, Methylenchlorid/Methanol = 10/1)

### Beispiel 46

### 3-[(N-(3-(3,5-Dimethoxy-phenoxy)-propyl)-piperidin-3-yl)-methyl]-7,8-methylendioxy-1,3,4,5-tetrahy- dro-2H-3-benzazepin-2-on-hvdrochlorid

Hergestellt aus 3-[(Piperidin-3-yl)-methyl]-7,8-methylendioxy-1,3,4,5-tetrahydro-2H-3-benzazepin-2-on und 3-(3,5-Dimethoxy-phenoxy)-propylchlorid analog Beispiel I.
Ausbeute: 46,4 % der Theorie,
Schmelzpunkt: 102-107°C
Ber.:C63,09H7,00N5,25
Gef.:62,966,865,50

### Beispiel 47

### 3-[(N-(2-Phenyl-ethyl)-piperidin-2-yl)-ethyl-2]-7,8-dimethoxy-1,3,4,5-tetrahydro-2H-3-benzazepin-2- on-hvdrochlorid

Hergestellt aus 3-[(Piperidin-2-yl)-ethyl-2]-7,8-dimethoxy-2H-3-benzazepin-2-on und 2-Phenylethylbromid analog Beispiel I.
Ausbeute: 37 % der Theorie,
Schmelzpunkt: 130-132°C
Ber.:C68,55H7,88N5,92
Gef.:68,427,975,75

### Beispiel 48

### 3-[(N-(3-(4-Methoxy-phenyl)-propyl)-piperidin-2-yl)-ethyl-2]-7,8-dimethoxy-1,3,4,5-tetrahydro-2H-3- benzaze^{p}in-2-on-hvdrochlorid

Hergestellt aus 3-[(Piperidin-2-yl)-ethyl-2]-7,8-dimethoxy-2H-3-benzazepin-2-on und 3-(4-Methoxyphenyl)-propylbromid analog Beispiel I.
Ausbeute: 43 % der Theorie,
Schmelzpunkt: 109-112°C
Ber.:C67,36H7,99N5,42
Gef.:67,197,885,38

### Beispiel 49

### 3-[(N-(2-(3-Methyl-phenyl)-ethyl)-piperidin-2-yl)-ethyl-2]-7,8-dimethoxy-1,3,4,5-tetrahydro-2H-3-benzazepin-2-on-hydrochlorid

Hergestellt aus 3-[(Piperidin-2-yl)-ethyl-2]-7,8-dimethoxy-2H-3-benzazepin-2-on und 2-(3-Methylphenyl)-ethylbromid analog Beispiel I.
Ausbeute: 31 % der Theorie,
Schmelzpunkt: 124-126°C
Ber.:C69,04H8,07N5,75
Gef.:68,917,695,72

### Beispiel 50

### 3-[(N-(2-(4-Methoxy-phenyl)-ethyl)-piperidin-2-yl)-ethyl-2]-7,8-dimethoxy-1,3,4,5-tetrahydro-2H-3- benzazepin-2-on-hvdrochlorid

Hergestellt aus 3-[(Piperidin-2-yl)-ethyl-2]-7,8-dimethoxy-2H-3-benzazepin-2-on und 2-(4-Methoxyphenyl)-ethylbromid analog Beispiel I.
Ausbeute: 48 % der Theorie,
Schmelzpunkt: 112-114°C
Ber.:C66,85H7,81N5,57
Gef.:66,697,865,65

### Beispiel 51

### 3-[(N-(2-(4-Nitro-phenyl)-ethyl)-piperidin-2-yl)-ethyl-2]-7,8-dimethoxy-1,3,4,5-tetrahydro-2H-3-benzazepin-2-on-hydrochlorid

Hergestellt aus 3-[(Piperidin-2-yl)-ethyl-2]-7,8-dimethoxy-2H-3-benzazepin-2-on und 2-(4-Nitrophenyl)-ethylbromid analog Beispiel I.
Ausbeute: 8 % der Theorie,
Schmelzpunkt: 126-128_{°}C
Ber.:C62,59H7,00N8,11
Gef.:62,566,918,16

### Beispiel 52

### 3-[(N-(2-(4-Methyl-phenyl)-ethyl)-piperidin-2-yl)-ethyl-2]-7,8-dimethoxy-1,3,4,5-tetrahydro-2H-3- benzazepin-2-on-hydrochlorid

Hergestellt aus 3-[(Piperidin-2-yl)-ethyl-2]-7,8-dimethoxy-2H-3-benzazepin-2-on und 2-(4-Methylphenyl)-ethylbromid analog Beispiel I.
Ausbeute: 23 % der Theorie,
Schmelzpunkt: 109-111°C
Ber.:C69,04H8,07N5,75
Gef.:68,847,906,03

### Beispiel 53

### 3-[(N-(2-(3-Methoxy-phenyl)-ethyl)-piperidin-2-yl)-ethyl-2]-7,8-dimethoxy-1,3,4,5-tetrahydro-2H-3- benzazepin-2-on-hydrochlorid

Hergestellt aus 3-[(Piperidin-2-yl)-ethyl-2]-7,8-dimethoxy-2H-3-benzazepin-2-on und 2-(3-Methoxyphenyl)-ethylbromid analog Beispiel I.
Ausbeute: 25 % der Theorie,
Schmelzpunkt: 125-127°C
Ber.:C66,85H7,81N5,57
Gef.:65,687,675,20

### Beispiel 54

### 3-[(N-(2-(3,4,5-Trimethoxy-phenyl)-ethyl)-piperidin-2-yl)-ethyl-2]-7,8-dimethoxy-1,3,4,5-tetrahydro-2H-3-benzazepin-2-on-hvdrochlorid

Hergestellt aus 3-[(Piperidin-2-yl)-ethyl-2]-7,8-dimethoxy-2H-3-benzazepin-2-on und 2-(3,4,5-Trimethoxyphenyl)-ethylbromid analog Beispiel I.
Ausbeute: 15 % der Theorie,
Schmelzpunkt: 138-140°C
Ber.:C63,98H7,70N4,97
Gef.:63,747,554,65

### Beispiel 55

### 3-[(N-(2-(3-Methoxy-4-methansulfonyloxy-phenyl)-ethyl)-piperidin-3-yl)-methyl]-7,8-dimethoxy-1,3,4,5-tetrahydro-2H-3-benzazepin-2-on-hydrochlorid

Hergestellt aus 3-[(N-(2-(3-Methoxy-4-hydroxy-phenyl)-ethyl)-piperidin-3-yl)-methyl]-7,8-dimethoxy-1,3,4,5-tetrahydro-2H-3-benzazepin-2-on-hydrochlorid und Methansulfonsäurechlorid analog Beispiel 9.
Ausbeute: 66 % der Theorie,
Schmelzpunkt: 202-204_{°}C
Ber.:C57,67H6,74N4,80S5,50
Gef.:57,726,914,876,31

### Beispiel 56

### 3-[(N-(2-(3-Methoxy-4-hydroxy-phenyl)-ethyl)-piperidin-3-yl)-methyl]-7,8-dimethoxy-1,3,4,5-tetrahy- dro-2H-3-benzazeDin-2-on

### a) 3-[(N-(2-(4-Benzyloxy-3-methoxy-phenyl)-ethyl)-piperidin-3-yl)-methyl]-7,8-dimethoxy-1,3,4,5-te- trahvdro-2H-3-benzazeDin-2-on

Hergestellt aus 3-[(Piperidin-3-yl)-methyl]-7,8-dimethoxy-1,3,4,5-tetrahydro-2H-3-benzazepin-2-on und 2-(4-Benzyloxy-3-methoxy-phenyl)-ethylbromid analog Beispiel I.
Ausbeute: 56 % der Theorie,
Schmelzpunkt: 216-217°C
Ber.:C68,61H7,28N4,71
Gef.:68,807,384,73

### b) 3-[(N-(2-(3-Methoxy-4-hydroxy-phenyl)-ethyl)-piperidin-3-yl)-methyl]-7,8-dimethoxy-1,3,4,5-te- trahydro-2H-3-benzazepin-2-on

Hergestellt aus 3-[(N-(2-(4-Benzyloxy-3-methoxy-phenyl)-ethyl)-piperidin-3-yl)-methyl]-7,8-dimethoxy-1,3,4,5-tetrahydro-2H-3-benzazepin-2-on in Eisessig analog Beispiel 8.
Ausbeute: 77 % der Theorie,
Schmelzpunkt: 173-175°C
Ber.:C69,21H7,74N5,98
Gef.:69,077,796,06

### Beispiel 57

### 3-[N-(2-(2-Fluorphenyl)-ethyl)-piperidin-3-yl)-methyl]-7,8-dimethoxy-1,3,4,5-tetrahydro-2H-3-benza- ze_{D}in-2-on-dihvdrochlorid

Hergestellt aus 3-[(Piperidin-3-yl)-methyl]-7,8-dimethoxy-1,3,4,5-tetrahydro-2H-3-benzazepin-2-on und 2-(2-Fluorphenyl)-ethylbromid analog Beispiel I.
Ausbeute: 49 % der Theorie,
Schmelzpunkt: ab 210°C
Ber.:C60,82H6,87N5,46
Gef.:60,886,735,60
R_{f}-Wert: 0,33 (Kieselgel, Methylenchlorid/Methanol = 1_{0/1})

### Beispiel 58

### 3-[N-(2-(4-Fluorphenyl)-ethyl)-piperidin-3-yl)-methyl]-7,8-dimethoxy-1,3,4,5-tetrahydro-2H-3-benzazepin-2-on-hydrochlorid

Hergestellt aus 3-[(Piperidin-3-yl)-methyl]-7,8-dimethoxy-1,3,4,5-tetrahydro-2H-3-benzazepin-2-on und 2-(4-Fluorphenyl)-ethylbromid analog Beispiel I.
Ausbeute: 56 % der Theorie,
Schmelzpunkt: 245°C (Zers.)
Ber.:C65,47H7,18N5,87
Gef.:65,787,255,99 R_{f}-Wert: 0,31 (Kieselgel, Methylenchlorid/Methanol =10/l)

### Beispiel I

### Tabletten zu 7.5 ma 3-[(N-(2-(3,4-Dimethoxy-phenyl)-ethyl)-piperidin-3-yl)-methyl]-7,8-dimethoxy-1,3,4,5-tetrahydro-2H-3-benzazepin-2-on

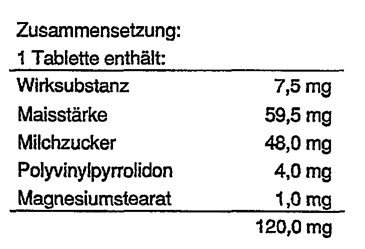

### Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45_{°}C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten. Tablettengewicht: 120 mg

### Beispiel 11

### Dragees zu 5 mg 3-[(N-(2-(3,4-Dimethoxy-phenyl)-ethyl)-piperidin-3-yl)-methyl]-7,8-dimethoxy-1,3,4,5-tetrahydro-2H-3-benzazepin-2-on

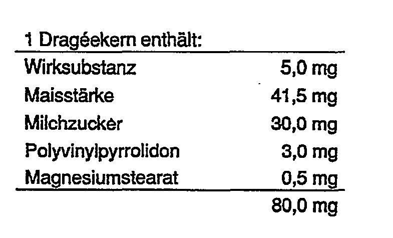

### Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit I mm-Maschenweite, trocknet bei ca. 45_{°}C und schlägt das Granulat anschließend durch dasselbe Sieb. Nach dem Zumischen von Magnesiumstearat werden auf einer Tablettiermaschine gewölbte Drageekerne mit einem Durchmesser von 6 mm gepreßt. Die so hergestellten Drageekerne werden auf bekannte Weise mit einer Schicht überzogen, die im wesentlichen aus Zucker und Talkum besteht. Die fertigen Dragees werden mit Wachs poliert. Dragéegewicht:130 mg

### Beispiel III

### Amullen zu 5 mg 3-[(N-(2-(3,4-Dimethoxy-phenyl)-ethyl)-piperidin-3-yl)-methyl]-7,8-dimethoxy-1,3,4,5-tetrahvdro-2H-3-benzazepin-2-on

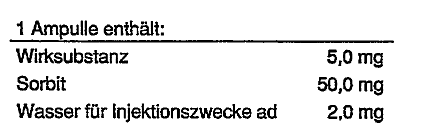

### Herstellungsverfahren

In einem geeigneten Ansatzgefäß wird der Wirkstoff in Wasser für Injektionszwecke gelöst und die Lösung mit Sorbit isotonisch gestellt.

Nach Filtration über einem Membranfilter wird die Lösung unter N₂-Begasung in gereinigte und sterilisierte Ampullen abgefüllt und 20 Minuten im strömenden Wasserdampf autoklaviert.

### Beispiel IV

### Suppositorien zu 10 mg 3-[(N-(2-(3,4-Dimethoxy-phenyl)-ethyl)-piperidin-3-yl)-methyl]-7,8-dimethoxy-1,3,4,5-tetrahydro-2H-3-benzazepin-2-on

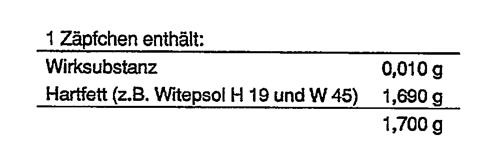

### Herstellunasverfahren:

Das Hartfett wird geschmolzen. Bei 38_{°}C wird die gemahlene Wirksubstanz in der Schmelze homogen dispergiert. Es wird auf 35_{°}C abgekühlt und in schwach vorgekühlte Suppositorienformen ausgegossen.

### Beispiel

### Tropfenlösung mit 10 ma 3-[(N-(2-(3,4-Dimethoxyphenyl)-ethyl)-piperidin-3-yl)-methyl]-7,8-dimethoxy-1,3,4,5-tetrahydro-2H-3-benzazepin-2-on

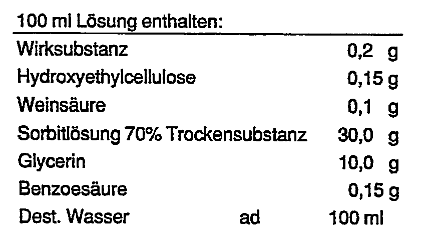

### Herstellungsverfahren:

Dest. Wasser wird auf 70_{°}C erhitzt. Hierin wird unter Rühren Hydroxyethylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

## Patentansprüche (Patentansprüche für folgende(n) Vertragsstaat(en) : BE, CH, DE, FR, GB, IT, LI, LU, NL, SE)

1. Neue cyclische Aminderivate der allgemeinen Formel 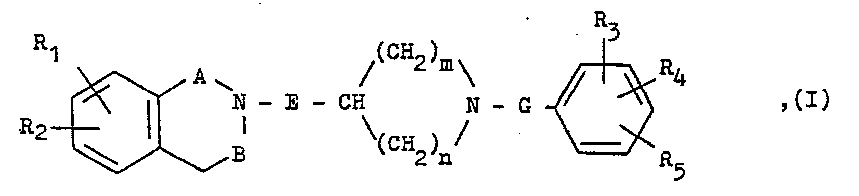
in der
A eine 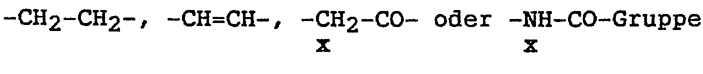 und
B eine Methylen-, Carbonyl- oder Thiocarbonylgruppe oder
A eine 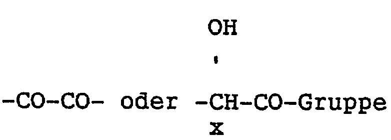 und B eine Methylengrupp wobei das mit x gekennzeichnete Atom jeweils mit dem Phenylkern verknüpft ist,
E eine gegebenenfalls durch eine Alkylgruppe mit 1 bis 3 Kohlenstoffatomen substituierte geradkettige Alkylengruppe mit 1 bis 3 Kohlenstoffatomen,
G eine gegebenenfalls durch eine Alkylgruppe mit 1 bis 3 Kohlenstoffatomen substituierte geradkettige Alkylengruppe mit 1 bis 5 Kohlenstoffatomen, wobei die zu dem Phenylkern benachbarte Methylengruppe einer gegebenenfalls durch eine Alkylgruppe substituierten geradkettigen Alkylengruppe mit 3 bis 5 Kohlenstoffatomen durch ein Sauerstoff- oder Schwefelatom, eine Imino-, Methylimino-, Sulfinyl-oder Sulfonylgruppe ersetzt sein kann,
R₁ ein Wasserstoff- oder Halogenatom, eine Trifluormethyl-, Nitro-, Amino-, Alkylamino-, Dialkylamino-, Alkyl-, Hydroxy-, Alkoxy- oder Phenylalkoxygruppe, wobei jeder Alkylteil jeweils I bis 3 Kohlenstoffatome enthalten kann,
R₂ ein Wasserstoffatom oder Halogenatom, eine Hydroxy-, Alkoxy-, Phenylalkoxy- oder Alkylgruppe, wobei jeder Alkylteil jeweils bis 3 Kohlenstoffatome enthalten kann, oder
Ri und R₂ zusammen eine Alkylendioxygruppe mit I oder 2 Kohlenstoffatomen,
R₃ ein Wasserstoff- oder Halogenatom, eine Alkyl- oder Alkoxygruppe mit jeweils I bis 3 Kohlenstoffatomen im Alkylteil, eine Hydroxy-, Nitro-, Cyano- oder Trifluormethylgruppe,
R₄ ein Wasserstoffatom, eine Alkoxy-, Alkansulfonyloxy-, Amino-, Alkylamino- oder Dialkylaminogruppe mit jeweils I bis 3 Kohlenstoffatomen in jedem Alkylteil, oder eine Alkanoylaminogruppe mit 2 oder 3 Kohlenstoffatomen im Alkanoylteil oder
R₃ und R₄ zusammen eine Alkylendioxygruppe mit I oder 2 Kohlenstoffatomen,
Rₛ ein Wasserstoff- oder Halogenatom, eine Hydroxy-, Alkyl- oder Alkoxygruppe mit jeweils I bis 3 Kohlenstoffatomen im Alkylteil,
m die Zahl I, 2, 3, 4 oder 5 und
n die Zahl 0, I oder 2, wobei jedoch n + m die Zahl 3, 4 oder 5 darstellen muß, bedeuten, deren Enantiomeren, deren Diastereomeren und deren Säureadditionssalze.

2. Neue cyclische Aminderivate der allgemeinen Formel gemäß Anspruch I, in der A, B, m und n wie im Anspruch 1 definiert sind, wobei jedoch m+ n die Zahl 3, 4 oder 5 darstellen muß,
E eine Methylen- oder Ethylengruppe,
G eine n-Alkylengruppe mit 1 bis 4 Kohlenstoffatomen, wobei die zu dem Phenylkern benachbarte Methylengruppe einer n-Propylen- oder n-Butylengruppe durch ein Sauerstoff- oder Schwefelatom, eine Imino-, Methylimino-, Sulfinyl- oder Sulfonylgruppe ersetzt sein kann,
R₁ ein Wasserstoff-, Fluor-, Chlor- oder Bromatom, eine Hydroxy-, Methoxy-, Trifluormethyl-, Methylamino- oder Dimethylaminogruppe,
R₂ ein Wasserstoff-, Chlor- oder Bromatom oder eine Methoxygruppe oder
R₁ und R₂ zusammen eine Methylendioxygruppe,
R₃ ein Wasserstoff-, Fluor-, Chlor- oder Bromatom, eine Methyl-, Hydroxy-, Methoxy- oder Nitrogruppe,
R₄ ein Wasserstoffatom, eine Methoxy-, Methansulfonyloxy-, wino- oder Acetylaminogruppe oder
Rs und R₄ zusammen eine Methylendioxygruppe und
Rs ein Wasserstoff-, Chlor- oder Bromatom oder eine Methoxygruppe bedeuten, deren Enantiomeren, deren Diastereomeren und deren Säureadditionssalze.

3. Neue cyclische Aminderivate der allgemeinen Formel gemäß Anspruch 1, in der
m und n wie im Anzpruch 1 definiert sind, wobei jedoch m+ n die Zahl 3, 4 oder 5 darstellen muß,
A eine -CH₂-CH₂- oder -CH=CH-Gruppe und B eine Methylen- oder Carbonylgruppe oder
A eine -CO-CO-Gruppe und B eine Methylengruppe,
E eine Methylen- oder Ethylengruppe,
G eine n-Alkylengruppe mit 2 bis 4 Kohlenstoffatomen, wobei die zu dem Phenylkern benachbarte Methylengruppe einer n-Propylen- oder n-Butylengruppe durch ein Sauerstoffatom ersetzt sein kann,
Ri ein Wasserstoffatom oder eine Methoxygruppe,
R₂ ein Wasserstoffatom oder eine Methoxygruppe oder
R₁ und R₂ zusammen eine Methylendioxygruppe,
R₃ ein Wasserstoffatom, eine Methyl-, Hydroxy- oder Methoxygruppe,
R₄ ein Wasserstoffatom oder eine Methoxygruppe oder
R₃ und R₄ zusammen eine Methylendioxygruppe und
R₅ ein Wasserstoffatom bedeuten, deren Enantiomeren, deren Diastereomeren und deren Säureadditionssalze.

4. 3-[(N-(2-(3, 4-Dimethoxy-phenyl)-ethyl)-piperidin-3-yl)methyl]-7, 8-dimethoxy-1, 3, 4, 5-tetrahy- dro-2H-3-benzazepin-2-on, dessen Enantiomere und dessen Säureadditionssalze.

4. 3-[(N-(2-(3,4-Dimethoxy-phenyl)-ethyl)-piperidin-3-yl)-methyl]-7,8-dimethoxy-1,3,4,5-tetrahydro-2H-3-benzazepin-2-on, dessen Enantiomere und dessen Säureadditionssalze.

5. 3-[(N-(2-(3,4-Dimethoxy-phenyl)-ethyl)-piperidin-2-yl)-ethyl-2]-7,8-dimethoxy-1,3,4,5-tetrahydro-2H-3-benzazepin-2-on, dessen Enantiomere und dessen Säureadditionssalze.

6. Physiologisch verträgliche Säureadditionssalze der Verbindungen gemäß den Ansprüchen bis 5 mit anorganischen oder organischen Säuren.

7. Arzneimittel, enthaltend eine Verbindung der allgemeinen Formel gemäß den Ansprüchen I bis 5 oder dessen physiologisch verträgliches Säureadditionssalz gemäß Anspruch 6 neben einem oder mehreren inerten Trägerstoffen und/oder Verdünnungsmitteln.

8. Arzneimittel gemäß Anspruch 7 geeignet zur Behandlung von Sinustachykardien und ischämischen Herzerkrankungen.

9. Verfahren zur Herstellung eines Arzneimittels gemäß den Ansprüchen 7 und 8, dadurch gekennzeichnet, daß auf nichtchemischem Wege eine Verbindung gemäß den Ansprüchen I bis 5 oder dessen physiologisch verträgliches Säureadditionssalz gemäß Anspruch 6 in einen oder mehreren inerten Trägerstoffen und/oder Verdünnungsmitteln eingearbeitet wird.

10. Verfahren zur Herstellung von neuen cyclischen Aminderivaten gemäß den Ansprüchen I bis 6, dadurch gekennzeichnet, daß
a.) eine Verbindung der allgemeinen Formel 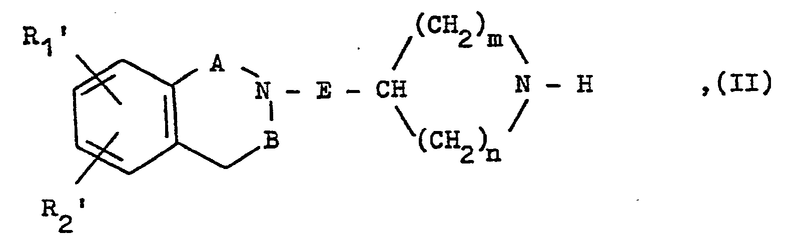 in der
A, B, E, m und n wie in den Ansprüchen I bis 5 definiert sind,
Ri' einen durch einen Schutzrest geschützte Hydroxy-, Amino- oder Alkylaminogruppe darstellt oder die für R₁ in den Ansprüchen I bis 5 erwähnten Bedeutungen besitzt und
R₂' einen durch einen Schutzrest geschützte Hydroxygruppe darstellt oder die für R₂ in den Ansprüchen I bis 5 erwähnten Bedeutungen besitzt, mit einer Verbindung der allgemeinen Formel 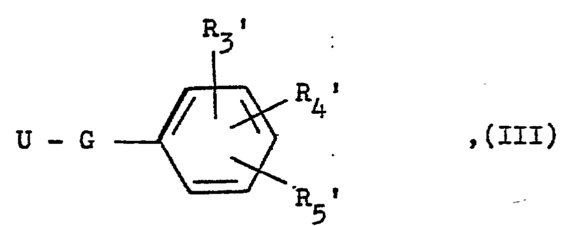 in der
R₃', R₄' und Rs' die für Rs, R₄ und R₅ in den Ansprüchen I bis 5 erwähnten Bedeutungen besitzen, wobei jedoch die in den Resten R₃ bis R₅ enthaltenen Hydroxy-, Amino- oder Alkylaminogruppen durch einen Schutzrest geschützt sein können, und
U eine nukleophile Austrittsgruppe wie ein Halogenatom oder eine Sulfonyloxygruppe darstellt, umgesetzt und gegebenenfalls anschließend ein verwendeter Schutzrest abgespalten wird oder
b.) zur Herstellung von Verbindungen der allgemeinen Formel I, in der G mit Ausnahme der eine Sulfenyl-, Sulfinyl- oder Sulfonylgruppe enthaltenden Reste die für G in den Ansprüchen I bis 5 erwähnten Bedeutungen besitzt, A die -CH₂-CH₂-Gruppe, B die Methylen- oder Carbonylgruppe und m + n die Zahl 4 darstellen, eine Verbindung der allgemeinen Formel 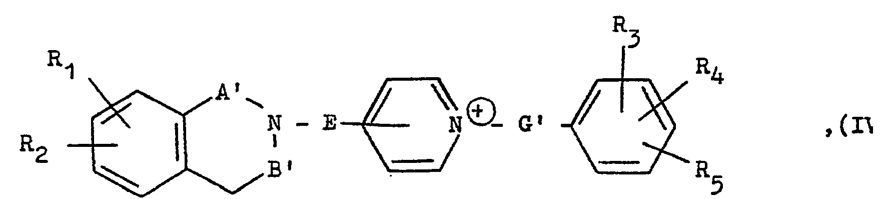 in der Rᵢ bis R₅ und E wie in den Ansprüchen 1 bis 5 definiert sind, G, mit Ausnahme der ein Schwefelatom, eine Sulfinyl- oder Sulfonylgruppe enthaltenden Reste die für G in den Ansprüchen 1 bis 5 erwähnten Bedeutungen besitzt, A, die -CH=CH- oder -CH₂CH₂-Gruppe und B, die Methylen- oder Carbonylgruppe darstellen, in einem Lösungsmittel oder Lösungsmittelgemisch hydriert wird oder
c.) zur Herstellung von Verbindungen der allgemeinen Formel I, in der B die Carbonyl- oder Methylengruppe darstellt, eine Verbindung der allgemeinen Formel 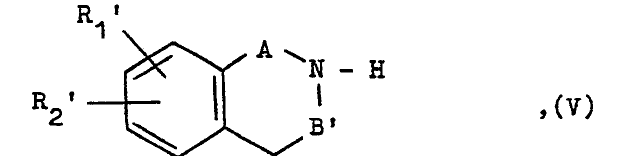 in der A wie in den Ansprüchen 1 bis 5 definiert ist, Ri, eine durch einen Schutzrezt geschützte Hydroxy-, Amino- oder Alkylaminogruppe darstellt oder die für R₁ in den Ansprüchen 1 bis 5 erwähnten Bedeutungen besitzt, R₂, eine durch einen Schutzrest geschützte Hydroxygruppe darstellt oder die für R₂ in den Ansprüchen 1 bis 5 erwähnten Bedeutungen besitzt, und B' eine Carbonyl- oder Methylengruppe darstellt, mit einer Verbindung der allgemeinen Formel 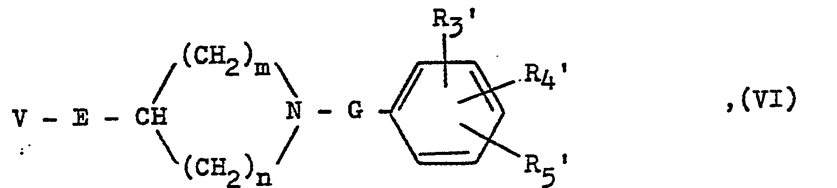 in der
E, G, m und n wie in den Ansprüchen I bis 5 definiert sind,
R₃', R₄' und R₅' die für R₃, R₄ und R₅ in den Ansprüchen I bis 5 erwähnten Bedeutungen besitzen, wobei jedoch den Resten R₃ bis R₅ enthaltenen Hydroxy-, Amino- oder Alkylaminogruppen durch einen Schutzrest geschützt sein können, und
V eine nukleophile Austrittsgruppe wie ein Halogenatom oder eine Sulfonyloxygruppe darstellt, umgesetzt und gegebenenfalls anschließend ein verwendeter Schutzrest abgespalten wird oder
d) zur Herstellung von Verbindungen der allgemeinen Formel I, in der A eine -CH₂-CH₂ oder - CH=CH-Gruppe und B eine Thiocarbonylgruppe darstellen, eine Verbindung der allgemeinen Formel 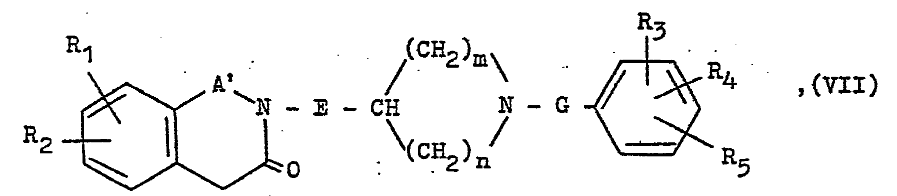 in der
R₁ bis Rs, E, G, m und n wie in den Ansprüchen I bis 5 definiert sind und
A' eine -CH₂-CH₂- oder -CH=CH-Gruppe darstellt, mit einem schwefeleinführenden Mittel umgesetzt wird oder
e) zur Herstellung von Verbindungen der allgemeinen Formel I,
in der A eine 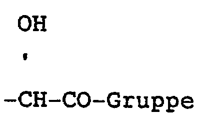 und B eine Methylengruppe dar-
stellen, eine Verbindung der allgemeinen Formel 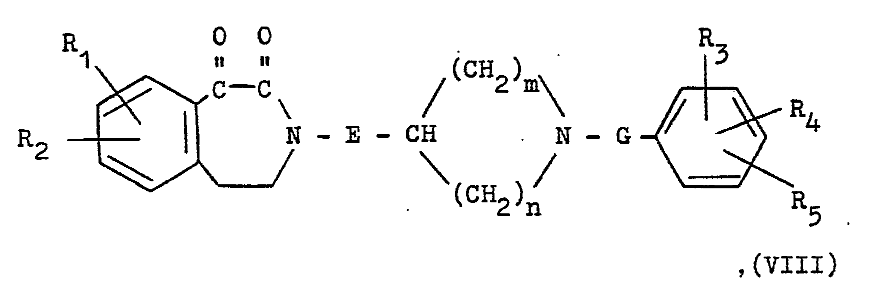 in der
R₁ bis Rₛ, E, G, m und n wie in den Ansprüchen I bis 5 definiert sind, reduziert wird oder
f) zur Herstellung von Verbindungen der allgemeinen Formel I, in der A eine -CH₂-CH₂- oder - CH=CH- und B eine Methylengruppe darstellen, eine Verbindung der allgemeinen Formel 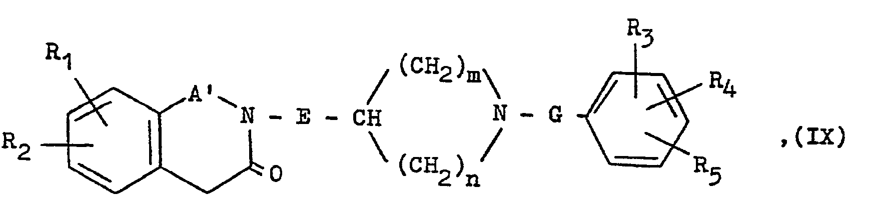 in der
R₁ bis R₅, E, G, m und n wie in den Ansprüchen I bis 5 definiert sind und
A' eine -CH₂-CH₂- oder -CH=CH-Gruppe darstellt, reduziert wird oder
g) zur Herstellung von Verbindungen der allgemeinen Formel I, in der A die -COCO-Gruppe darstellt, eine Verbindung der allgemeinen Formel 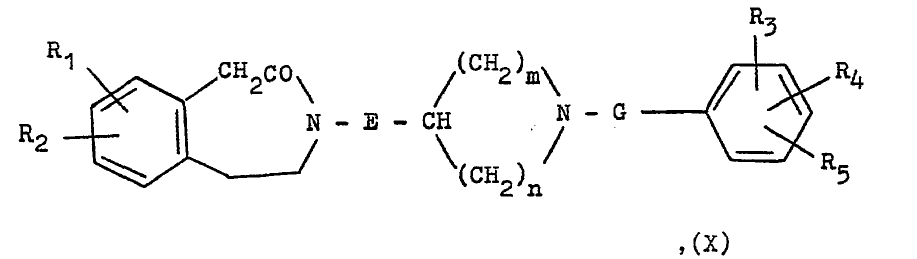 in der
R₁ bis Rₛ, E, G, m und n wie in den Ansprüchen I bis 5 definiert sind, oxidiert wird oder
h) zur Herstellung von Verbindungen der allgemeinen Formel I, in der G mit Ausnahme der ein Schwefelatom, eine Sulfinyl- oder Sulfonylgruppe enthaltenden Reste die für G in den Ansprüchen I bis 5 erwähnten Bedeutungen besitzt, A die -CH₂-CH₂-Gruppe und B die Methylen- oder Carbonylgruppe darstellen, eine Verbindung der allgemeinen Formel 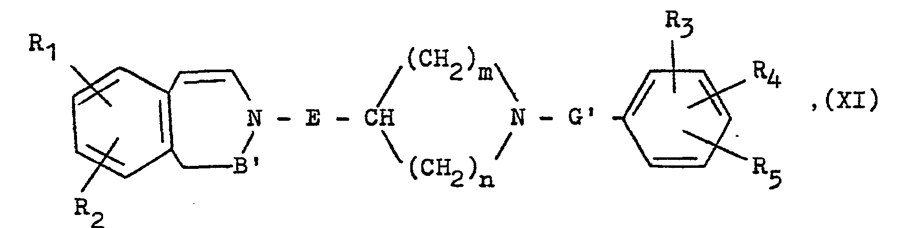 in der R₁ bis Rs, E, m und n wie in den Ansprüchen I bis 5 definiert sind,
G' mit Ausnahme der ein Schwefelatom, eine Sulfinyl- oder Sulfonylgruppe enthaltenden Reste die für G in den Ansprüchen 1 bis 5 erwähnten Bedeutungen besitzt und B' die Methylen- oder Carbonylgruppe darstellt, hydriert wird
und anschließend gewünschtenfalls eine so erhaltene Verbindung der allgemeinen Formel I, in der Ri und/oder R₃ eine Nitrogruppe darstellt, mittels Reduktion in eine entsprechende Aminoverbindung der allgemeinen Formel übergeführt wird oder
eine so erhaltene Verbindung der allgemeinen Formel I, in der R₄ eine Hydroxy- oder Aminogruppe darstellt, mittels Acylierung in eine entsprechende Alkansulfonyloxy- oder Alkanoylaminoverbindung der allgemeinen Formel übergeführt wird oder
eine so erhaltene Verbindung der allgemeinen Formel I, welche mindestens ein chirales Zentrum enthält, in ihre Diastereomeren oder in ihre Enantiomeren aufgetrennt wird und/oder
eine so erhaltene Verbindung der allgemeinen Formel I in ihre Säureadditionssalze, insbesondere in ihre physiologisch verträglichen Säureadditionssalze mit anorganischen oder organischen Säuren übergeführt wird.

11. Verfahren nach Anspruch 10, dadurch gekennzeichnet, daß die Umsetzung in einem Lösungsmittel durchgeführt wird.

12. Verfahren nach den Ansprüchen 10a oder 10c, dadurch gekennzeichnet,daß die Umsetzung in einem Lösungsmittel und in Gegenwart eines säurebindenden Mittels durchgeführt wird.

## Patentansprüche (Patentansprüche für folgende(n) Vertragsstaat(en) : AT, ES, GR)

I. Verfahren zur Herstellung von neuen cyclischen Aminderivaten der allgemeinen Formel 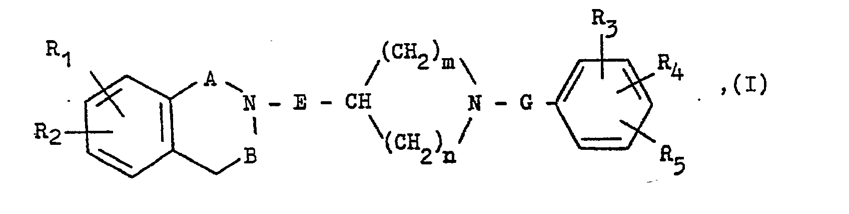
in der A eine 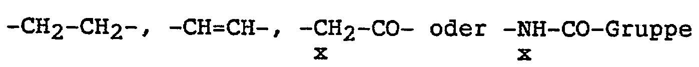 und
B eine Methylen-, Carbonyl- oder Thiocarbonylgruppe oder A eine 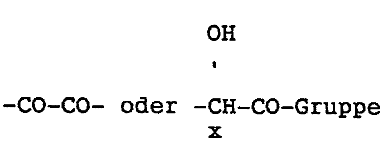 und B eine Methylengruppe, wobei das mit x gekennzeichnete Atom jeweils mit dem Phenylkern verknüpft ist,
E eine gegebenenfalls durch eine Alkylgruppe mit 1 bis 3 Kohlenstoffatomen substituierte geradkettige Alkylengruppe mit 1 bis 3 Kohlenstoffatomen,
G eine gegebenenfalls durch eine Alkylgruppe mit I bis 3 Kohlenstoffatomen substituierte geradkettige Alkylengruppe mit I bis 5 Kohlenstoffatomen, wobei eine zu einem Phenylkern benachbarte Methylengruppe einer gegebenenfalls durch eine Alkylgruppe substituierten geradkettigen Alkylengruppe mit 3 bis 5 Kohlenstoffatomen durch ein Sauerstoff- oder Schwefelatom, eine Imino-, Methylimino-, Sulfinyl-oder Sulfonylgruppe ersetzt sein kann,
R₁ ein Wasserstoff- oder Halogenatom, eine Trifluormethyl-, Nitro-, Amino-, Alkylamino-, Dialkylamino-, Alkyl-, Hydroxy-, Alkoxy- oder Phenylalkoxygruppe, wobei jeder Alkylteil jeweils I bis 3 Kohlenstoffatome enthalten kann,
R₂ ein Wasserstoffatom oder Halogenatom, eine Hydroxy-, Alkoxy-, Phenylalkoxy- oder Alkylgruppe, wobei jeder Alkylteil jeweils I bis 3 Kohlenstoffatome enthalten kann, oder
R₁ und R₂ zusammen eine Alkylendioxygruppe mit I oder 2 Kohlenstoffatomen,
R₃ ein Wasserstoff- oder Halogenatom, eine Alkyl- oder Alkoxygruppe mit jeweils I bis 3 Kohlenstoffatomen im Alkylteil, eine Hydroxy-, Nitro-, Cyano- oder Trifluormethylgruppe,
R₄ ein Wasserstoffatom, eine Alkoxy-, Alkansulfonyloxy-, Amino-, Alkylamino- oder Dialkylaminogruppe mit jeweils I bis 3 Kohlenstoffatomen in jedem Alkylteil, oder eine Alkanoylaminogruppe mit 2 oder 3 Kohlenstoffatomen im Alkanoylteil oder
R₃ und R₄ zusammen eine Alkylendioxygruppe mit I oder 2 Kohlenstoffatomen,
Rs ein Wasserstoff- oder Halogenatom, eine Hydroxy-, Alkyl- oder Alkoxygruppe mit jeweils I bis 3 Kohlenstoffatomen im Alkylteil,
m die Zahl I, 2, 3, 4 oder 5 und
n die Zahl Q, I oder 2, wobei jedoch n + m die Zahl 3, 4 oder 5 darstellen muß, bedeuten, von deren Enantiomeren, von deren Diastereomeren und von deren Säureadditionssalzen, dadurch gekennzeichnet, daß
a.) eine Verbindung der allgemeinen Formel 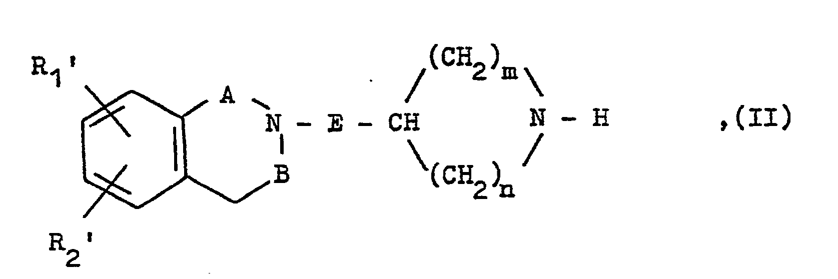 in der
A, B, E, m und n wie eingangs definiert sind,
R₁' einen durch einen Schutzrest geschützte Hydroxy-, Amino- oder Alkylaminogruppe darstellt oder die für R₁ eingangs erwähnten Bedeutungen besitzt und
R₂' einen durch einen Schutzrest geschützte Hydroxygruppe darstellt oder die für R₂ eingangs erwähnten Bedeutungen besitzt, mit-einer Verbindung der allgemeinen Formel 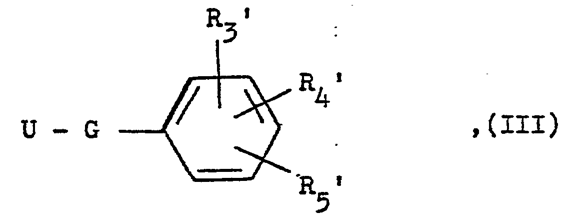 in der
R₃', R₄' und Rs' die für Rs, R₄ und Rs eingangs erwähnten Bedeutungen besitzen, wobei jedoch die in den Resten R₃ bis R₅ enthaltenen Hydroxy-, Amino- oder Alkylaminogruppen durch einen Schutzrest geschützt sein können, und
U eine nukleophile Austrittsgruppe wie ein Halogenatom oder eine Sulfonyloxygruppe darstellt, umgesetzt und gegebenenfalls anschließend ein verwendeter Schutzrest abgespalten wird oder
b.) zur Herstellung von Verbindungen der allgemeinen Formel I, in der G mit Ausnahme der eine Sulfenyl-, Sulfinyl- oder Sulfonylgruppe enthaltenden Reste die für G eingangs erwähnten Bedeutungen besitzt, A die -CH₂-CH₂-Gruppe, B die Methylen- oder Carbonylgruppe und m + n die Zahl 4 darstellen, eine Verbindung der allgemeinen Formel 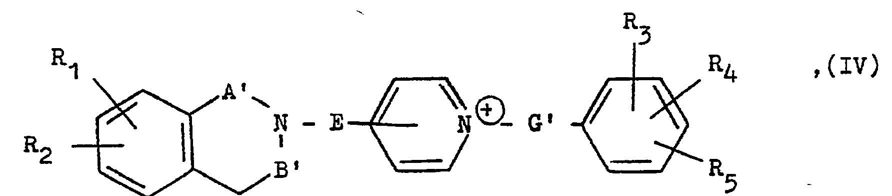 in der
R₁ bis R₅ und E wie eingangs definiert sind,
G' mit Ausnahme der ein Schwefelatom, eine Sulfinyl- oder Sulfonylgruppe enthaltenden Reste die für G in den Ansprüchen 1 bis 5 erwähnten Bedeutungen besitzt,
A' die -CH=CH- oder -CH₂CH₂-Gruppe und
B' die Methylen- oder Carbonylgruppe darstellen, hydriert wird oder
c.) zur Herstellung von Verbindungen der allgemeinen Formel I, in der B die Carbonyl- oder Methylengruppe darstellt, eine Verbindung der allgemeinen Formel 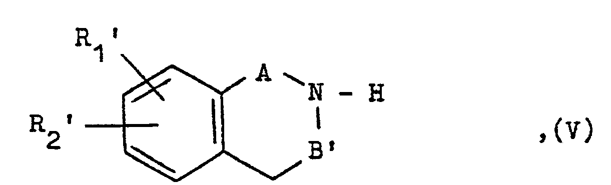 in der
A wie eingangs definiert ist,
R₁' eine durch einen Schutzrest geschützte Hydroxy-, Amino- oder Alkylaminogruppe darstellt oder die für R₁ eingangs erwähnten Bedeutungen besitzt,
R2', eine durch einen Schutzrest geschützte Hydroxygruppe darstellt oder die für R₂ eingangs erwähnten Bedeutungen besitzt, und
B' eine Carbonyl- oder Methylengruppe darstellt, mit einer Verbindung der allgemeinen Formel in der
E, G, m und n wie eingangs definiert sind,
R₃', R₄' und R₅' die für Rs, R₄ und R₅ eingangs erwähnten Bedeutungen besitzen, wobei jedoch die in den Resten R₃ bis R₅ enthaltenen Hydroxy-, Amino- oder Alkylaminogruppen durch einen Schutzrest geschützt sein können, und
V eine nukleophile Austrittsgruppe wie ein Halogenatom oder eine Sulfonyloxygruppe darstellt, umgesetzt und gegebenenfalls anschließend ein verwendeter Schutzrest abgespalten wird oder
d) zur Herstellung von Verbindungen der allgemeinen Formel I, in der A eine -CH₂-CH₂- oder - CH=CH-Gruppe und B eine Thiocarbonylgruppe darstellen, eine Verbindung der allgemeinen Formel 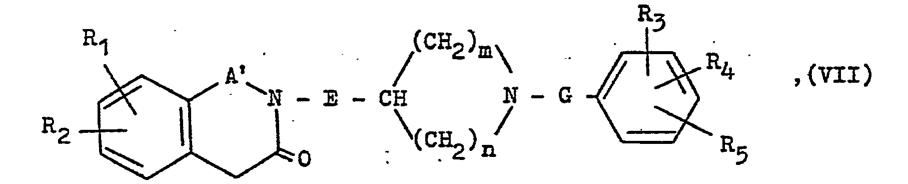 in der
R₁ bis R₅, E, G, m und n wie eingangs definiert sind und
A' eine -CH₂-CH₂- oder -CH=CH-Gruppe darstellt, mit einem schwefeleinführenden Mittel umgesetzt wird oder
e) zur Herstellung von Verbindungen der allgemeinen Formel I, in der A eine 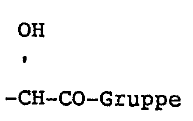 und B eine Methylengruppe darstellen, eine Verbindung der allgemeinen Formel 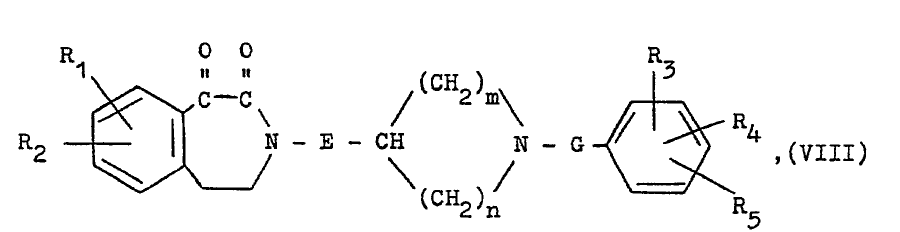 in der
R₁ bis Rₛ, E, G, m und n wie eingangs definiert sind, in einem geeigneten Lösungsmittel reduziert wird oder
f) zur Herstellung von Verbindungen der allgemeinen Formel I, in der A eine -CH₂-CH₂- oder - CH=CH- und B eine Methylengruppe darstellen, eine Verbindung der allgemeinen Formel 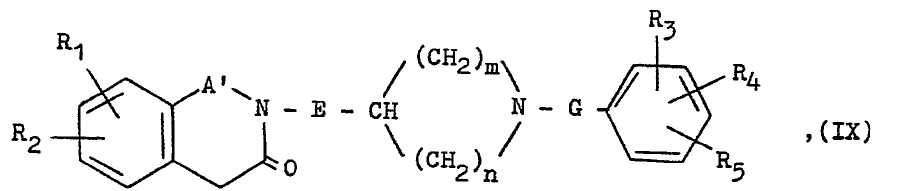 in der
R₁ bis Rₛ, E, G, m und n wie eingangs definiert sind und
A' eine -CH₂-CH₂- oder -CH=CH-Gruppe darstellt, reduziert wird oder
g) zur Herstellung von Verbindungen der allgemeinen Formel I, in der A die -COCO-Gruppe darstellt, eine Verbindung der allgemeinen Formel 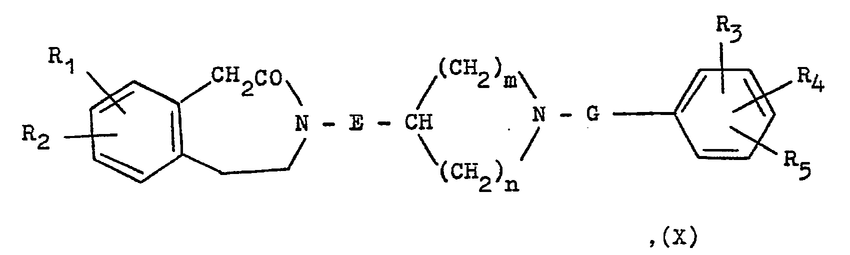 in der
Ri bis Rs, E, G, m und n wie eingangs definiert sind,
oxidiert wird oder
h) zur Herstellung von Verbindungen der allgemeinen Formel I, in der G mit Ausnahme der ein Schwefelatom, eine Sulfinyl- oder Sulfonylgruppe enthaltenden Reste die für G eingangs erwähnten Bedeutungen besitzt, A die -CH₂-CH₂-Gruppe und B die Methylen- oder Carbonylgruppe darstellen, eine Verbindung der allgemeinen Formel 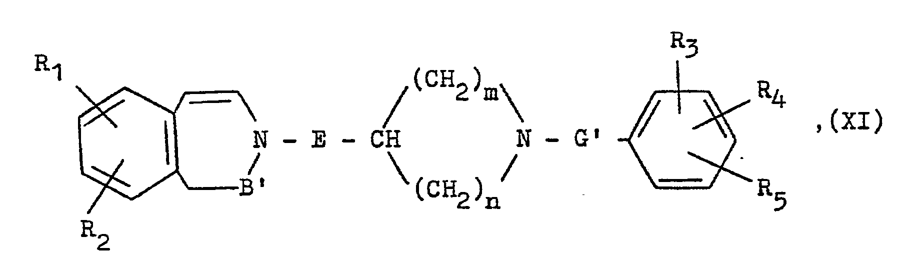 in der
Ri bis R₅, E, m und n wie eingangs definiert sind,
G' mit Ausnahme der ein Schwefelatom, eine Sulfinyl- oder Sulfonylgruppe enthaltenden Reste die für G eingangs erwähnten Bedeutungen besitzt und
B' die Methylen- oder Carbonylgruppe darstellt, hydriert wird
und anschließend gewünschtenfalls eine so erhaltene Verbindung der allgemeinen Formel I, in der R₁ und/oder R₃ eine Nitrogruppe darstellt, mittels Reduktion in eine entsprechende Aminoverbindung der allgemeinen Formel I übergeführt wird oder
eine so erhaltene Verbindung der allgemeinen Formel I, in der R₄ eine Hydroxy- oder Aminogruppe darstellt, mittels Acylierung in eine entsprechende Alkansulfonyloxy- oder Alkanoylaminoverbindung der allgemeinen Formel I übergeführt wird oder
eine so erhaltene Verbindung der allgemeinen Formel I, welche mindestens ein chirales Zentrum enthält, in ihre Diastereomeren oder in ihre Enantiomeren aufgetrennt wird und/oder
eine so erhaltene Verbindung der allgemeinen Formel I in ihre Säureadditionssalze, insbesondere in ihre physiologisch verträglichen Säureadditionssalze mit anorganischen oder organischen Säuren übergeführt wird.

2. Verfahren nach Anspruch I zur Herstellung von neuen cyclischen Aminderivaten der allgemeinen Formel 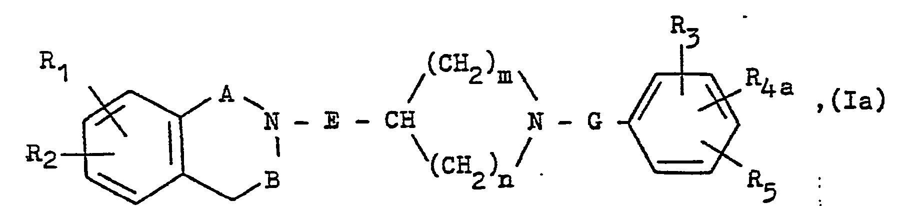 in der
Ri bis R₃' Rs, A, B, E, G, m und n wie im Anspruch I definiert sind und
R₄a ein Wasserstoffatom, eine Alkoxy-, Amino-, Alkylamino- oder Dialkylaminogruppe mit jeweils I bis 3 Kohlenstoffatomen in jedem Alkylteil, oder eine Alkanoylaminogruppe mit 2 oder 3 Kohlenstoffatomen im Alkanoylteil oder
R₃ und R₄a zusammen eine Alkylendioxygruppe mit 1 oder 2 Kohlenstoffatomen bedeuten, von deren Enantiomeren, von deren Diastereomeren und von deren Säureadditionssalzen, dadurch gekennzeichnet, daß
a.) eine Verbindung der allgemeinen Formel 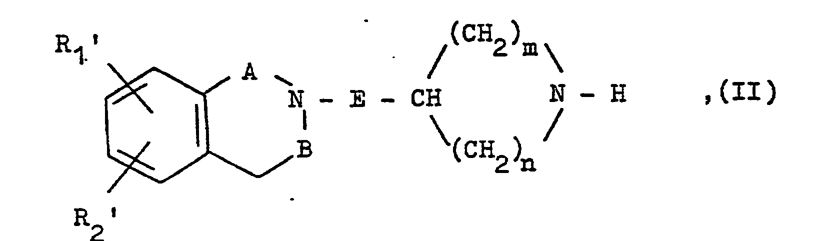 in der
R₁', R2', A, B, E, m und n wie im Anspruch I definiert sind, mit einer Verbindung der allgemeinen Formel 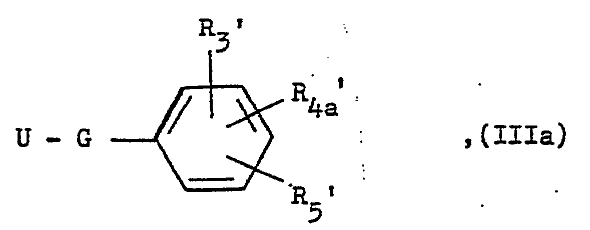 in der
Rs', Rs', G und U wie im Anspruch I definiert sind und
R₄ₐ' die für R₄a eingangs erwähnten Bedeutungen besitzt, wobei jedoch die in den Resten R₃, R₄ₐ und Rs enthaltenen Hydroxy-, Amino- oder Alkylaminogruppen durch einen Schutzrest geschützt sein können, umgesetzt und gegebenenfalls anschließend ein verwendeter Schutzrest abgespalten wird oder
b.) zur Herstellung von Verbindungen der allgemeinen Formel la, in der G mit Ausnahme der eine Sulfenyl-, Sulfinyl- oder Sulfonylgruppe enthaltenden Reste die für G eingangs erwähnten Bedeutungen besitzt, A die -CH₂-CH₂-Gruppe, B die Methylen- oder Carbonylgruppe und m + n die Zahl 4 darstellen, eine Verbindung der allgemeinen Formel 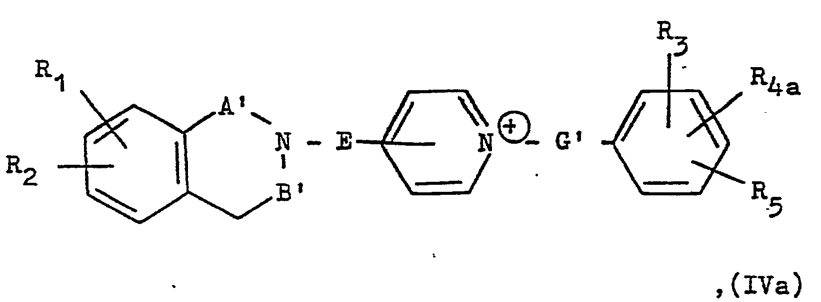 in der
R₁ bis Rs, R₅, A', B', E und G, wie im Anspruch 1 definiert sind und
R₄a wie eingangs definiert ist, in einem Lösungsmittel oder Lösungsmittelgemisch hydriert wird oder
c) zur Herstellung von Verbindungen der allgemeinen Formel la, in der B die Carbonyl- oder Methylengruppe darstellt, eine Verbindung der allgemeinen Formel 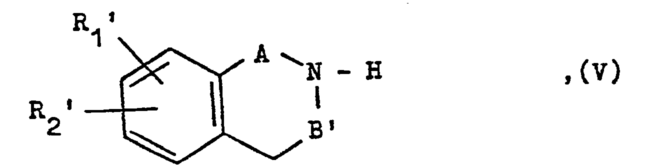 in der R₁', R_{2'}, A und B, wie im Anspruch 1 definiert sind, mit einer Verbindung der allgemeinen Formel 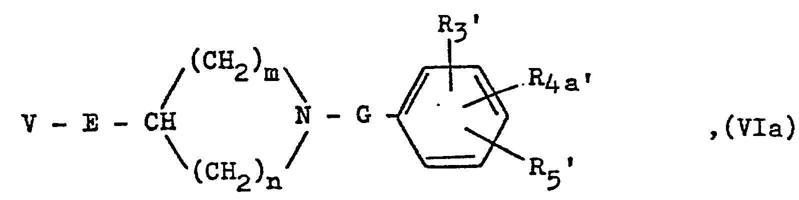 in der
R_{3'}, Rs', E, G, V, m und n wie im Anspruch 1 definiert sind und
R₄' die für R₄ eingangs erwähnten Bedeutungen besitzt, wobei jedoch die in den Resten R₃, R₄ₐ und Rs enthaltenen Hydroxy-, Amino- oder Alkylaminogruppen durch einen Schutzrest geschützt sein können, umgesetzt und gegebenenfalls anschließend ein verwendeter Schutzrest abgespalten wird oder
d) zur Herstellung von Verbindungen der allgemeinen Formel la,
in der A eine -CH₂-CH₂- oder -CH=CH-Gruppe und B eine Thiocarbonylgruppe darstellen, eine Verbindung der allgemeinen Formel 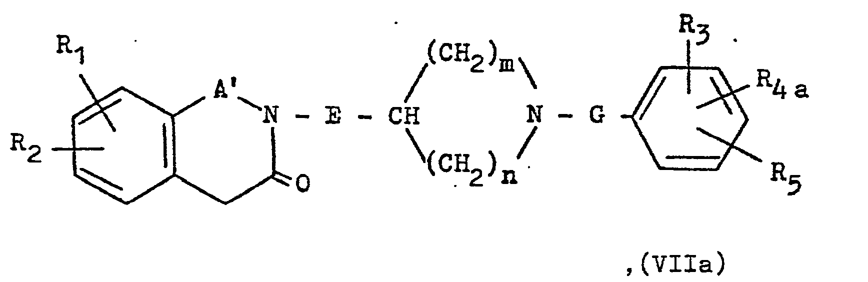 in der
R₁ bis R₃, Rs, A', E, G, m und n wie im Anspruch 1 definiert sind und
R₄ₐ wie eingangs definiert ist, mit einem schwefeleinführenden Mittel umgesetzt wird oder
e) zur Herstellung von Verbindungen der allgemeinen Formel la, in der A eine 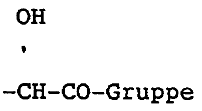 und B eine Methylengruppe dar. stellen, eine Verbindung der allgemeinen Formel 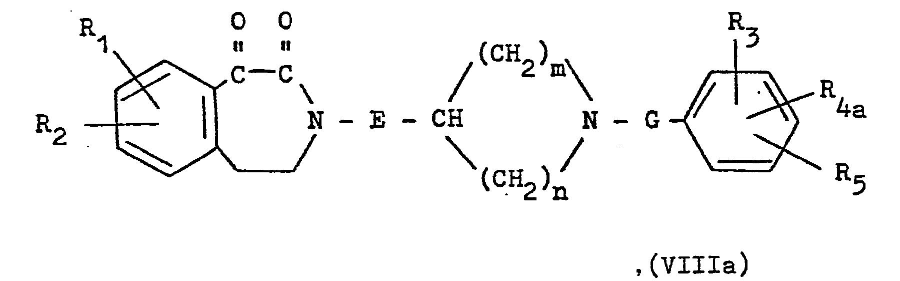 in der
R₁ bis Rs, R₅, E, G, m und n wie im Anspruch 1 definiert sind und
R₄a wie eingangs definiert ist, in einem geeigneten Lösungsmittel reduziert wird oder
f) zur Herstellung von Verbindungen der allgemeinen Formel la, in der A eine -CH₂-CH₂- oder - CH=CH- und Beine Methylengruppe darstellen, eine Verbindung der allgemeinen Formel 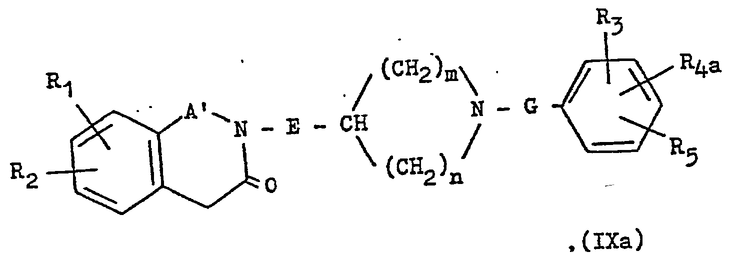
in der R₁ bis R₃, Rₛ, A', E, G, m und n wie im Anspruch 1 definiert sind und
R₄ₐ wie eingangs definiert ist, in einem geeigneten Lösungsmittel reduziert wird oder
g) zur Herstellung von Verbindungen der allgemeinen Formel la, in der A die -COCO-Gruppe darstellt, eine Verbindung der allgemeinen Formel 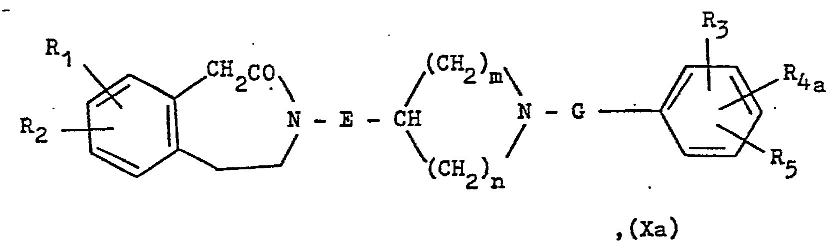 in der
Ri bis R₃, R₅, E, G, m und n wie im Anspruch 1 definiert sind und
R₄a wie eingangs definiert ist, in einem geeigenten Lösungsmittel oder Lösungsmittelgemisch oxidiert wird oder
h) zur Herstellung von Verbindungen der allgemeinen Formel la, in der G mit Ausnahme der ein Schwefelatom, eine Sulfinyl- oder Sulfonylgruppe enthaltenden Reste die für G eingangs erwähnten Bedeutungen besitzt, A die -CH₂-CH₂-Gruppe und B die Methylen- oder Carbonylgruppe darstellen, eine Verbindung der allgemeinen Formel 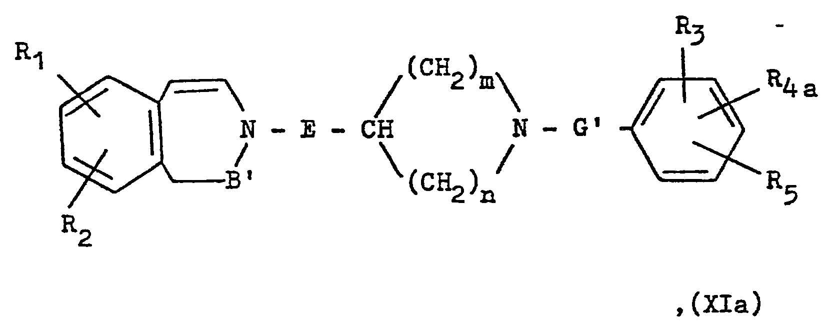 in der
R₁ bis R₃, Rs, B', E, G', m und n wie im Anspruch 1 definiert sind und
R₄a wie eingangs definiert ist, in einem Lösungsmittel oder Lösungsmittelgemisch hydriert wird
und anschließend gewünschtenfalls eine so erhaltene Verbindung der allgemeinen Formel la, in der R₁ und/oder R₃ eine Nitrogruppe darstellt, mittels Reduktion in eine entsprechende Aminoverbindung der allgemeinen Formel la übergeführt wird oder
eine so erhaltene Verbindung der allgemeinen Formel la, in der R₄ₐ eine Aminogruppe darstellt, mittels Alkanoylierung in eine entsprechende Alkanoylaminoverbindung der allgemeinen Formel la übergeführt wird oder eine so erhaltene Verbindung der allgemeinen Formel la, welche mindestens ein chirales Zentrum enthält, in ihre Diastereomeren oder in ihre Enantiomeren wird und/oder
eine so erhaltene Verbindung der allgemeinen Formel la in ihre Säureadditionssalze, insbesondere in ihre physiologisch verträglichen Säureadditionssalze mit anorganischen oder organischen säuren übergeführt wird.

3. Verfahren nach den Ansprüchen I und 2, dadurch gekennzeichnet, daß die Umsetzung in einem Lösungsmittel durchgeführt wird.

4. Verfahren nach den Ansprüchen le, 2e und 3, dadurch gekennzeichnet, daß die Umsetzung mit ei- nem Metallhydrid wie Natriumborhydrid bei Temperaturen zwischen 0 und 80_{°}C, vorzugsweise bei Tem- peraturen zwischen 15 und 40_{°}C, durchgeführt wird.

5. Verfahren nach den Ansprüchen If, 2f und 3, dadurch gekennzeichnet, daß die Umsetzung mit ei- nem Metallhydrid wie Lithiumaluminiumhydrid oder Diboran oder mit einem Komplex aus Boran und einem Thioäther, z.B. mit dem Komplex aus Diboran und Dimethylsulfid, bei Temperaturen zwischen 0 und 25_{°}C, vorzugsweise jedoch bei Temperaturen zwischen 10 und 25_{°}C, durchgeführt wird.

6. Verfahren nach den Ansprüchen Ig, 2g und 3, dadurch gekennzeichnet, daß die Oxidation mit Kali- umpermanganat, Selendioxid oder Natriumdichromat bei Temperaturen zwischen 0 und 100_{°}C durchge- führt wird.

7. Verfahren zur Herstellung eines Arzneimittels, dadurch gekennzeichnet, daß auf nicht-chemischem Wege eine Verbindung der allgemeinen Formel 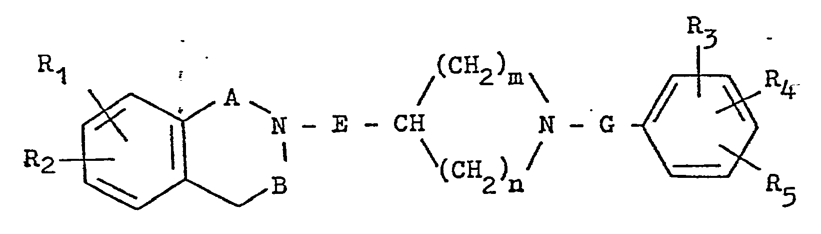 wherein in der
R₁ bis R₅, A, B, E, G, m und n wie im Anspruch I definiert sind, oder dessen physiologisch verträgliches Säureadditionssalz in einen oder mehrere inerte Trägerstoffe und/oder Verdünnungsmittel eingearbeitet wird.

## Claims (Claims for the following Contracting State(s) : s: BE, CH, DE, FR, GB, IT, LI, LU, NL, SE)

1. New cyclic amine derivatives of general formula 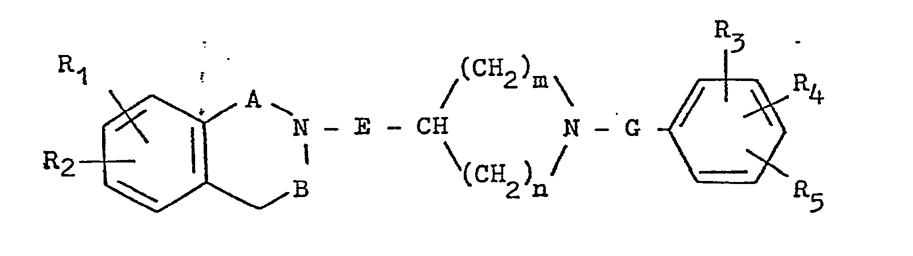 wherein A represents a 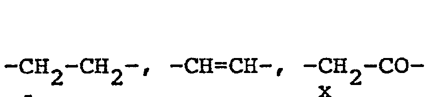 or 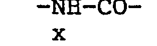 group and B represents a methylene, carbonyl or thiocarbonyl group or A represents a 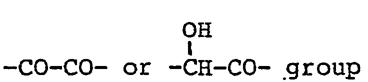 and B represents a methylene group, whilst die atom marked x is linked to the phenyl nucleus,
E represents a straight-chained alkylene group with 1 to 3 carbon atoms optionally substituted by an alkyl group with to 3 carbon atoms,
G represents a straight-chained alkylene group with 1 to 5 carbon atoms optionally substituted by an alkyl group with 1 to 3 carbon atoms, whilst a methylene group, adjacent to a phenyl nucleus, of a straight-chained alkylene group with 3 to 5 carbon atoms optionally substituted by an alkyl group may be replaced by an oxygen or sulphur atom or by an imino, methylimino, sulphinyl or sulphonyl group,
R₁ represents a hydrogen or halogen atom, a trifluoromethyl, nitro, amino, alkylamino, dialkylamino, alkyl, hydroxy, alkoxy or phenylalkoxy group, whilst each alkyl moiety may contain from 1 to 3 carbon atoms,
R₂ represents a hydrogen or halogen atom, a hydroxy, alkoxy, phenylalkoxy or alkyl group, whilst each alkyl moiety may contain from 1 to 3 carbon atoms, or
R₁ and R₂ together represent an alkylenedioxy group with 1 or 2 carbon atoms,
R₃ represents a hydrogen or halogen atom, an alkyl or alkoxy group with 1 to 3 carbon atoms in the alkyl moiety, or a hydroxy, nitro, cyano or trifluoromethyl group,
R₄ represents a hydrogen atom, an alkoxy, alkanesulphonyloxy, amino, alkylamino or dialkylamino group with 1 to 3 carbon atoms in each alkyl moiety or an alkanoylamino group with 2 or 3 carbon atoms in the alkanoyl moiety or
R₃ and R₄ together represent an alkylenedioxy group with 1 or 2 carbon atoms,
R₅ represents a hydrogen or halogen atom, a hydroxy, alkyl or alkoxy group with 1 to 3 carbon atoms in the alkyl moiety,
m represents the number 1, 2, 3, 4 or 5 and
n represents the number 0, 1 or 2, but n + m must represent the number 3, 4 or 5,
the enantiomers, diastereomers and acid addition salts thereof.

2. New cyclic amine derivatives of general formula I as claimed in claim 1, wherein
A, B, m and n are defined as hereinbefore but m + n must represent the number 3, 4 or 5,
E represents a methylene or ethylene group,
G represents an n-alkylene group with 1 to 4 carbon atoms, whilst a methylene group of an n-propylene or n-butylene group adjacent to a phenyl nucleus may be replaced by an oxygen or sulphur atom or an imino, methylimino, sulphinyl or sulphonyl group,
R₁ represents a hydrogen, fluorine, chlorine or bromine atom, or a hydroxy, methoxy, trifluoromethyl, methylamino or dimethylamino group,
R₂ represents a hydrogen, chlorine or bromine atom or a methoxy group or
Ri and R2 together represent a methylenedioxy group,
R₃ represents a hydrogen, fluorine, chlorine or bromine atom or a methyl, hydroxy, methoxy or nitro group,
R₄ represents a hydrogen atom or a methoxy, methanesulphonyloxy, amino or acetylamino group or
R₃ and R₄ together represent a methylenedioxy group and
R₅ represents a hydrogen, chlorine or bromine atom or a methoxy group,
and the enantiomers, diastereomers and acid addition salts thereof.

3. New cyclic amine derivatives of general formula I as claimed in claim 1, wherein
m and n are defined as in claim 1 but m + n must represent the number 3, 4 or 5,
A represents a -CH₂CH₂- or -CH=CH- group and B represents a methylene or carbonyl group or
A represents a -CO-CO- group and B represents a methylene group,
E represents a methylene or ethylene group,
G represents an n-alkylene group with 2 to 4 carbon atoms, whilst a methylene group of an n-propylene or n-butylene group adjacent to a phenyl nucleus may be replaced by an oxygen atom,
R₁ represents a hydrogen atom or a methoxy group,
R₂ represents a hydrogen atom or a methoxy group or
R₁ and R₂ together represent a methylenedioxy group,
R₃ represents a hydrogen atom or a methyl, hydroxy or methoxy group,
R₄ represents a hydrogen atom or a methoxy group or
R₃ and R₄ together represent a methylenedioxy group and
Rs represents a hydrogen atom,
and the enantiomers, diastereomers and acid addition salts thereof.

4. 3-[(N-(2-(3,4-Dimethoxy-phenyl)-ethyl)-piperidin-3-yl)-methyl]-7,8-dimethoxy-1,3,4,5-tetrahydro-2H-3-benzazepin-2-one, the enantiomers and the acid addition salts thereof.

5. 3-[(N-(2-(3,4-Dimethoxy-phenyl)-ethyl)-piperidin-2-yl)-ethyl-2]-7,8-dimethoxy-1,3,4,5-tetrahydro-2H-3-benzazepin-2-one, the enantiomers and the acid addition salts thereof.

6. Physiologically acceptable acid addition salts of the compounds as claimed in claims 1 to 5 with inorganic or organic acids.

7. Pharmaceutical compositions containing a compound of general formula I as claimed in claims 1 to 5 or a physiologically acceptable acid addition salt thereof as claimed in claim 6 together with one or more inert carriers and/or diluents.

8. Pharmaceutical compositions as claimed in claim 7 suitable for the treatment of sinus tachycardia and ischaemic heart disease.

9. Process for preparing a pharmaceutical composition as claimed in claims 7 and 8, characterised in that a compound as claimed in claims 1 to 5 or a physiologically acceptable acid addition salt thereof as claimed in claim 6 is incorporated in one or more inert carriers and/or diluents by a non-chemical method.

10. Process for preparing new cyclic amine derivatives as claimed in claims 1 to 6, characterised in that
a) a compound of general formula 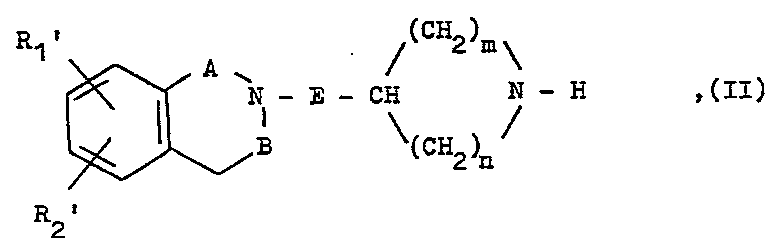 wherein
A, B, E, m and n are defined as in claims 1 to 5,
R_{1'} represents a hydroxy, amino or alkylamino group protected by a protecting group or has the meanings given for R₁ in claims 1 to 5 and
R_{2*} represents a hydroxy group protected by a protecting group or has the meanings given for R₂ in claims 1 to 5, is reacted with a compound of general formula 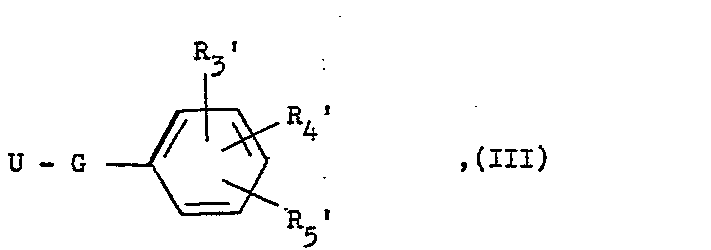 wherein
R_{3'}, R4 and R₅, have the meanings given for R₃, R₄ and R₅ in claims 1 to 5 but the hydroxy, amino or alkylamino groups contained in the groups R₃ to Rₛ may be protected by a protecting group, and
U represents a nucleophilic leaving group such as a halogen atom or a sulphonyloxy group, and optionally any protecting group used is subsequently split off, or
b) in order to prepare compounds of general formula I wherein G has the meanings given for Gin claims 1 to 5, with the exception of the groups containing a sulphenyl, sulphinyl or sulphonyl group, A represents a -CH₂-CH_{z}- group, B represents a methylene or carbonyl group and m + n represent the number 4, a compound of general formula 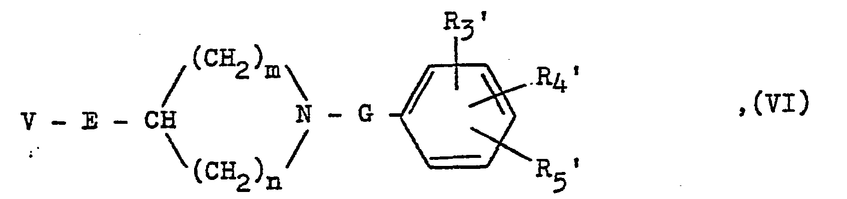 wherein
R₁ to R₅ and E are defined as in claims 1 to 5,
G' has the meanings given for Gin claims 1 to 5 with the exception of the radicals containing a sulphur atom or a sulphinyl or sulphonyl group,
A' represents a -CH=CH- or -CH₂CH₂- group and
B' represents a methylene or carbonyl group, is hydrogenated in a solvent or mixture of solvents, or
c) in order to prepare compounds of general formula I wherein B represents a carbonyl or methylene group, a compound of general formula 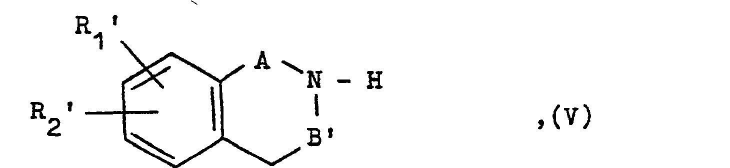 wherein
A is defined as in claims 1 to 5,
R₁' represents a hydroxy, amino or alkylamino group protected by a protecting group or has the meanings given for R₁ in claims 1 to 5,
R₂' represents a hydroxy group protected by a protecting group or has the meanings given for R₂ in claims 1 to 5, and
B' represents a carbonyl or methylene group, is reacted with a compound of general formula 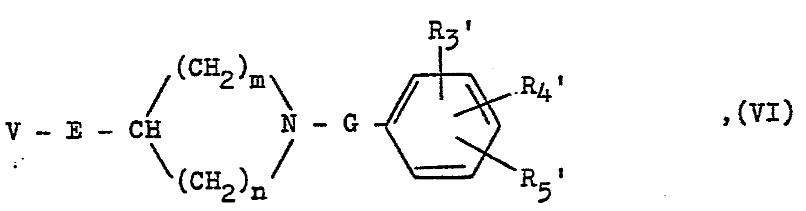 wherein
E, G, m and n are defined as in claims 1 to 5,
R_{3'}, R₄' and Rs' have the meanings given for Rs, R₄ and R₅ in claims 1 to 5, but the hydroxy, amino or alkylamino groups contained in the groups R₃ to R₅ may be protected by a protecting group, and
V represents a nucleophilically exchangeable group such as a halogen atom or a sulphonyloxy group, and optionally any protecting group used is subsequently split off, or
d) in order to prepare compounds of general formula I wherein A represents a -CH₂-CH₂- or - CH=CH- group and B represents a thiocarbonyl group, a compound of general formula 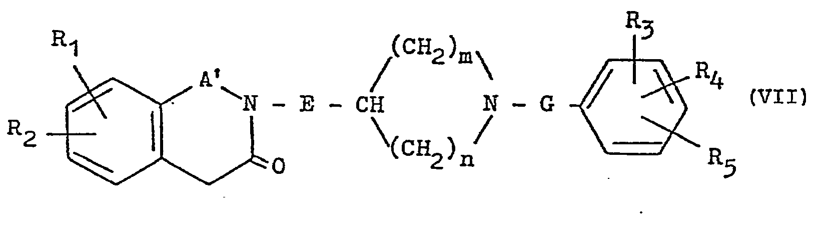
wherein
^{R}_{I} to R₅, E, G, m and n are defined as in claims
1 to 5 and A' represents a -CH₂-CH₂- or -CH=CH- group,
is reacted with a sulphurising agent, or
e) in order to prepare compounds of general formula OH
I wherein A represents a -CH-CO- group and B represents
a methylene group,
a compound of general formula 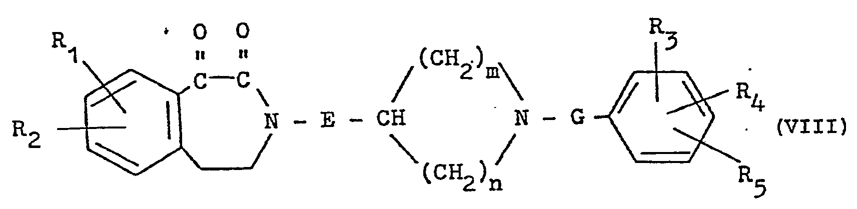 wherein
R₁ to R5, E, G, m and n are defined as in claims 1 to 5, is reduced in a suitable solvent, or
f) in order to prepare compounds of general formula I wherein A represents a -CH₂-CH₂- or - CH=CH- group and B represents a methylene group, a compound of general formula 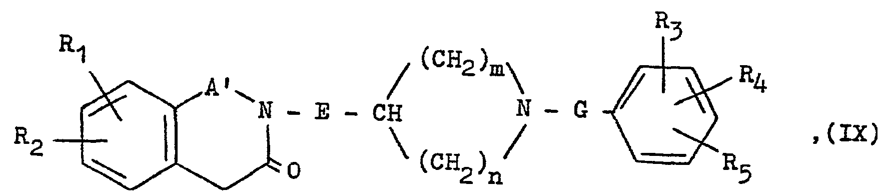 wherein
R₁ to Rₛ, E, G, m and n are defined as in claims 1 to 5 and A' represents a -CH₂-CH₂- or -CH=CH- group, is reduced in a suitable solvent, or
g) in order to prepare compounds of general formula I wherein A represents a -COCO- group, a compound of general formula 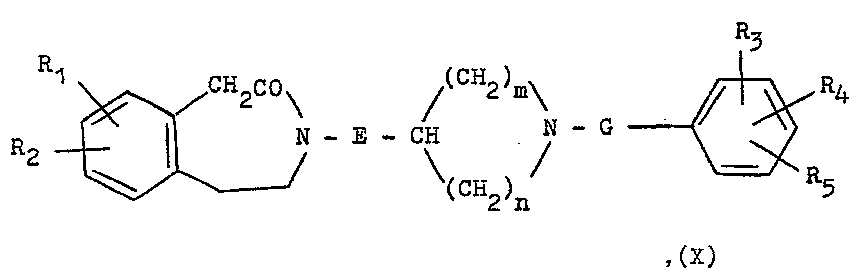 wherein
R₁ to Rs, E, G, m and n are defined as in claims 1 to 5, is oxidised in a suitable solvent or mixture of solvents, or
h) in order to prepare compounds of general formula I wherein G has the meanings given for G in claims 1 to 5 with the exception of the radicals containing a sulphur atom or a sulphinyl or sulphonyl group, A represents a -CH₂-CH₂- group and B represents a methylene or carbonyl group, a compound of general formula 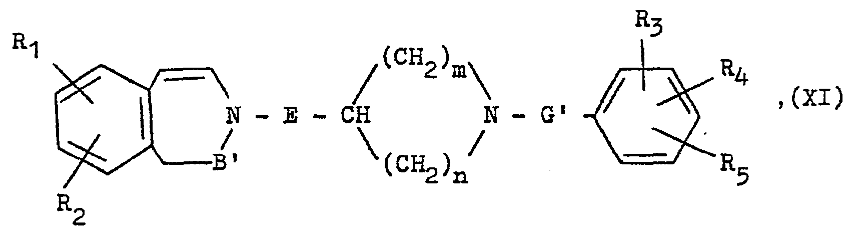 wherein
R₁ to Rs, E, m and n are defined as in claims 1 to 5,
G' has the meanings given for Gin claims 1 to 5, with the exception of the radicals containing a sulphur atom or a sulphinyl or sulphonyl group and
B' represents a methylene or carbonyl group, is hydrogenated in a solvent or mixture of solvents
and subsequently, if desired, a compound of general formula I thus obtained wherein R₁ and/or R₃ represents a nitro group is converted by reduction into a corresponding amino compound of general formula I, or
a compound of general formula I thus obtained wherein R₄ represents a hydroxy or amino group is converted by acylation into a corresponding alkanesulphonyloxy or alkanoylamino compound of general formula I, or
a compound of general formula I thus obtained which contains at least one chiral centre is resolved into its diastereomers or into its enantiomers and/or
a compound of general formula I thus obtained is converted into the acid addition salts thereof, particularly its physiologically acceptable acid addition salt with inorganic or organic acids.

11. Process as claimed in claim 10, characterised in that the reaction is carried out in a solvent.

12. Process as claimed in claims 10a or 10c, characterised in that the reaction is carried out in a solvent and in the presence of an acid-binding agent.

## Claims (Claims for the following Contracting State(s) : s: AT, ES, GR)

1.Process for preparing new cyclic amine derivatives of general formula 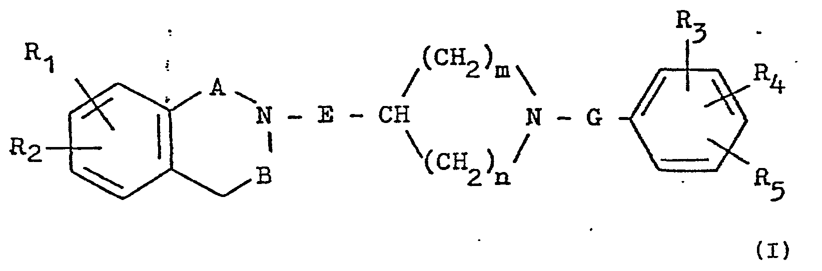
wherein
A represents a 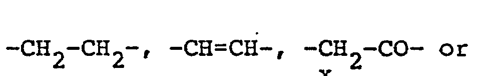 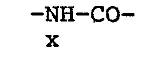
group and
B represents a methylene, carbonyl or thiocarbonyl
group or
A represents a 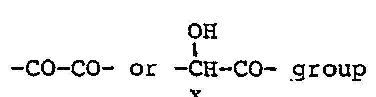 and B represents
a methylene group, whilst tfie atom marked x is
linked to the phenyl nucleus, a methylene group, whilst the atom marked x is linked to the phenyl nucleus,
E represents a straight-chained alkylene group with 1 to 3 carbon atoms optionally substituted by an alkyl group with 1 to 3 carbon atoms,
G represents a straight-chained alkylene group with 1 to 5 carbon atoms optionally substituted by an alkyl group with 1 to 3 carbon atoms, whilst a methylene group, adjacent to a phenyl nucleus, of a straight-chained alkylene group with 3 to 5 carbon atoms optionally substituted by an alkyl group may be replaced by an oxygen or sulphur atom or by an imino, methylimino, sulphinyl or sulphonyl group,
Rᵢ represents a hydrogen or halogen atom, a trifluoromethyl, nitro, amino, alkylamino, dialkylamino, alkyl, hydroxy, alkoxy or phenylalkoxy group, whilst each alkyl moiety may contain from 1 to 3 carbon atoms,
R₂ represents a hydrogen or halogen atom, a hydroxy, alkoxy, phenylalkoxy or alkyl group, whilst each alkyl moiety may contain from 1 to 3 carbon atoms, or
R₁ and R₂ together represent an alkylenedioxy group with 1 or 2 carbon atoms,
R₃ represents a hydrogen or halogen atom, an alkyl or alkoxy group with 1 to 3 carbon atoms in the alkyl moiety, or a hydroxy, nitro, cyano or trifluoromethyl group,
R₄ represents a hydrogen atom, an alkoxy, alkanesulphonyloxy, amino, alkylamino or dialkylamino group with 1 to 3 carbon atoms in each alkyl moiety or an alkanoylamino group with 2 or 3 carbon atoms in the alkanoyl moiety or
R₃ and R₄ together represent an alkylenedioxy group with 1 or 2 carbon atoms,
Rₛ represents a hydrogen or halogen atom, a hydroxy, alkyl or alkoxy group with 1 to 3 carbon atoms in the alkyl moiety,
m represents the number 1, 2, 3, 4 or 5 and
n represents the number 0, 1 or 2, but n + m must represent the number 3, 4 or 5,
the enantiomers, diastereomers and acid addition salts thereof, characterised in that
a) a compound of general formula 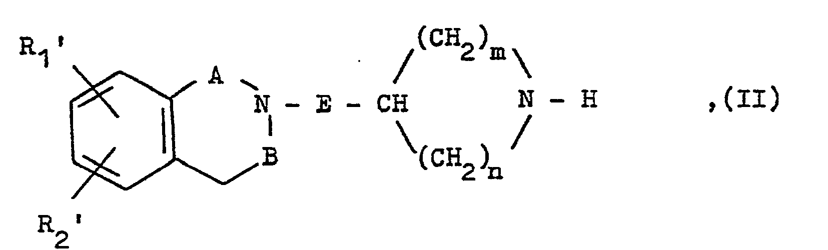 wherein
A, B, E, m and n are defined as hereinbefore,
R₁' represents a hydroxy, amino or alkylamino group protected by a protecting group or has the meanings given for R₁ hereinbefore and
R₂' represents a hydroxy group protected by a protecting group or has the meanings given for R₂ hereinbefore, is reacted with a compound of general formula 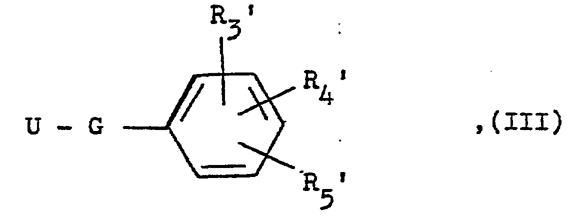 wherein
R₃', R_{4'} and R₅' have the meanings given for Rs, R₄ and Rs hereinbefore but the hydroxy, amino or alkylamino groups contained in the groups R₃ to R₅ may be protected by a protecting group, and
U represents a nucleophilic leaving group such as a halogen atom or a sulphonyloxy group, and optionally any protecting group used is subsequently split off, or
b) in order to prepare compounds of general formula I wherein G has the meanings given for Gin claims 1 to 5, with the exception of the groups containing a sulphenyl, sulphinyl or sulphonyl group, A represents a -CH₂-CH2- group, B represents a methylene or carbonyl group and m + n represent the number 4, a compound of general formula 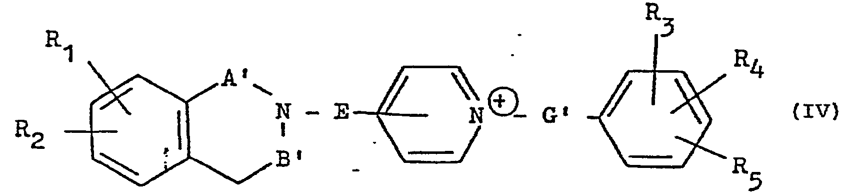 wherein
R₁ to R₅ and E are defined as hereinbefore,
G' has the meanings given for Gas hereinbefore with the exception of the radicals containing a sulphur atom or a sulphinyl or sulphonyl group,
A' represents a -CH=CH- or -CH₂CH₂- group and
B' represents methylene or carbonyl group, is hydrogenated in a solvent or mixture of solvents, or
c) in order to prepare compounds of general formula I wherein B represents a carbonyl or methylene group, a compound of general formula 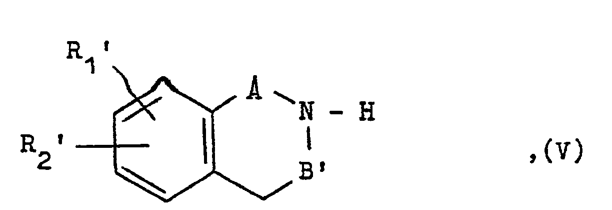 wherein
A is defined as hereinbefore,
R₁' represents a hydroxy, amino or alkylamino group protected by a protecting group or has the meanings given for Rᵢ hereinbefore,
R_{2'} represents a hydroxy group protected by a protecting group or has the meanings given for R₂ hereinbefore, and
B' represents a carbonyl or methylene group, is reacted with a compound of general formula 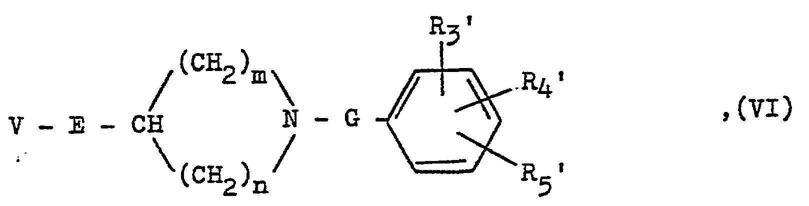 wherein
E, G, m and n are defined as hereinbefore,
R_{3'}, R_{4'} and Rs_{'} have the meanings given for R₃, R₄ and Rs hereinbefore, but the hydroxy, amino or alkylamino groups contained in the groups R₃ to R₅ may be protected by a protecting group, and
V represents a nucleophilically exchangeable group such as a halogen atom or a sulphonyloxy group, and optionally any protecting group used is subsequently split off, or
d) in order to prepare compounds of general formula I wherein A represents a -CH₂-CH₂- or - CH=CH- group and B represents a thiocarbonyl group, a compound of general formula 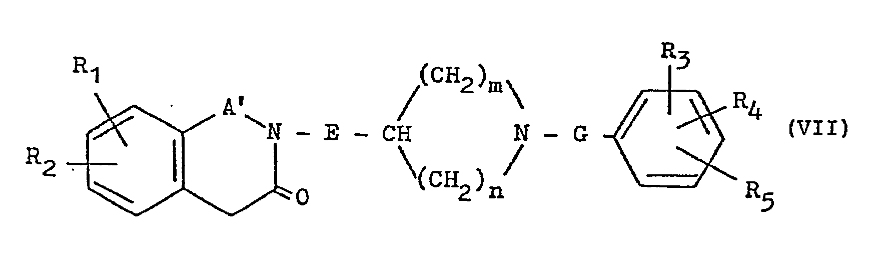 wherein
^{R}₁ to R₅, E, G, m and n are defined as hereinbefore and A' represents a -CH₂-CH₂- or -CH=CH- group,
is reacted with a sulphurising agent, or
e) in order to prepare compounds of general formula OH
I wherein A represents a -CH-CO- group and B represents
a methylene group,
a compound of general formula 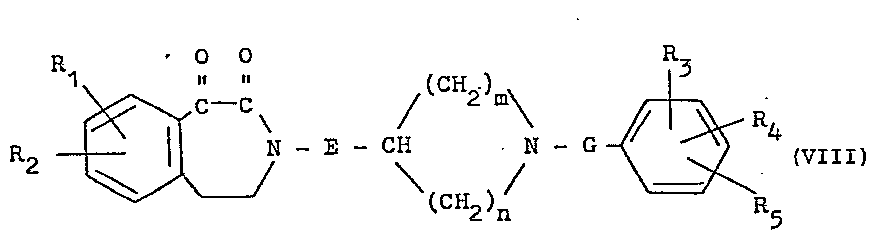 wherein
R₁ to R₅, E, G, m and n are defined as hereinbefore is reduced in a suitable solvent, or
f) in order to prepare compounds of general formula I wherein A represents a -CH₂-CH₂- or - CH=CH- group and B represents a methylene group,
a compound of general formula 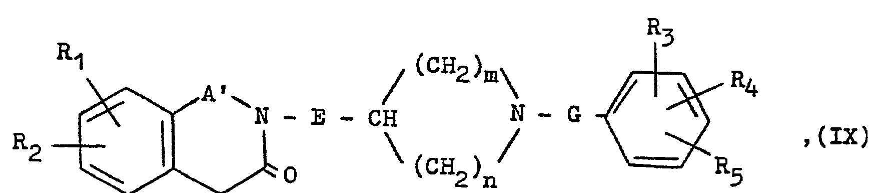 wherein
R₁ to Rs, E, G, m and n are defined as hereinbefore and A, represents a -CH₂-CH₂- or -CH=CH- group, is reduced in a suitable solvent, or
g) in order to prepare compounds of general formula I wherein A represents a -COCO- group, a compound of general formula 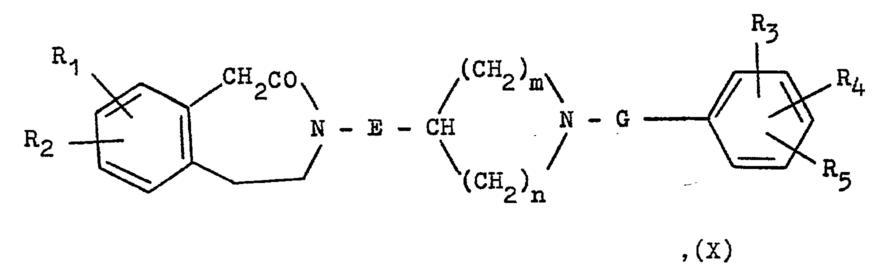 wherein
R₁ to Rₛ, E, G, m and n are defined as hereinbefore is oxidised in a suitable solvent or mixture of solvents, or
h) in order to prepare compounds of general formula I wherein G has the meanings given for G hereinbefore with the exception of the radicals containing a sulphur atom or a sulphinyl or sulphonyl group, A represents a -CH₂-CH₂- group and B represents a methylene or carbonyl group,
a compound of general formula 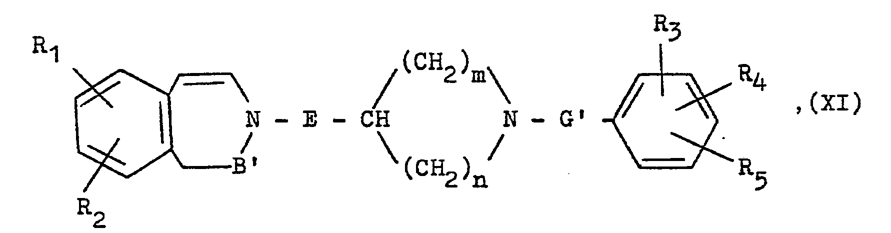 wherein
R₁ to R₅, E, m and n are defined as hereinbefore,
G_{'} has the meanings given for G hereinbefore, with the exception of the radicals containing a sulphur atom or a sulphinyl or sulphonyl group and
B' represents a methylene or carbonyl group, is hydrogenated in a solvent or mixture of solvents
and subsequently, if desired, a compound of general formula thus obtained wherein R₁ and/or R₃ represents a nitro group is converted by reduction into a corresponding amino compound of general formula I, or
a compound of general formula I thus obtained wherein R₄ represents a hydroxy or amino group is converted by acylation into a corresponding alkanesulphonyloxy or alkanoylamino compound of general formula I, or
a compound of general formula I thus obtained which contains at least one chiral centre is resolved into its diastereomers or into its enantiomers and/or
a compound of general formula I thus obtained is converted into the acid addition salts thereof, particularly its physiologically acceptable acid addition salts with inorganic or organic acids.

2. Process according to claim 1 for preparing new cyclic amine derivative of general formula 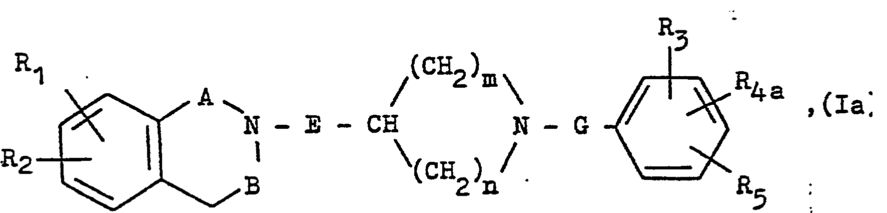 wherein
R₁ to R₃, Rs, A, B, E, G, m and n are defined as in claim 1 and
R₄ₐ represents a hydrogen atom, an alkoxy, amino, alkylamino or dialkylamino group each having 1 to 3 carbon atoms in each alkyl moiety, or an alkanoylamino group having 2 or 3 carbon atoms in the alkanoyl moiety or
R₃ and R₄ₐ together represent an alkylenedioxy group having 1 or 2 carbon atoms, the enantiomers, diastereomers and acid addition salts thereof, characterised in that
a) a compound of general formula 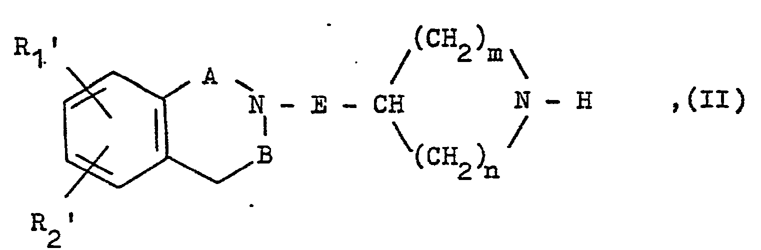 wherein R₁', R_{2'}, A, B, E, m and n are defined as in claim 1, is reacted with a compound of general formula 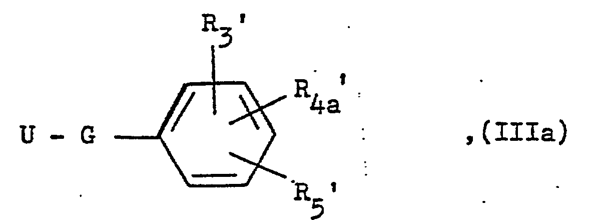 wherein
R_{3'}, Rs_{'}, G and U are defined as in claim 1 and
R₄a_{'} has the meanings given for R₄ₐ hereinbefore, except that the hydroxy, amino or alkylamino groups contained in the groups Rₛ, R₄ₐ and R₃ may be protected by a protecting group, and subsequently, if desired, a protecting group used is split off or
b) in order to prepare compounds of general formula la wherein G has the meanings given for G hereinbefore, with the exception of the radicals containing a sulphenyl, sulphinyl or sulphonyl group, A represents a -CH₂-CH₂ group, B represents a methylene or carbonyl group and m + n represent the number 4, d compound of general formula 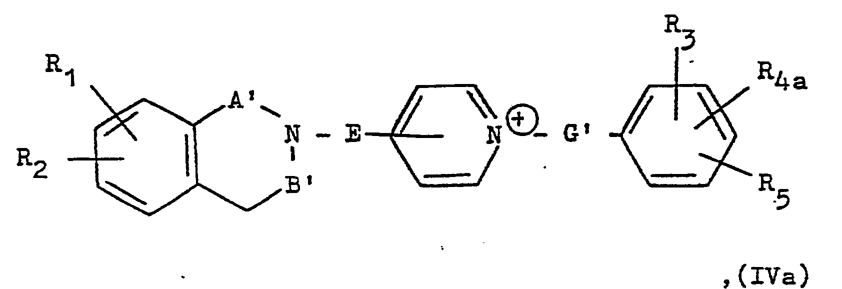 wherein R₁ to Rs, Rs, A', B', E and G_{'} are defined as in claim 1 and
R₄ₐ is defined as hereinbefore, is hydrogenated in a solvent or mixture of solvents or
c) in order to prepare compounds of general formula la wherein B represents a carbonyl or methylene group, a compound of general formula 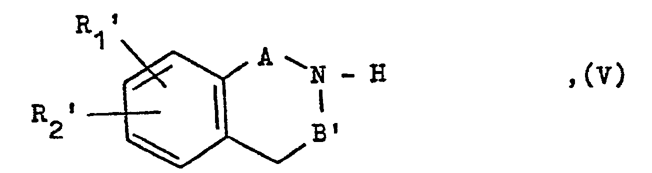 wherein R₁', R₂', A and B, are defined as in claim 1, is reacted with a compound of general formula 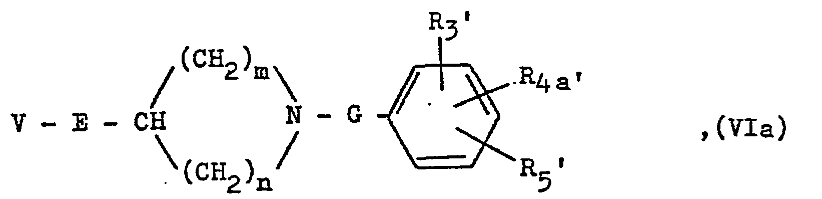 wherein R_{3'}, Rs_{'}, E, G, V, m and n are defined as in claim 1 and
R₄' has the meanings given for R₄ hereinbefore, except that the hydroxy, amino or alkylamino groups contained in the radicals R₃, R₄ₐ and Rs may be protected by a protecting group and any protecting group used is subsequently split off or
d) in order to prepare compounds of general formula la wherein A represents a -CH₂-CH₂ or - CH=CH group and B represents a thiocarbonyl group, a compound of general formula 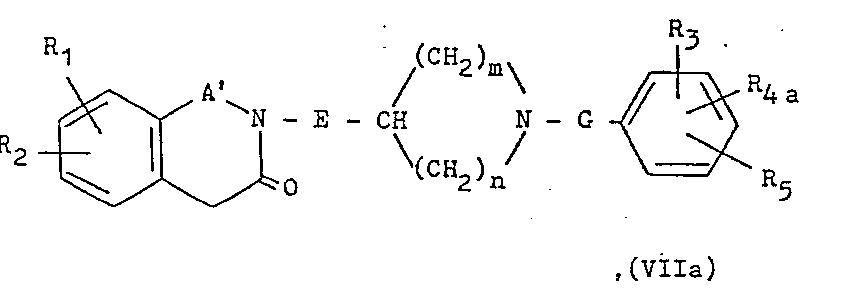
wherein
R₁ to R₃, Rₛ, A' , E, G, m and n are defined as in claim 1 and
R₄ₐ is defined as hereinbefore,
is reacted with a sulphurising agent or
e) in order to prepare compounds of general formula Ia wherein
A represents a 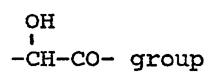 and B represents a methylene group,
a compound of general formula 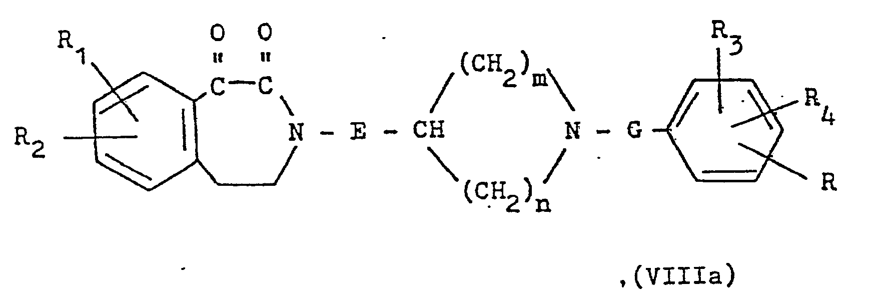 wherein
R₁ to R₃, R₅, E, G, m and n are defined as in claim 1 and
R₄ₐ is defined as hereinbefore, is reduced in a suitable solvent or
f) in order to prepare compounds of general formula la wherein A represents a -CH₂-CH₂ or - CH=CH group and B
represents a methylene group,
a compound of general formula 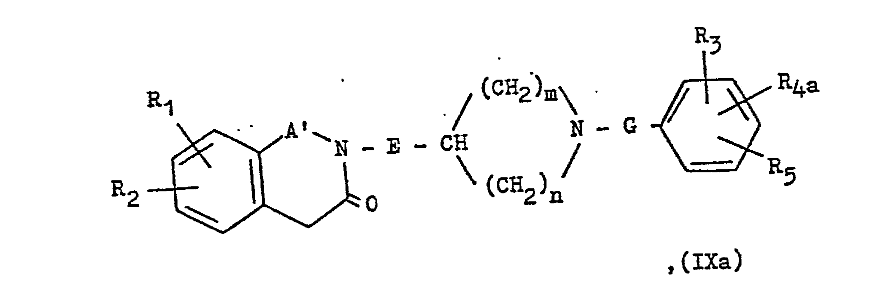 wherein R₁ to R₃, Rs, A', E, G, m and n are defined as in claim 1 and
R₄ₐ is defined as hereinbefore, is reduced in a suitable solvent or
g) in order to prepare compounds of general formula la wherein A represents a -COCO group, a compound of general formula 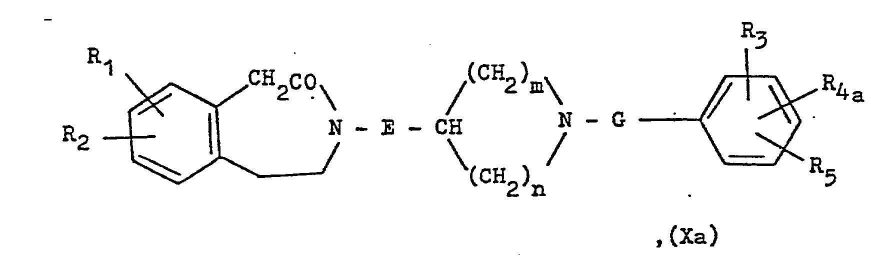 wherein R₁ to R₃, Rs, E, G, m and n are defined as in claim 1 and
R₄ₐ is defined as hereinbefore, is oxidised in a suitable solvent or mixture of solvents or
h) in order to prepare compounds of general formula la wherein G has the meanings given for G hereinbefore, with the exception of the radicals containing a sulphur atom or a sulphinyl or sulphonyl group, A represents a
-CH₂-CH2 group and B represents a methylene or carbonyl group, a compound of general formula 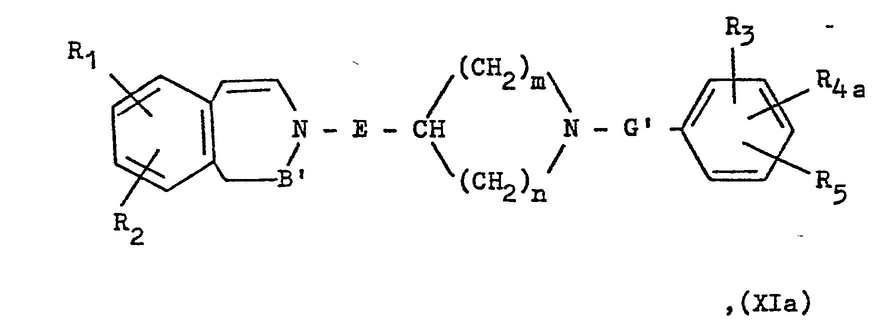 wherein
R₁ to R₃, Rs, B', E, G', m and n are defined as in claim 1 and
R4a is defined as hereinbefore, is hydrogenated in a solvent or mixture of solvents
and subsequently, if desired, a compound of general formula la thus obtained wherein R₁ and/or R₃ represents a nitro group, is converted by reduction into a corresponding amino compound of general formula la or
a compound of general formula la thus obtained wherein R₄ₐ represents an amino group, is converted by alkanoylation into a corresponding alkanoylamino compound of general formula la or
a compound of general formula la thus obtained which contains at least one chiral centre, is resolved into the diastereomers or enantiomers thereof and/or
a compound of general formula la thus obtained is converted into the acid addition salts thereof, more particularly the physiologically acceptable acid addition salts with organic or inorganic acids.

3. Process according to claims 1 and 2, characterised in that the reaction is carried out in a solvent.

4. Process according to claims le, 2e and 3, characterised in that the reaction is carried out with a metal hydride such as sodium borohydride at temperatures of between 0 and 80_{°}C, preferably at temperatures of between 15 and 40°C.

5. Process according to claims 1f, 2f and 3, characterised in that the reaction is carried out with a metal hydride such as lithium aluminium hydride or diboran or with a complex of boran and a thioether, e.g. with a complex of diboran and dimethylsulphide, at temperatures of between 0 and 25_{°}C, but preferably at temperatures of between 10 and 25°C.

6. Process according to claims 1g, 2g and 3, characterised in that the oxidation is carried out with potassium permanganate, selenium dioxide or sodium dichromate at temperatures of between 0 and 100_{°}C.

7. Process for preparing a pharmaceutical composition, characterised in that, by a non-chemical method, a compound of general formula 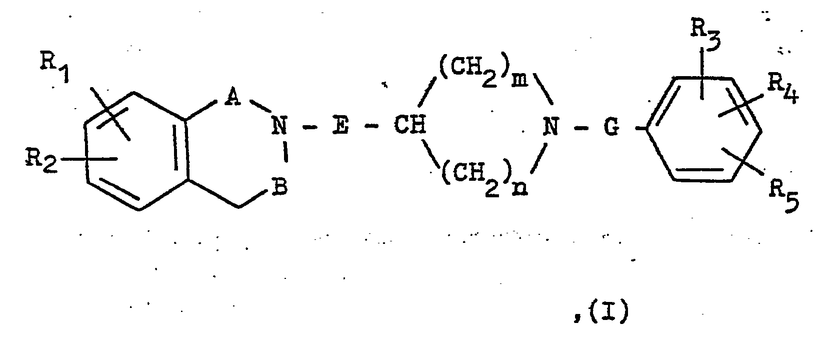 wherein, (I)
Ri to Rs, A, B, E, G, m and n are defined as in claim 1, or a physiologically acceptable acid addition salt thereof is incorporated in one or more inert carriers and/or diluents.

## Revendications (Revendications pour l'(les) Etat(s) contractant(s) suivant(s) : BE, CH, DE, FR, GB, IT, LI, LU, NL, SE pour les états contractants: BE, CH, DE, FR, GB, IT, LI, LU, NL, SE)

1. Nouveaux dérivés amines cycliques de formule générale 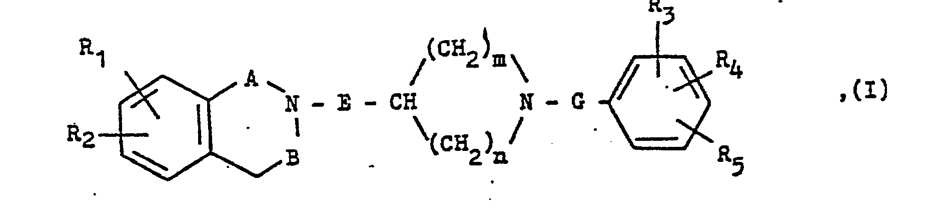
dans laquelle
A représente un groupe 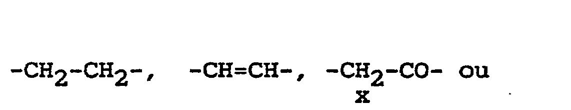 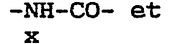 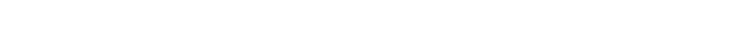
B représente un groupe méthylène, carbonyle ou thiocarbonyle ou
A représente un groupe 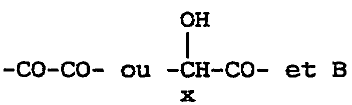 un groupe
méthylène, l'atome marqué de x étant dans chaque cas relié au noyau phényle,
E représente un groupe alcoylène avec 1 à 3 atomes de carbone, rectiligne, éventuellement substitué par un groupe alcoyl avec 1 à 3 atomes de carbone,
G représente un groupe alcoylène avec 1 à 5 atomes de carbone, rectiligne, éventuellement substitué par un groupe alcoyle avec 1 à 3 atomes de carbone, le groupe méthylène voisin du noyau phényle, d'un groupe alcoylène avec 3 à 5 atomes de carbon, éventuellement substitué par un groupe alcoyle pouvant être remplacé par un atome d'oxygène ou de soufre, un groupe imino, méthylimino, sulfinyle ou sulfonyle, R₁ représente un atome d'hydrogène ou d'halogène, un groupe trifluorométhyle, nitro, amino, alcoylamino, dialcoylamino, alcoyle, hydroxy, alcoxy ou phénylalcoxy, chaque partie alcoyle pouvant chacune contenir 1 à 3 atomes de carbone,
R₂ représente un atome d'hydrogène ou d'halogène, un groupe hydroxy, alcoxy, phénylalcoây ou alcoyle, chaque partie alcoyle pouvant contenir dans chaque cas 1 à 3 atomes de carbone, ou
R₁ et R₂ représentent ensemble un groupe alcoylènedioxy avec 1 ou 2 atomes de carbone,
R₃ représente un atome d'hydrogène ou d'halogène, un groupe alcoyle ou alcoxy avec chacun 1 à 3 atomes de carbone dans la partie alcoyle, un groupe hydroxy, nitro, cyano ou trifluorométhyle,
R₄ représente un atome d'hydrogène, un groupe alcoxy, alcanesulfonyloxy, amino, alcoylamino ou dialcoylamino avec chacun 1 à 3 atomes de carbone dans chaque partie alcoyle, ou un groupe alcanoylamino avec 2 ou 3 atomes de carbone dans la partie alcanoyle ou
R₃ et R₄ représentent ensemble un groupe alcoylènedioxy avec 1 ou 2 atomes de carbone,
R₅ représente un atome d'hydrogène ou d'halogène, un groupe hydroxy, alcoyle ou alcoxy avec chacun 1 à 3 atomes de carbone dans la partie alcoyle,
m représente le nombre 1, 2, 3, 4 ou 5 et
n représente le nombre 0, 1 ou 2, n + m devant toutefois représenter le nombre 3, 4 ou 5, leurs énantiomères, leurs diastéréoisomères et leurs sels d'addition d'acides.

2. Nouveaux dérivés amines cycliques de formule générale 1 selon la revendication 1, dans laquelle A, B, met n sont définis comme dans la revendication 1, m + n devant toutefois représenter le nombre 3, 4 ou 5,
E représente un groupe méthylène ou éthylène,
G représente un groupe n-alcoylène avec 1 à 4 atomes de carbone, le groupe méthylène voisin du noyau phényle d'un groupe n-propylène ou n-butylène pouvant être remplacé par un atome d'oxygène ou de soufre, un groupe imino, méthylimino, sulfinyle ou sulfonyle,
Rᵢ représente un atome d'hydrogène, de fluor, de chlore ou de brome, un groupe hydroxy, méthoxy, trifluorométhyle, méthylamino ou diméthylamino,
R₂ représente un atome d'hydrogène, de chlore ou de brome, ou un groupe méthoxy ou
R₁ et R₂ représentent ensemble un groupe méthylènedioxy,
Rₛ représente un atome d'hydrogène, de fluor, de chlore ou de brome, un groupe méthyle, hydroxy, méthoxy ou nitro,
R₄ représente un atome d'hydrogène, un groupe méthoxy, méthanesulfonyloxy, amino ou acétylamino ou R₃ et R₄ représentent ensemble un groupe méthylènedioxy et
Rₛ représente un atome d'hydrogène, de chlore ou de brome ou un groupe méthoxy, leurs énantiomères, leurs diastéréoisomères et leurs sels d'addition d'acides.

3. Nouveaux dérivés amines cycliques de formule générale I selon la revendication 1, dans laquelle
m et n sont définis comme dans la revendication 1, m + n devant toutefois représenter le nombre 3, 4 ou 5, A représente un groupe -CH₂-CH₂- ou -CH=CH- et B un groupe méthylène ou carbonyle, ou
A représente un groupe -CO-CO- et B un groupe méthylène,
E représente un groupe méthylène ou éthylène,
G représente un groupe n-alcoylène avec 2 à 4 atomes de carbone, le groupe méthylène voisin du noyau phényle d'un groupe n-propylène ou n-butylène pouvant être remplacé par un atome d'oxygène
R₁ représente un atome d'hydrogène ou un groupe méthoxy,
R₂ représente un atome d'hydrogène ou un groupe méthoxy ou
R₁ et R₂ représentent ensemble un groupe méthylènedioxy,
R₃ représente un atome d'hydrogène, un groupe méthyle, hydroky ou méthoxy,
R₄ représente un atome d'hydrogène ou un groupe méthoxy ou
R₃ et R₄ représentent ensemble un groupe méthylènedioxy et
Rₛ représente un atome d'hydrogène, leurs énantiomères, leurs diastéréoisomères et leurs sels d'addition d'acides.

4. La 3-[(N-(2-(3,4-diméthoxy-phényl)-éthyl)-pipéridine-3-yl)-méthyl]-7,8-diméthoxy-1,3,4,5-tétra- hydro-2H-3-benzazépine-2-one, ses énantiomères et ses sels d'addition d'acides.

5. La 3-[(N-(2-(3,4-diméthoxy-phényl)-éthyl)-pipéridine-2-yl)-éthyl-2]-7,8-diméthoxy-1,3,4,5-tétra- hydro-2H-3-benzazépine-2-one, ses énantiomères et ses sels d'addition d'acides.

6. Sels d'addition d'acides physiologiquement supportables des composés selon les revendications 1 à 5 avec des acides non organiques ou organiques.

7. Médicament contenant un composé de formule générale 1 selon les revendications 1 à 5 ou son sel d'addition d'acide physiologiquement supportable selon la revendication 6, conjointement à un ou plusieurs excipients et/ou diluants inertes.

8. Médicament selon la revendication 7 convenant au traitement des tachycardies sinusales et des maladies cardiaques ischémiques.

9. Procédé pour la fabrication d'un médicament selon les revendications 7 et 8, caractérisé en ce que l'on introduit, par voie non chimique, un composé selon les revendications 1 à 5 ou son sel d'addition d'acide physiologiquement supportable selon la revendication 6 dans un ou plusieurs excipients et/ou diluants inertes.

10. Procédé pour la fabrication des nouveaux dérivés amines cycliques selon les revendications 1 à 6, caractérisé en ce que
a) on fait réagir un composé de formule générale 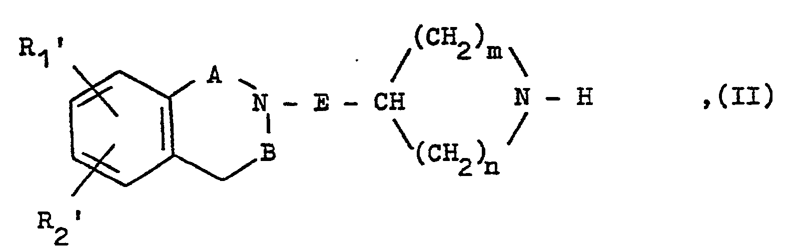 dans laquelle A, B, E, m et n sont définis comme dans les revendications 1 à 5,
R₁' représente un groupe hydroxy, amino ou alcoylamino, protégé par un radical protecteur ou possède les significations mentionnées pour R1 dans les revendications 1 à 5, et
R₂' représente un groupe hydroxy protégé par un groupe protecteur ou possède les significations mentionnées pour R₂ dans les revendications 1 à 5, avec un composé de formule générale 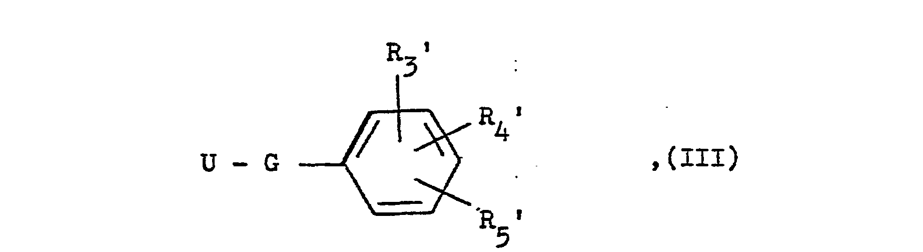 dans laquelle
Rs_{'}, R_{4'} et Rs_{'} possèdent les significations mentionnées pour R₃, R₄ et Rₛ dans les revendications 1 à 5, les groupes hydroxy, amino ou alcoylamino contenus dans les radicaux R₃ à Rₛ pouvant toutefois être protégés par un groupe protecteur, et
U représente un groupe partant nucléophile tel qu'un atome d'halogène ou un groupe sulfonyloxy, et en ce qu'éventuellement ensuite un groupe protecteur utilisé est clivé, ou
b) pour la fabrication des composés de formule générale I dans laquelle G possède les significations mentionnées pour G dans les revendications 1 à 5 à l'exception des radicaux contenant un groupe sulfényle, sulfinyle ou sulfonyle, A représente le groupe -CH₂-CH2-, B représente le groupe méthylène ou carbonyle et m + n représentent le nombre 4, on hydrogène un composé de formule générale 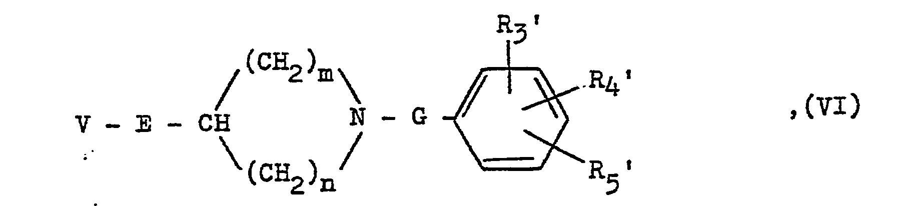 dans laquelle Ri à R₅ et E sont définis comme dans les revendications 1 à 5,
G' possède les significations mentionnées pour G dans les revendications 1 à 5, à l'exception des radicaux contenant un atome de soufre, un groupe sulfinyle ou sulfonyle,
A' représente le groupe -CH=CH- ou -CH₂-CH₂- et B' représente le groupe méthylène ou carbonyle, dans un solvant ou mélange de solvants ou
c) pour la fabrication des composés de formule générale I dans laquelle B représente le groupe carbonyle ou méthylène, on fait réagir un composé de formule générale 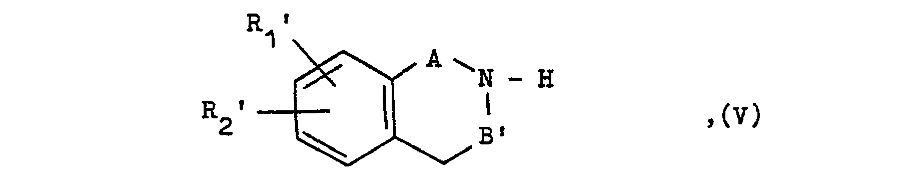 dans laquelle
A est défini comme dans les revendications 1 à 5,
R₁' représente un groupe hydroxy, amino ou alcoylamino protégé par un radical protecteur ou possède les significations mentionnées pour R₁ dans les revendications 1 à 5,
R₂' représente un groupe hydroxy protégé par un radical protecteur ou possède les significations mentionnées pour R₂ dans les revendications 1 à 5, et
B' représente un groupe carbonyle ou méthylène, avec un composé de formule générale 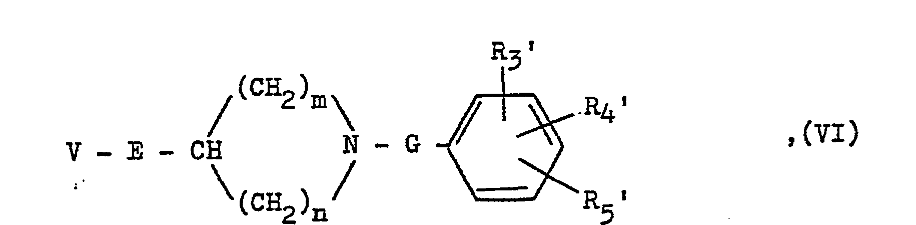 dans laquelle
E, G, m et n sont définis comme dans les revendications 1 à 5,
R_{3'}, R₄' et Rs_{'} possèdent les significations mentionnées pour R₃, R₄ et R₅ dans les revendications 1 à 5, les groupes hydroxy, amino ou alcoylamino contenus dans les radicaux Rs à Rs pouvant toutefois être protégés par un radical protecteur, et V représente un groupe partant nucléophile tel qu'un atome d'halogène ou un groupe sulfonyloxy, et éventuellement, on clive ensuite un radical protecteur utilisé ou
d) pour la fabrication des composés de formule générale I dans laquelle A représente un groupe - CH₂-CH₂- ou -CH=CH- et B un groupe thiocarbonyle, on fait réagir un composé de formule générale 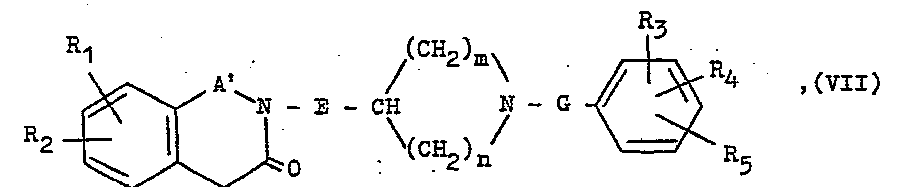 dans laquelle
R₁ à R5, E, G, m et n sont définis comme dans les revendications 1 à 5 et
A' représente un groupe -CH₂-CH₂- ou -CH=CH-, avec un agent introduisant du soufre ou
e) pour la fabrication des composés de formule générale
I dans laquelle A représente un groupe 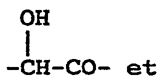 B représente un groupe méthylène, on réduit un composé de
formule générale 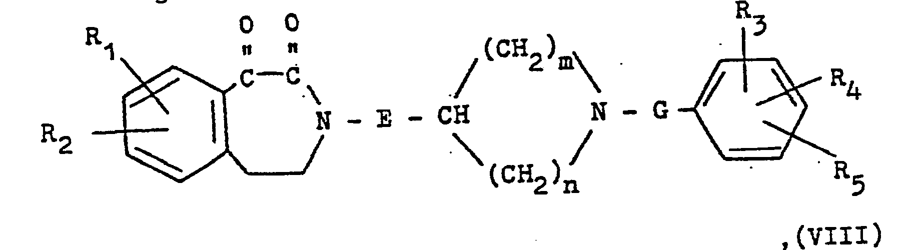 dans laquelle
R₁ à Rs, E, G m et n sont définis comme dans les revendications 1 à 5, dans un solvant approprié ou
f) pour la fabrication des composés de formule générale 1 dans laquelle A représente un groupe - CH₂-CH₂- ou -CH=CH- et B représente un groupe méthylène, on réduit un composé de formule 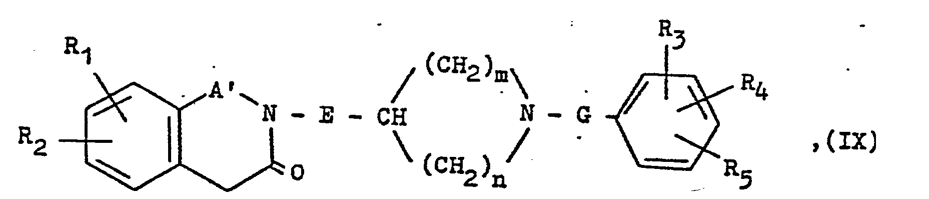 dans laquelle
R₁ à R₅, E, G, m et n sont définis comme dans les revendications 1 à 5, et
A' représente un groupe -CH₂-CH₂- ou -CH=CH-, dans un solvant approprié ou
g) pour la fabrication des composés de formule I dans laquelle A représente le groupe -COCO-, on oxyde un composé de formule générale 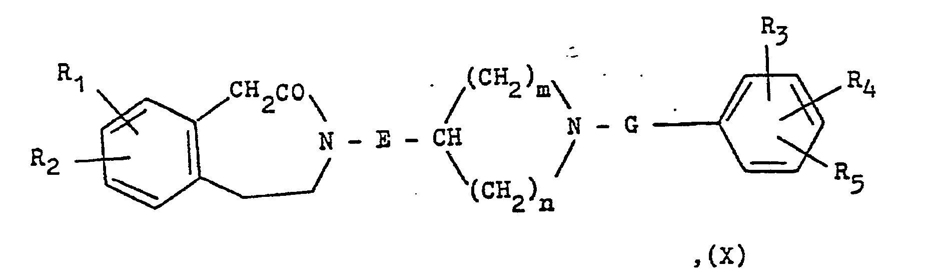 dans laquelle
R₁ à R₅, E, G, m et n sont définis comme dans les revendications 1 à 5, dans un solvant ou mélange de solvants approprié ou
h) pour la fabrication des composés de formule générale I dans laquelle G possède les significations mentionnées dans les revendications 1 à 5 pour G, à l'exception des radicaux contenant un atome de soufre, un groupe sulfinyle ou sulfonyle, A représente le groupe -CH₂-CH₂- et B le groupe méthylène ou carbonyle, on hydrogène un composé de formule générale 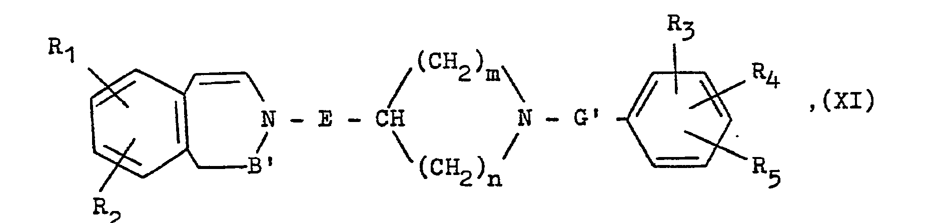 dans laquelle
R₁ à R₅, E, m et n sont définis comme dans les revendications 1 à 5,
G' possède les significations mentionnées pour G dans les revendications 1 à 5, à l'exception des radicaux contenant un atome de soufre, un groupe sulfinyle ou sulfonyle, et B' représente le groupe méthylène, ou carbonyle, dans un solvant ou mélange de solvants,
et ensuite, si on le désire, on transforme un composé ainsi obtenu de formule générale I, dans laquelle R₁ et/ou R₃ représente un groupe nitro, au moyen d'une réduction, en un composé amino correspondant de formule générale 1 ou
on transforme un composé ainsi obtenu de formule générale t, dans laquelle R₄ représente un groupe hydroxy ou amino, au moyen d'une acylation en un composé alcanesulfonyloxy ou alcanoylamino correspondant de formule générale I ou
on sépare un composé ainsi obtenu de formule générale I qui contient au moins un centre chiral, en ses diastéréoisomères ou en ses énantiomères et/ou
on transforme un composé ainsi obtenu de formule générale I en ses sels d'addition d'acides, en particulier en ses sels d'addition d'acides physiologiquement supportables avec des acides non organiques ou organiques.

11. Procédé selon la revendication 10, caractérisé en ce que la réaction est effectuée dans un solvant.

12. Procédé selon les revendications 10a ou 10c, caractérisé en ce que la réaction est effectuée dans un solvant et en présence d'un agent fixant les acides.

## Revendications (Revendications pour l'(les) Etat(s) contractant(s) suivant(s) : AT, ES, GR)

1. Procédé pour la fabrication de nouveaux dérivés amines cycliques de formule générale 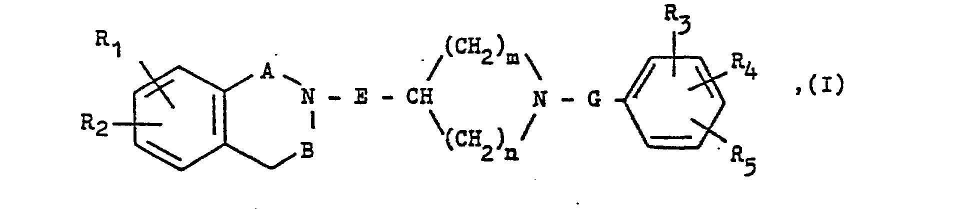
dans laquelle
A représente un groupe 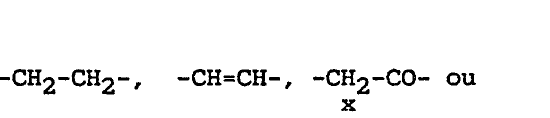 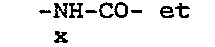
B représente un groupe méthylène, carbonyle ou thiocarbonyle ou
A représente un groupe 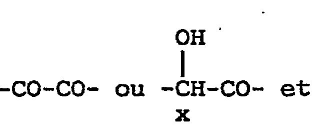 B un groupe méthylène, l'atome marqué par x étant dans chaque cas relié au noyau phényle,
E représente un groupe alcoylène rectiligne avec 1 à 3 atome de carbone, éventuellement substitué par un groupe alcoyle avec 1 à 3 atomes de carbone,
G représente un groupe alcoylène rectiligne, avec 1 à 5 atomes de carbone, éventuellement substitué par un groupe alcoyle avec 1 à 3 atomes de carbone, le groupe méthylène voisin du noyau phényle d'un groupe alcoylène rectiligne avec 3 à 5 atomes de carbone, éventuellement substitué par un groupe alcoyle pouvant être remplacé par un atome d'oxygène ou de soufre, un groupe imino, méthylimino, sulfinyle ou sulfonyle,
Rᵢ représente un atome d'hydrogène ou d'halogène, un groupe trifluorométhyle, nitro, amino, alcoylamino, dialcoylamino, alcoyle, hydroxy, alcoxy ou phénylalcoxy, chaque partie alcoyle pouvant contenir chacune 1 à 3 atomes de carbone,
R₂ représente un atome d'hydrogène ou d'halogène, un groupe hydroxy, alcoxy, phénylalcoxy ou alcoyle, chaque partie alcoyle pouvant contenir à chaque fois 1 à 3 atomes de carbone, ou
R₁ et R₂ représentent ensemble un groupe alcoylènedioxy avec 1 ou 2 atomes de carbone,
R₃ représente un atome d'hydrogène ou d'halogène, un groupe alcoyle ou alcoxy avec chacun 1 à 3 atomes. de carbone dans la partie alcoyle, un groupe hydroxy, nitro, cyano ou trifluorométhyle,
R₄ représente un atome d'hydrogène, un groupe alcoxy, alcanesulfonyloxy, amino, alcoylamino ou dialcoylamino avec chacun 1 à 3 atomes de carbone dans chaque partie alcoyle, ou un groupe alcanoylamino avec 2 ou 3 atomes de carbone dans la partie alcanoyle ou
R₃ et R₄ représentent ensemble un groupe alcoylènedioxy avec 1 ou 2 atomes de carbone,
R₅ représente un atome d'hydrogène ou d'halogène, un groupe hydroxy, alcoyle ou alcoxy avec chacun 1 à 3 atomes de carbone dans la partie alcoyle,
m représente le nombre 1, 2, 3, 4 ou 5 et
n représente le nombre 0, 1 ou 2, n + m devant toutefois représenter le nombre 3, 4 ou 5, de leurs énantiomères,
de leurs diastéréoisomères et de leurs sels d'addition d'acides, caractérisé en ce que
a) on fait réagir un composé de formule générale 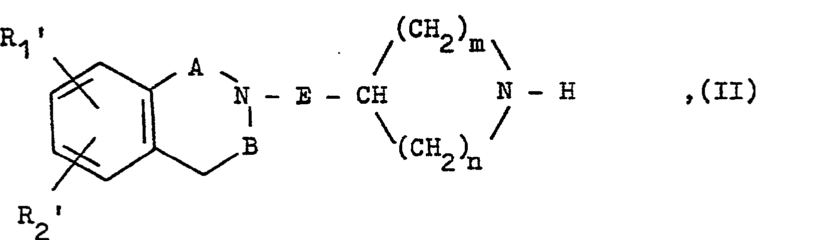 dans laquelle A, B, E, m et n sont définis comme au début,
R₁', représente un groupe hydroxy, amino ou alcoylamino, protégé par un radical protecteur ou possède les significations mentionnées au début pour Ri, et
R_{2'} représente un groupe hydroxy protégé par un radical protecteur ou possède les significations mentionnées au début pour R₂, avec un composé de formule générale 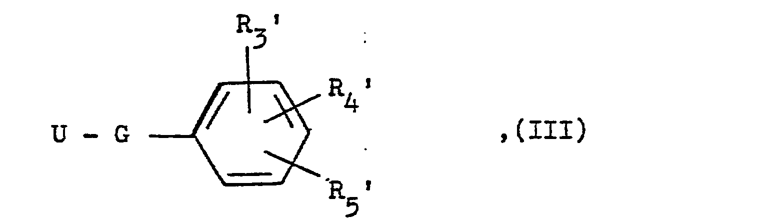 dans laquelle
Rs_{'}, R_{4'} et Rs_{'} possèdent les significations mentionnées au début pour Rs, R₄ et Rs, les groupes hydroxy, amino ou alcoylamino contenus dans les radicaux R₃ à R₅ pouvant toutefois être protégés par un radical protecteur, et U représente un groupe partant nucléophile tel qu'un atome d'halogène ou un groupe sulfonyloxy, et éventuellement ensuite on clive un groupe protecteur utilisé, ou
b) pour la fabrication des composés de formule générale I dans laquelle G est définis comme au début, à l'exception des radicaux contenant un groupe sulfényle, sulfinyle ou sulfonyle, A représente le groupe -CH₂-CH₂-, B représente le groupe méthylène ou carbonyle et m + n représentent le nombre 4, on hydrogène un composé de formule générale 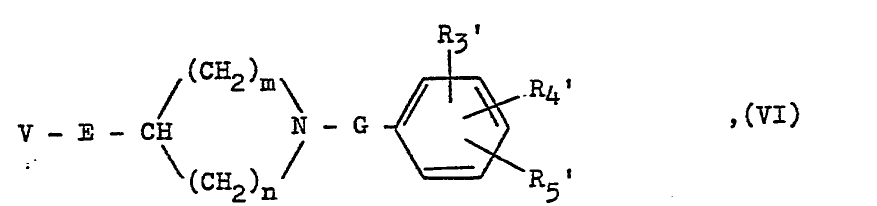 dans laquelle R₁ à R₅ et E sont définis comme au début,
G' possède les significations mentionnées dans les revendications 1-5 pour G, à l'exception des radicaux contenant un atome de soufre, un groupe sulfinyle ou sulfonyle,
A' représente le groupe -CH=CH- ou -CH₂-CH₂- et
B' représente le groupe méthylène ou carbonyle, dans un solvant ou mélange de solvants ou
c) pour la fabrication des composés de formule générale 1 dans laquelle B représente le groupe carbonyle ou méthylène, on fait réagir un composé de formule générale 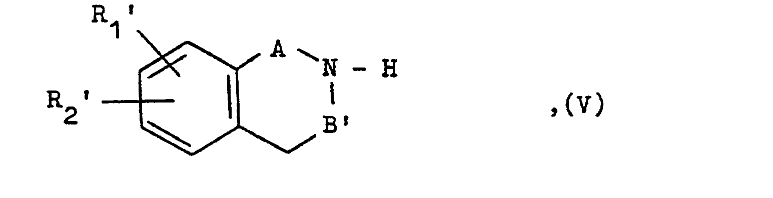 dans laquelle
A est défini comme au début,
R₁' représente un groupe hydroxy, amino ou alcoylamino protégé par un radical protecteur ou possède les significations mentionnées au début pour Ri,
R₂' représente un groupe hydroxy protégé par un radical protecteur ou possède les significations mentionnées au début pour R₂, et
B' représente un groupe carbonyle ou méthylène, avec un composé de formule générale 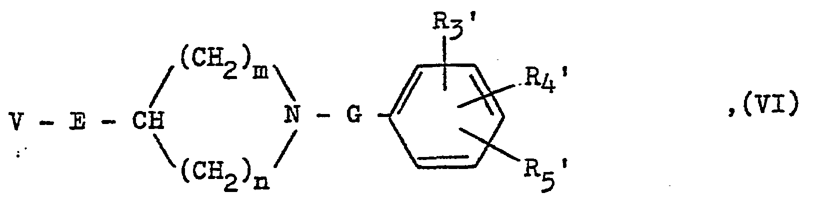 dans laquelle
E, G, met n sont définis comme au début,
R_{3'}, R_{4'} et Rs_{'} possèdent les significations mentionnées au début pour R₃, R₄ et Rs, les groupes hydroxy, amino ou alcoylamino contenus dans les radicaux R₃ à R₅ pouvant toutefois être protégés par un radical protecteur, et V représente un groupe partant nucléophile tel qu'un atome d'halogène ou un groupe sulfonyloxy, et éventuellement ensuite, on clive un radical protecteur utilisé ou
d) pour la fabrication de composés de formule générale I dans laquelle A représente un groupe -CH₂-CH₂- ou -CH=CH- et B un groupe thiocarbonyle, on fait réagir un composé de formule générale 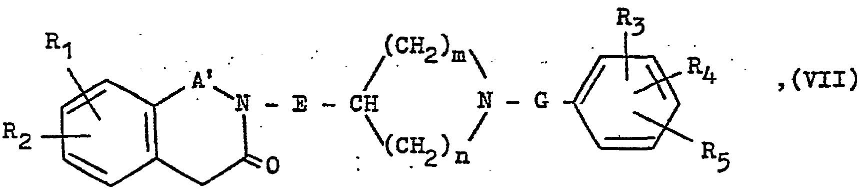 dans laquelle
Ri à Rs, E, G, met n sont définis comme au début, et
A' représente un groupe -CH₂-CH₂- ou -CH=CH-, avec un agent introduisant du soufre ou
e) pour la fabrication des composés de formule générale
I dans laquelle A représente un groupe 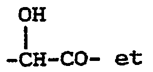 B représente un groupe méthylène, on réduit un composé de formule générale 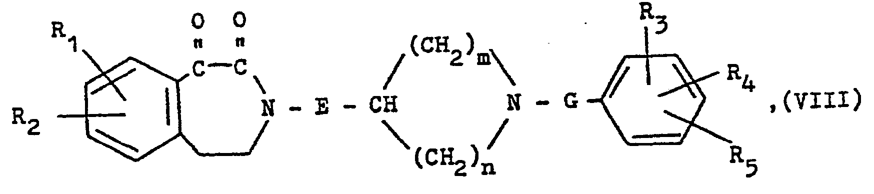 dans laquelle
R₁ à Rs, E, G, met n sont définis comme au début, dans un solvant approprié ou
f) pour la fabrication des composés de formule générale I dans laquelle A représente un groupe - CH₂-CH₂- ou -CH=CH- et B représente un groupe méthylène, on réduit un composé de formule générale 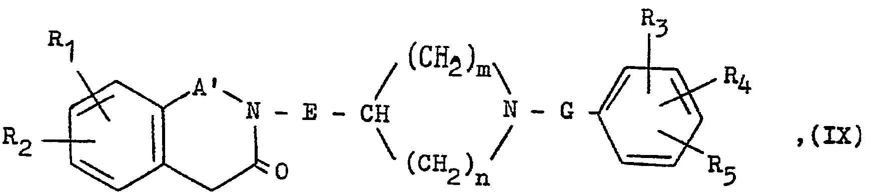 dans laquelle
R₁ à R₅, E, G, met n sont définis comme au début, et A, représente un groupe -CH₂-CH₂- ou - CH=CH-, dans un solvant approprié ou
g) pour la fabrication des composés de formule générale I dans laquelle A représente le groupe -CO-CO-, on oxyde un composé de formule générale 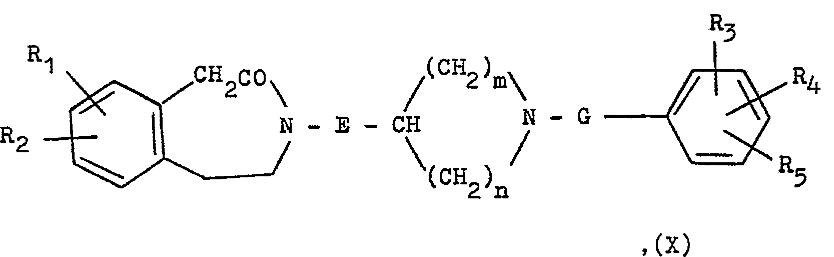 dans laquelle
Ri à Rs, E, G, met n sont définis comme au début, dans un solvant ou mélange de solvants approprié ou
h) pour la fabrication des composés de formule générale I dans laquelle G possède les significations mentionnées au début pour G, à l'exception des radicaux contenant un atome de soufre, un groupe sulfinyle ou sulfonyle, A représente le groupe -CH₂-CH₂- et B représente le groupe méthylène ou carbonyle, on hydrogène un composé de formule générale 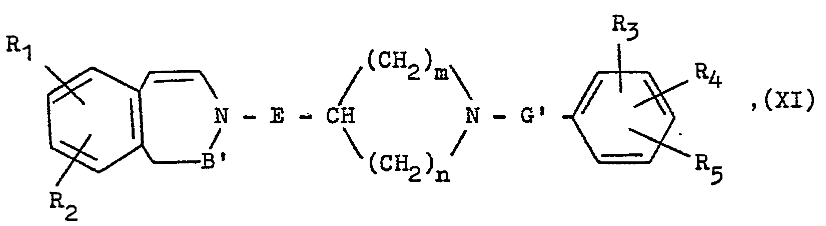 dans laquelle
Rᵢ à Rs, E, met n sont définis comme au début,
G_{'} possède les significations mentionnées au début pour G, à l'exception des radicaux contenant un atome de soufre, un groupe sulfinyle ou sulfonyle, et
B' représente le groupe méthylène ou carbonyle, dans un solvant ou mélange de solvants,
et ensuite, si on le désire, on transforme un composé ainsi obtenu de formule générale I, dans laquelle R₁ et/ou R₃ représente un groupe nitro, au moyen d'une réduction en un composé amino correspondant de formule générale I ou
on transforme un composé ainsi obtenu de formule générale I, dans laquelle R₄ représente un groupe hydroxy ou amino, au moyen d'une acylation en un composé alcanesulfonyloxy ou alcanoylamino correspondant de formule générale I ou
on sépare un composé ainsi obtenu de formule générale 1
qui contient au moins un centre chiral, en ses diastéréoisomères ou en ses énantiomères et/ou on transforme un composé ainsi obtenu de formule générale 1 en ses sels d'addition d'acides, en particulier en ses sels d'addition d'acides physiologiquement supportables avec des acides non organiques ou organiques.

2. Procédé selon la revendication 1 pour la fabrication des nouveaux dérivés amines cycliques de formule générale, 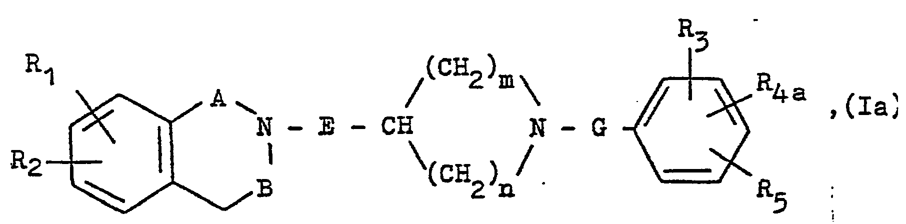 dans laquelle
R₁ à Rs, Rₛ, A, B, E, G, met n sont définis comme dans la revendication 1 et
R₄a représente un atome d'hydrogène, un groupe alcoxy, amino, alcoylamino ou dialcoylamino avec chacun 1 à 3 atomes de carbone dans chaque partie alcoyle, ou un groupe alcanoylamino avec 2 ou 3 atomes de carbone dans la partie alcanoyle, ou
Rs et R₄ₐ représentent ensemble un groupe alcoylènedioxy avec 1 ou 2 atomes de carbone, de leurs énantiomères, de leurs diastéréoisomères et de leurs sels d'addition d'acides, caractérisé en ce que
a) on fait réagir un composé de formule générale 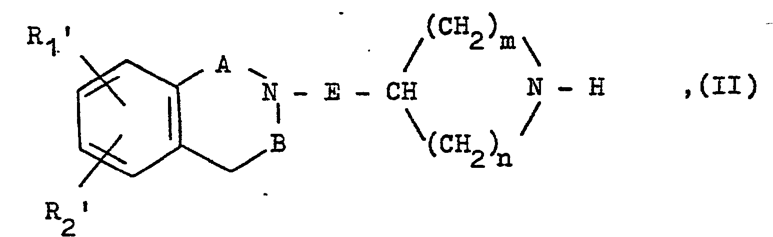 dans laquelle R1', R2', A, B, E, met n sont définis comme dans la revendication 1, avec un composé de formule générale 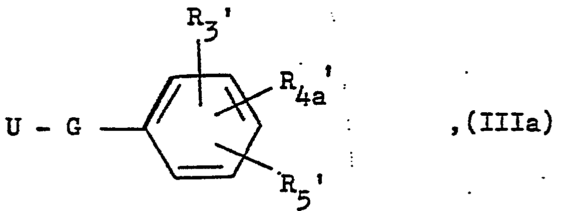 dans laquelle
R_{3'}, R₅' G et U sont définis comme dans la revendication 1, et
R₄ₐ' possède les significations mentionnées au début pour R₄ₐ, les groupes hydroxy, amino ou alcoylamino contenus dans les radicaux R₃, R₄ₐ et Rs pouvant toutefois être protégés par un radical protecteur et éventuellement, on clive ensuite un radical protecteur utilisé ou
b) pour la fabrication des composés de formule générale la dans laquelle G possède les significations mentionnées au début pour G à l'exception des radicaux contenant un groupe sulfényle, sulfinyle ou sulfonyle, A représente le groupe -CH₂-CH₂-, B représente le groupe méthylène ou carbonyle et m + n représentent le nombre 4, on hydrogène un composé de formule générale 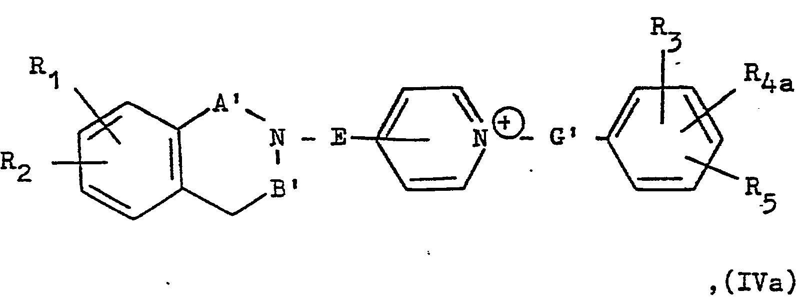 dans laquelle
R₁ à R₃, R₅, A' , B', E et G' sont définis comme dans la revendication 1, et
R₄a est défini comme au début, dans un solvant ou mélange de solvants ou
c) pour la fabrication des composés de formule générale la dans laquelle B représente le groupe carbonyle ou méthylène, on fait réagir un composé de formule générale 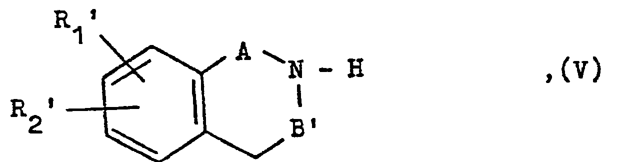 dans laquelle R₁', R₂', A et B' sont définis comme dans la revendication 1, avec un composé de formule générale 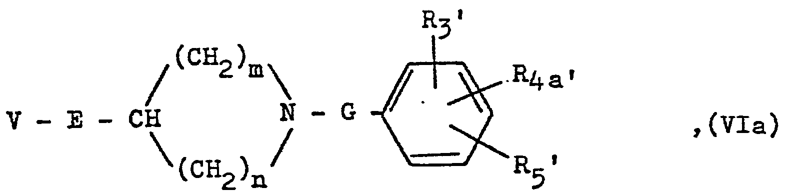 dans laquelle
Rs_{'}, Rs_{'}, E, G, V, met n sont définis comme dans la revendication 1, et
R₄' possède les significations mentionnées au début pour R₄, les groupes hydroxy, amino ou alcoylamino contenus dans les radicaux R₃, R₄ₐ et R5 pouvant toutefois être protégés par un radical protecteur, et éventuellement ensuite on clive un radical protecteur utilisé ou
d) pour la fabrication des composés de formule générale la dans laquelle A représente un groupe - CH₂-CH₂- ou -CH=CH- et B un groupe thiocarbonyle, on fait réagir un composé de formule générale 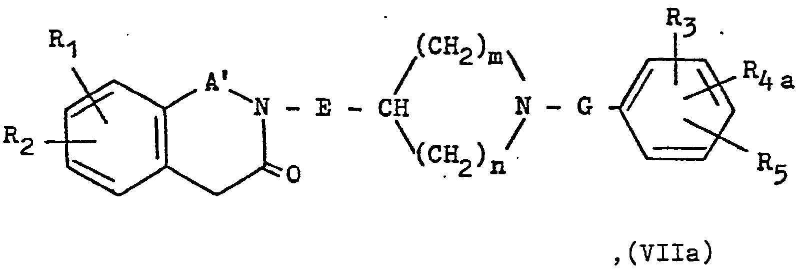 dans laquelle
R₁ à R₃, Rs, A', E, G, m et n sont définis comme dans la revendication 1, et
R₄ₐ est défini conmlê au début, avec un agent introduisant du soufre ou
e) pour la fabrication des composés de formule générale
Ia dans laquelle A représente un groupe 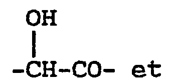 B représente un groupe méthylène, on réduit un composé de formule générale 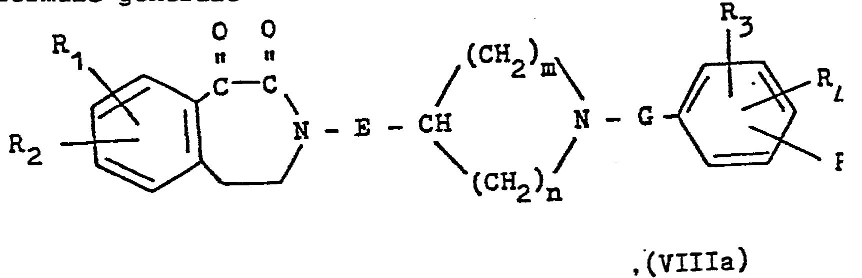 dans laquelle
Ri à R₃, Rs, E, G, m et n sont définis conune dans la revendication 1, et R₄ₐ est défini comme au début, dans un solvant approprié, ou
f) pour la fabrication des composés de formule générale la dans laquelle A représente un groupe - CH₂-CH₂- ou -CH=CH- et B représente un groupe méthylène, on réduit un composé de formule générale 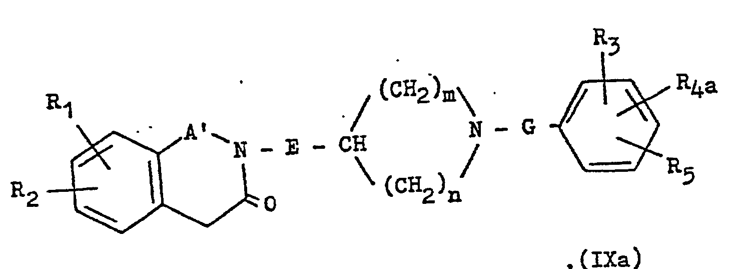 dans laquelle Ri à R₃, Rs, A' E, G, m et n sont définis conune dans 1 revendication 1, et
R₄a est défini comme du début, dans un solvant approprié, ou
g) pour la fabrication des composés de formule la dans laquelle A représente le groupe -COCO-, on oxyde un composé de formule générale 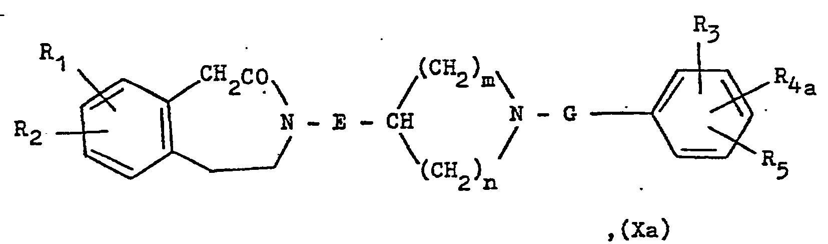 dans laquelle
Ri à R₃, Rs, E, G, met n sont définis comme dans la révendication 1, et
R₄ₐ est défini comme au début, dans un solvant ou mélange de solvants approprié, ou
h) pour la fabrication des composés de formule générale la dans laquelle G possède les significations mentionnées au début pour G, à l'exception des radicaux contenant un atome de soufre, un groupe sulfinyle ou sulfonyle, A représente le groupe -CH₂-CH₂- et B représente le groupe méthylène ou carbonyle, on hydrogène un composé de formule générale 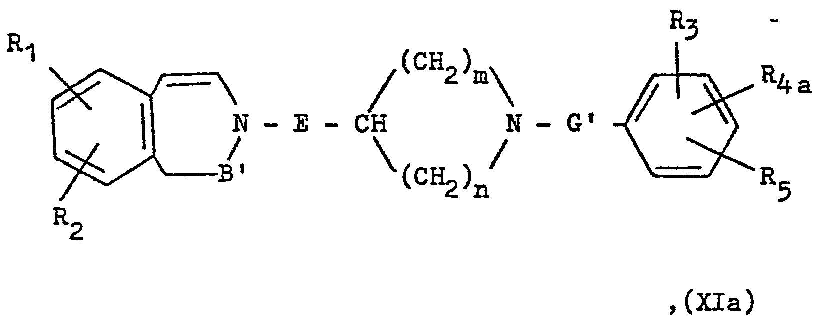 dans laquelle
R₁ à R₃, Rs, B', E, G', met n sont définis comme dans la revendication 1, et
R₄ₐ est défini comme au début, dans un solvant ou mélange de solvants,
et ensuite, si on le désire, on transforme un composé ainsi obtenu de formule générale la, dans laquelle R₁ et/ou R₃ représente un groupe nitro, au moyen d'une réduction, en un composé amino correspondant de formule générale la ou
on transforme un composé ainsi obtenu de formule générale la, dans laquelle R₄a représente un groupe amino, au moyen d'une alcanoylation, en un composé alcanoylamino correspondant de formule générale la ou
on sépare un composé ainsi obtenu de formule générale la qui contient au moins un centre chiral, en ses diastéréoisomères ou en ses énantiomères et/ou
on transforme un composé ainsi obtenu de formule générale la en ses sels d'addition d'acides, en particulier en ses sels d'addition d'acides physiologiquement supportables avec des acides non organiques ou organiques.

3. Procédé selon les revendications 1 et 2, caractérisé en ce que la réaction est effectuée dans un solvant.

4. Procédé selon les revendications le, 2e et 3, caractérisé en ce que la réaction est effectuée au moyen d'un hydrure métallique tel que le borhydrure de sodium, à des températures entre 0 et 80_{°}C, de préférence à des températures entre 15 et 40_{°}C.

5. Procédé selon les revendications lf, 2f et 3, caractérisé en ce que la réaction est effectuée au moyen d'un hydrure métallique tel que l'hydrure de lithium et d'aluminium ou le diborane ou au moyen d'un complexe du borane et d'un thioéther, par exemple avec le complexe du diborane et du diméthylsulfure, à des températures entre 0 et 25_{°}C, de préférence toutefois à des températures entre 10 et 25_{°}C.

6. Procédé selon les revendications 1 g, 2g et 3,
caractérisé en ce que l'oxydation est effectuée au moyen de permanganate de potassium, de dioxyde de sélénium ou de bichromate de sodium à des températures entre 0 et 100_{°}C.

7. Procédé pour la fabrication d'un médicament, caractérisé en ce que l'on introduit par voie non chimique, un composé de formule générale 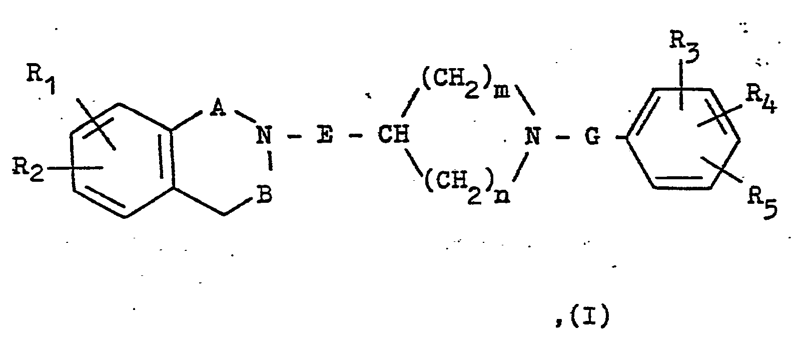
dans laquelle R₁ à R₅, A, B, E, G, met n sont définis comme dans la revendication 1, ou son sel d'addition d'acide physiologiquement supportable, dans un ou plusieurs excipients et/ou diluants inertes.
